(19) Europäisches Patentamt / European Patent Office / Office européen des brevets

(11) **EP 3 271 397 B1**

(12) **EUROPEAN PATENT SPECIFICATION**

(45) Date of publication and mention of the grant of the patent:
**03.03.2021 Bulletin 2021/09**

(21) Application number: **16765829.3**

(22) Date of filing: **18.03.2016**

(51) Int Cl.:
*C07K 16/28* (2006.01)    *G01N 33/68* (2006.01)
*A61K 39/395* (2006.01)    *A61K 47/68* (2017.01)
*A61P 35/00* (2006.01)

(86) International application number:
**PCT/US2016/023144**

(87) International publication number:
**WO 2016/149621 (22.09.2016 Gazette 2016/38)**

(54) **INHIBITORY MONOCLONAL ANTIBODY TARGETING POTASSIUM CHANNEL KCNK9**

INHIBITORISCHER MONOKLONALER ANTIKÖRPER GEGEN DEN KALIUMKANAL KCNK9

ANTICORPS MONOCLONAL INHIBITEUR CIBLANT LE CANAL POTASSIQUE KCNK9

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.03.2015 US 201562134724 P**
**30.06.2015 US 201562186772 P**

(43) Date of publication of application:
**24.01.2018 Bulletin 2018/04**

(73) Proprietors:
• **The Johns Hopkins University**
**Baltimore, MD 21218 (US)**
• **University Of Maryland, Baltimore**
**Baltimore, Maryland 21201-1508 (US)**

(72) Inventors:
• **LATERRA, John J.**
**Baltimore, Maryland 21212 (US)**
• **LI, Min**
**Lutherville-Timonium, Maryland 21093 (US)**
• **SUN, Han**
**Baltimore, Maryland 21201 (US)**
• **FULTON, Amy M.**
**Baltimore, Maryland 21201 (US)**

(74) Representative: **Murgitroyd & Company**
**Murgitroyd House**
**165-169 Scotland Street**
**Glasgow G5 8PL (GB)**

(56) References cited:
**WO-A1-2005/075510    WO-A1-2012/057421**

US-A1- 2009 149 496

• ILONA KOVÁCS ET AL: "TASK-3 immunoreactivity shows differential distribution in the human gastrointestinal tract", VIRCHOWS ARCHIV, SPRINGER, BERLIN, DE, vol. 446, no. 4, 1 April 2005 (2005-04-01) , pages 402-410, XP019344713, ISSN: 1432-2307, DOI: 10.1007/S00428-005-1205-7
• ANNI INNAMAA ET AL: "Expression and prognostic significance of the oncogenic K2P potassium channel KCNK9 (TASK-3) in ovarian carcinoma.", ANTICANCER RESEARCH, vol. 33, 1 January 2013 (2013-01-01), pages 1401-1408, XP055507445,
• DENG P Y ET AL: "Serotonin increases GABA release in rat entorhinal cortex by inhibiting interneuron TASK-3 K<+> channels", MOLECULAR AND CELLULAR NEUROSCIENCES, SAN DIEGO, US, vol. 39, no. 2, 1 October 2008 (2008-10-01), pages 273-284, XP025434233, ISSN: 1044-7431, DOI: 10.1016/J.MCN.2008.07.005 [retrieved on 2008-07-18]
• POCSAI K ET AL: "Melanoma cells exhibit strong intracellular TASK-3-specific immunopositivity in both tissue sections and cell culture", CELLULAR AND MOLECULAR LIFE SCIENCES, BIRKHÄUSER-VERLAG, BA, vol. 63, no. 19-20, 28 September 2006 (2006-09-28), pages 2364-2376, XP019440754, ISSN: 1420-9071, DOI: 10.1007/S00018-006-6166-8

- C. E. CLARKE ET AL: "The M1P1 Loop of TASK3 K2P Channels Apposes the Selectivity Filter and Influences Channel Function", JOURNAL OF BIOLOGICAL CHEMISTRY, vol. 283, no. 25, 20 June 2008 (2008-06-20), pages 16985-16992, XP055311689, US ISSN: 0021-9258, DOI: 10.1074/jbc.M801368200
- CLARKE ET AL.: 'The M1P1 loop of TASK3 K2P channels apposes the selectivity filter and influences channel function' JOURNAL OF BIOLOGICAL CHEMISTRY vol. 283, no. 25, 2008, pages 16985 - 16992, XP055311681
- KIM ET AL.: 'TASK-3, a new member of the tandem pore K+ channel family' JOURNAL OF BIOLOGICAL CHEMISTRY vol. 275, no. 13, 2000, pages 9340 - 9347, XP002970106
- HUANG ET AL.: 'Targeling potassium cnanneis in cancer' THE JOURNAL OF CELL BIOLOGY vol. 206, no. 2, 2014, pages 151 - 162, XP055311685
- SUN ET AL.: 'A monoclonal antibody against KCNK9 K+ channel extracellular domain inhibits tumour growth and metastasis' NATURE COMMUNICATIONS vol. 7, no. 10339, 04 February 2016, pages 1 - 12, XP055311687

**Description**

MATERIAL SUBMITTED ELECTRONICALLY

This application contains a sequence listing. It has been submitted electronically via EFS-Web as an ASCII text file entitled "111232-00498_ST25.txt". The sequence listing is 20,480 bytes in size, and was created on March 15, 2016.

BACKGROUND

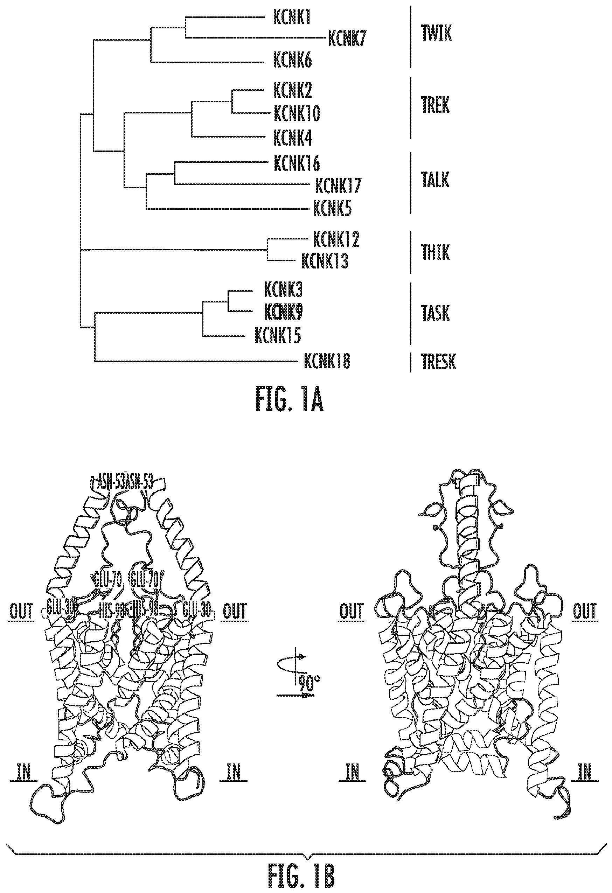

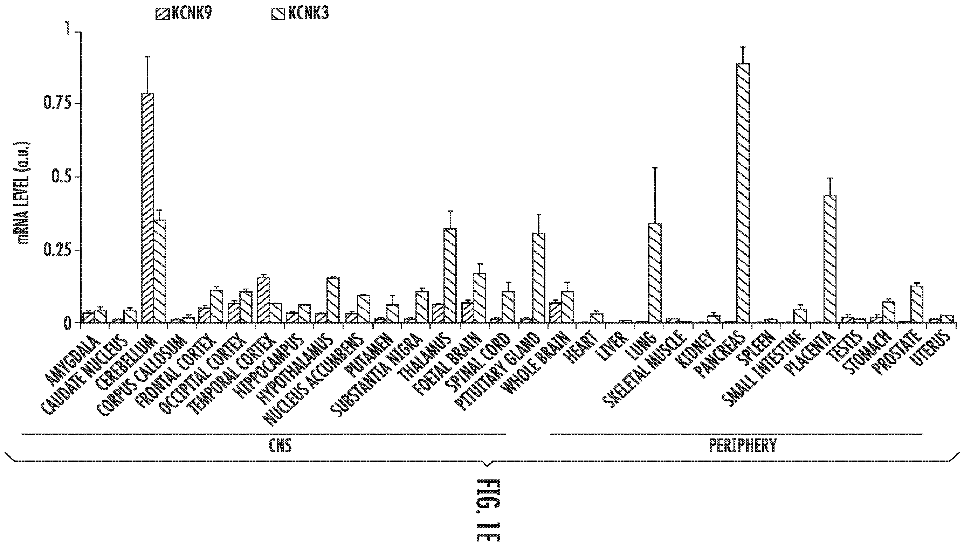

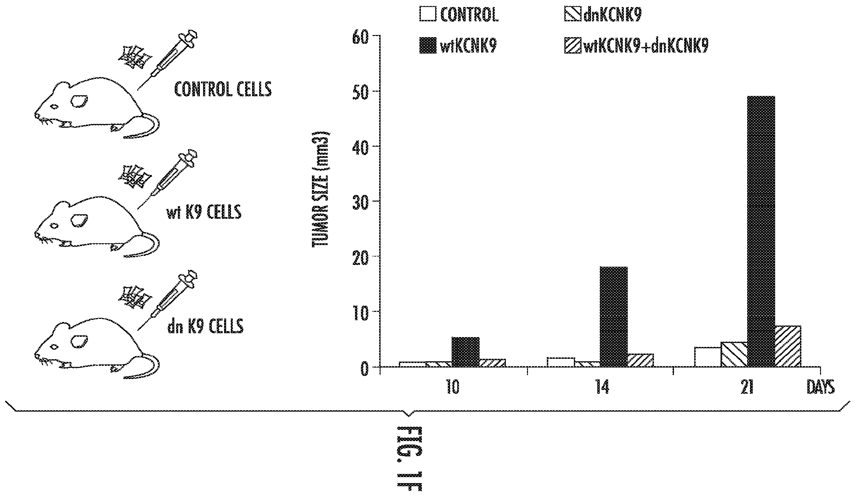

Ion channels govern portals that facilitate ionic passage across cellular membranes in all organisms. Transient change of ionic distribution alters membrane potential that forms the basis for a variety of biological processes. Potassium ($K^+$) channels are the most abundant and diverse ion channels (Sun & Li (2013) Acta Pharmacol Sin 34, 199-204). Among them, two-pore domain $K^+$ (K2P) channels are the newest members. To date, fifteen mammalian K2P channel subtypes have been discovered (FIG. 1A; Enyedi & Czirjak, (2010) Physiological Reviews 90, 559-605) and each subtype plays a distinct role in physiological processes and disease, including mental retardation, familial migraine and cancer (Enyedi & Czirjak, (2010) Physiological Reviews 90, 559-605; Barel et al. (2008) The American Journal of Human Genetics 83, 193-199; Lafreniere et al. (2010) Nat Med 16, 1157-1160; Mu et al. (2003) Cancer Cell 3, 297-302). Despite their significance, limited knowledge has been gained about individual K2P subtypes, partly due to K2Ps' nature of being highly homologous and the paucity of subtype-specific tools.

KCNK9 is a member of the K2P channel family. Under physiological conditions, KCNK9 is primarily expressed in tissues of the central nervous system such as the cerebellum, acting to maintain resting membrane potential and regulate action potential firing (Enyedi & Czirjak, (2010) Physiological Reviews 90, 559-605). KCNK9 has also been implicated in cancer based on genetic evidence including genomic amplification, mRNA and protein over-expression in human breast tumors and lung tumors (Mu et al. (2003) Cancer Cell 3, 297-302). Enforced KCNK9 expression has been shown to promote transformation of mouse embryonic fibroblasts in nude mice, possibly by improving cell survival under hypoxic or serum-deprived conditions (Mu et al. (2003) Cancer Cell 3, 297-302; Pei et al. (2003) Proceedings of the National Academy of Sciences 100, 7803-7807). However, how endogenous KCNK9 contributes to neoplasia and its potential as a therapeutic target has remained elusive due to the lack of specific modulators of KCNK9 functions. Genetic studies of K2P channels are often difficult to interpret due to developmental and compensatory effects (Linden et al. (2008) Journal of Pharmacology and Experimental Therapeutics 327, 277-286). High throughput screening of small molecules has been carried out to identify specific modulators of KCNK9 but has nevertheless made limited progress (Miller et al. (2012) Probe Reports from the NIH Molecular Libraries Program [Internet]). This is partly because it is difficult to rationally design chemical screens and develop small molecules against targets that share high sequence and structure homology. Antibodies, known for their exquisite specificity, have been broadly used to target cell surface receptors and antigens, especially as applied to cancer treatment (Chan & Carter (2010) Nat Rev Immunol 10, 301-316; Zhang et al. (2007) Cell Res 17, 89-99). However, the feasibility of using antibodies to modulate ion channel activity is not well explored.

K2P channels share considerable architectural similarity. They assemble as dimers with each subunit containing two concatenated pore-lining regions (P1, P2) and four transmembrane domains (M1-M4). One signature feature of K2P channels is a loop of about 60 amino acids on the extracellular side between the M1 and P1 domains (M1P1). Crystal structure analysis of human K2P channels reveals this loop as a structured domain that "caps" the extracellular ion pathway, providing an explanation for K2P's insensitivity to common channel blockers (Fink et al. (1998) Embo J 17, 3297-3308; Lesage et al. (1996) Embo J 15, 1004-1011). Mutational analysis and chimera studies have provided compelling evidence for M1P1's role in sensing extracellular stimuli and regulating channel gating (Clarke et al. (2008) Journal of Biological Chemistry 283, 16985-16992; Lotshaw (2007) Cell Biochemistry and Biophysics 47, 209-256).

Ilona Kovács et al., Virchows Archiv, Springer, Berlin, DE, vol. 446, no. 4, 1 April 2005, pages 402-410, describes the distribution of the TASK-3 protein, which is encoded by the KCNK9 gene, in the human gastrointestinal system using immunohistochemistry. An anti-TASK-3 antibody designated APC-044 is disclosed in Table 2 as binding to the extracellular domain of TASK-3 corresponding to amino acid residues 57-73. WO 2012/057421 describes cancer-metastasis suppression by means of increased TASK-3 potassium channel expression. Anni Innamaaa et al., Anticancer Research, vol. 33, 1 January 2013, pages 1401-1408, describes the expression and prognostic significance of KCNK9 in ovarian carcinoma. TASK-3 blockers caused a significant reduction in cell proliferation and an increase in apoptosis in SKOV-3 and OVCAR-3 cell lines.

BACKGROUND

The practice of the present invention will typically employ, unless otherwise indicated, conventional techniques of cell biology, cell culture, molecular biology, transgenic biology, microbiology, recombinant nucleic acid (e.g., DNA) technology, immunology, and RNA interference (RNAi) which are within the skill of the art. Non-limiting descriptions of certain of these techniques are found in the following publications: Ausubel, F., et al., (eds.), Current Protocols in Molecular Biology, Current Protocols in Immunology, Current Protocols in Protein Science, and Current Protocols in Cell Biology, all John Wiley & Sons, N.Y., edition as of December 2008; Sambrook, Russell, and Sambrook, Molecular Cloning. A Laboratory Manual, 3rd ed., Cold Spring Harbor Laboratory Press, Cold Spring Harbor, 2001; Harlow, E. and Lane, D., Antibodies-A Laboratory Manual, Cold Spring Harbor Laboratory Press, Cold Spring Harbor, 1988; Freshney, R. I., "Culture of Animal Cells, A Manual of Basic Technique", 5th ed., John Wiley & Sons, Hoboken, N.J., 2005. Non-limiting information regarding therapeutic agents and human diseases is found in Goodman and Gilman's The Pharmacological Basis of Therapeutics, 11th Ed., McGraw Hill, 2005, Katzung, B. (ed.) Basic and Clinical Pharmacology, McGraw-Hill/Appleton & Lange 10th ed. (2006) or 11th edition (July 2009). Non-limiting information regarding genes and genetic disorders is found in McKusick, V. A.: Mendelian Inheritance in Man. A Catalog of Human Genes and Genetic Disorders. Baltimore: Johns Hopkins University Press, 1998 (12th edition) or the more recent online database: Online Mendelian Inheritance in Man, OMIM™. McKusick-Nathans Institute of Genetic Medicine, Johns Hopkins University (Baltimore, Md.) and National Center for Biotechnology Information, National Library of Medicine (Bethesda, Md.), as of May 1, 2010, World Wide Web URL: http://www.ncbi.nlm.nih.gov/omim/ and in Online Mendelian Inheritance in Animals (OMIA), a database of genes, inherited disorders and traits in animal species (other than human and mouse), at http://omia.angis.org.au/contact.shtml. Standard art-accepted meanings of terms are used herein unless indicated otherwise. Standard abbreviations for various terms are used herein.

SUMMARY OF THE INVENTION

The invention is as defined in the claims.

The present invention provides an isolated antibody or antigen-binding fragment thereof as claimed in claim 1 that specifically binds to an epitope of the extracellular domain of the KCNK9 potassium channel between amino acids Glu30 and Ile88 of SEQ ID NO: 1 with an affinity $K_D$ of 0.7 nM or less, wherein the antibody or antigen-binding fragment thereof induces internalization of the KCNK9 potassium channel from the surface of a cell. In some embodiments, the presently disclosed antibody or antigen-binding fragment thereof does not bind to other potassium channels. In some embodiments, the presently disclosed antibody comprises a monoclonal antibody. In some embodiments, the presently disclosed antibody comprises a humanized antibody (e.g., a humanized monoclonal antibody).

In an aspect, the present invention provides a pharmaceutical composition comprising the antibody or antigen-binding fragment thereof according to the invention.

In an aspect, the present invention provides a diagnostic composition comprising the antibody or antigen-binding fragment thereof according to the invention.

In an aspect, the present invention provides a kit comprising the antibody or antigen-binding fragment thereof according to the invention.

In an aspect, the present invention provides a method of assessing for the presence of KCNK9 expressing cells comprising contacting in vitro a cell or tissue suspected of expressing KCNK9 on its surface with the antibody, antigen-binding fragment thereof or diagnostic composition according to the invention.

In an aspect, there is provided the antibody or antigen-binding fragment thereof according to the invention for use, according to claim 11, in inhibiting growth and/or metastasis of a tumor in a subject having or suspected of having breast cancer or lung cancer.

Disclosed, but not claimed, is a method of inhibiting KCNK9 activity comprising contacting a cell or tissue expressing KCNK9 on its surface with the presently disclosed antibody, antibody fragment, or derivative thereof, or the presently disclosed pharmaceutical composition.

Disclosed, but not claimed, is a method of inhibiting the growth or survival of a KCNK9 expressing cancer cell comprising contacting a cancer cell expressing KCNK9 on its surface with the presently disclosed antibody, antibody fragment, or derivative thereof, or the presently disclosed pharmaceutical composition.

Disclosed, but not claimed, is a method of inhibiting growth and/or metastasis of a tumor in a subject having or suspected of having a cancer, the method comprising administering to the subject the presently disclosed antibody, antibody fragment, or derivative thereof, or the presently disclosed pharmaceutical composition, in an amount effective to inhibit growth and/or metastasis of the tumor in the subject.

Disclosed, but not claimed, is a method of stimulating complement-dependent cancer cell cytotoxicity in a subject having or suspected of having cancer, the method comprising administering to the subject the presently disclosed antibody, antibody fragment, or derivative thereof, or the presently disclosed pharmaceutical composition, in an amount effective to stimulate complement-dependent cancer cell cytotoxicity in the subject.

Disclosed, but not claimed, is a method for the treatment of a KCNK9 expressing cancer in a subject in need thereof, the method comprising administering to the subject the presently disclosed antibody, antibody fragment, or derivative thereof, or the presently disclosed pharmaceutical composition, in an amount effective to treat the KCNK9 expressing cancer in the subject.

Certain aspects of the presently disclosed subject matter having been stated hereinabove, other aspects will become evident as the description proceeds when taken in connection with the accompanying Examples and Figures as best described herein below.

BRIEF DESCRIPTION OF THE DRAWINGS

Having thus described the presently disclosed subject matter in general terms, reference will now be made to the accompanying Figures, which are not necessarily drawn to scale, and wherein:

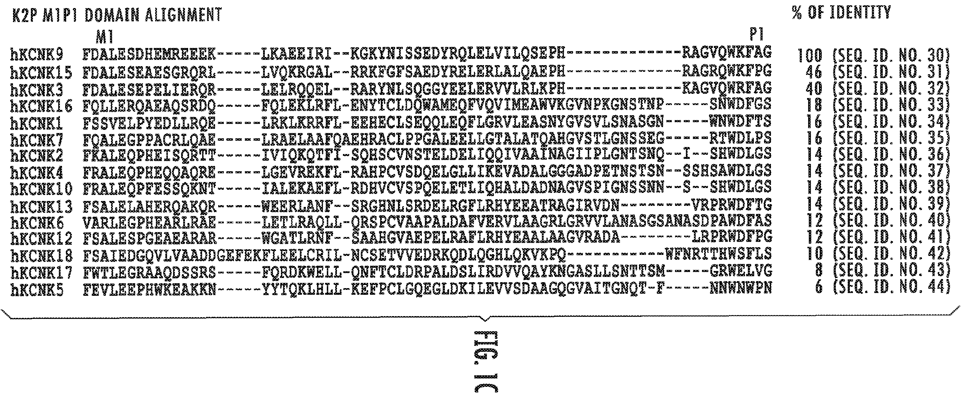

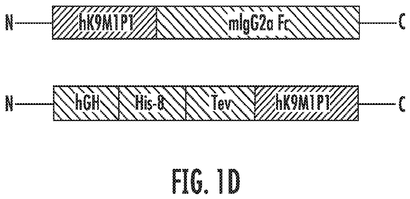

FIG. 1A, FIG. 1B, FIG. 1C, and FIG. 1D show characteristics of the target, epitopes and antigens. FIG. 1A shows a K2P phylogenetic tree calculated from a sequence alignment of the 15 human channel pore domain 1 sequences. Channels can be divided into six subfamilies based on sequence similarity and functional resemblance. FIG. 1B shows a 3D structure of hKCNK9 predicted by homology modeling based on hKCNK4 crystal structure. M1P1 domain targeted by antibody is colored blue. Residues within M1P1 domain essential for gating are annotated. FIG. 1C shows a sequence alignment corresponding to the M1P1 domain of 15 hK2P subtypes. M1P1 domain chosen for antibody generation is colored blue. Pairwise percentage of identical residues between the M1P1 domain of hKCNK9 and other K2P subtypes is calculated. FIG. 1D shows a schematic representation of recombinant antigens - hGH-hK9M1P1 and hK9M1P1-mIgG.

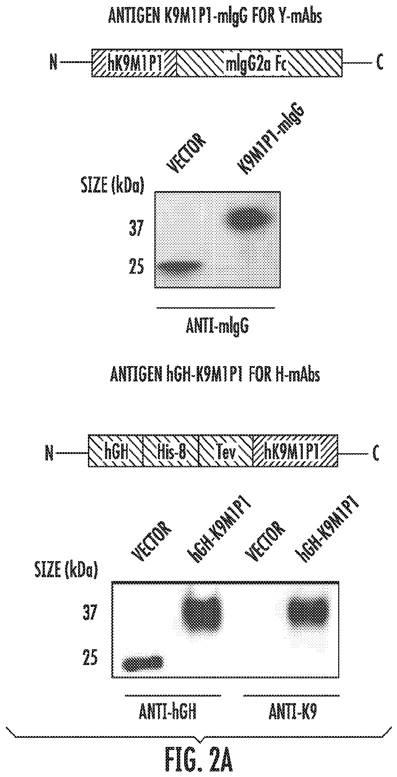

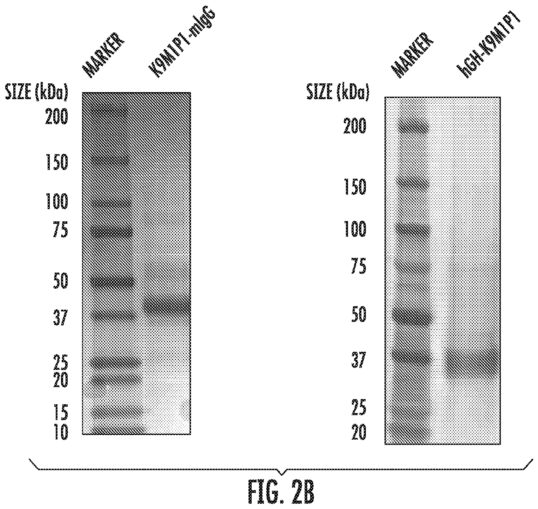

FIG. 2A and FIG. 2B show antigens generated for mAb development. FIG. 2A shows that hGH-K9M1P1 and K9M1P1-mIgG antigen (~37kDa) expression was verified by western blot (vector: 25kDa). FIG. 2B shows silver staining which was used to assess the purity of antigen;

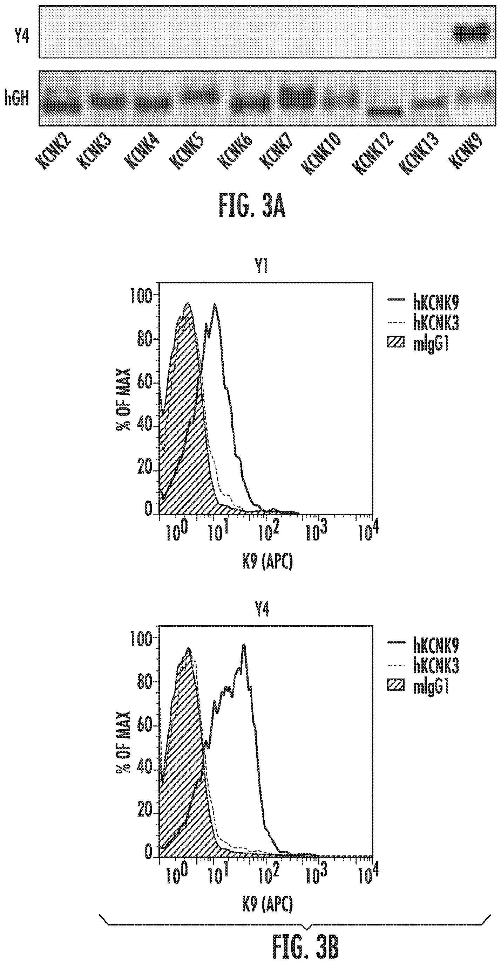

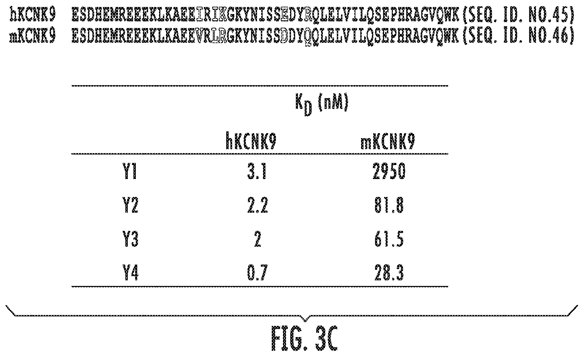

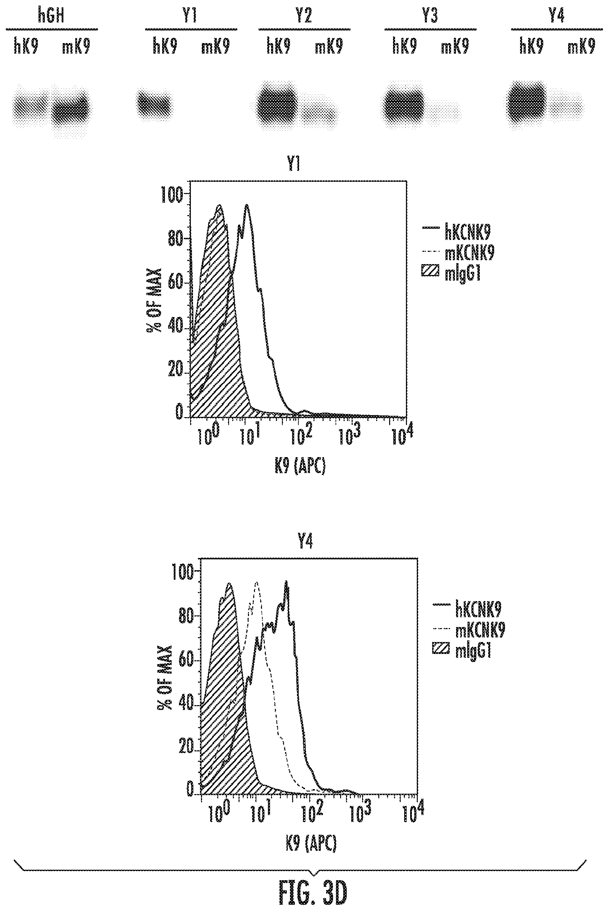

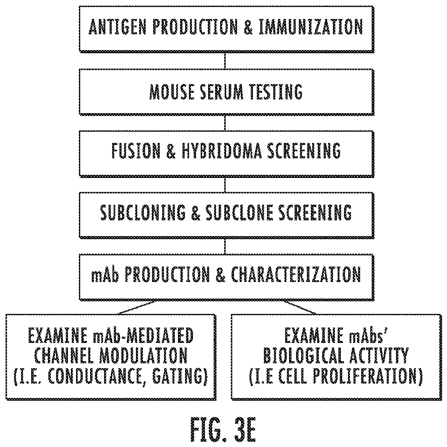

FIG. 3A, FIG. 3B, FIG. 3C, and FIG. 3D show that Y4 binds to hKCNK9 with subtype specificity and high affinity. FIG. 3A shows western blot analysis of hGH-K2PM1P1 recombinant proteins blotted with Y4 and anti-hGH antibodies. FIG. 3B shows flow cytometry analysis of HEK293 cells transiently expressing hKCNK9 or hKCNK3 stained first with mAbs then counterstained with a secondary APC-conjugated goat anti-mouse IgG antibody. FIG. 3C shows binding kinetics of Y1-4 mAbs determined by Octet platform. Association and dissociation rates are measured in real-time by incubating probes coated with Y1-4 mAbs in the presence of hGH-hK9M1P1 or hGH-mK9M1P1 recombinant protein. FIG. 3D shows Y1-4 mAbs' differential affinity to human versus murine KCNK9 validated by western blotting of recombinant proteins and flow cytometry analysis of HEK293 cells transiently expressing hKCNK9 or mKCNK9;

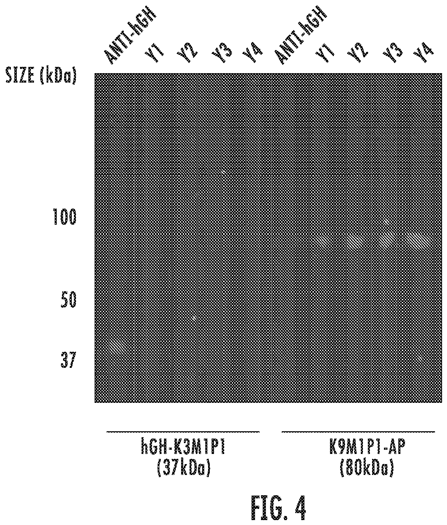

FIG. 4 shows preferential binding of Y-mAbs to hKCNK9 over hKCNK3. Western blot analysis of Y-mAbs against hGH-hKCNK3 and hKCNK9-alkaline phosphatase recombinant proteins was performed. Anti-hGH and nonspecific mIgG1 antibodies were used as control;

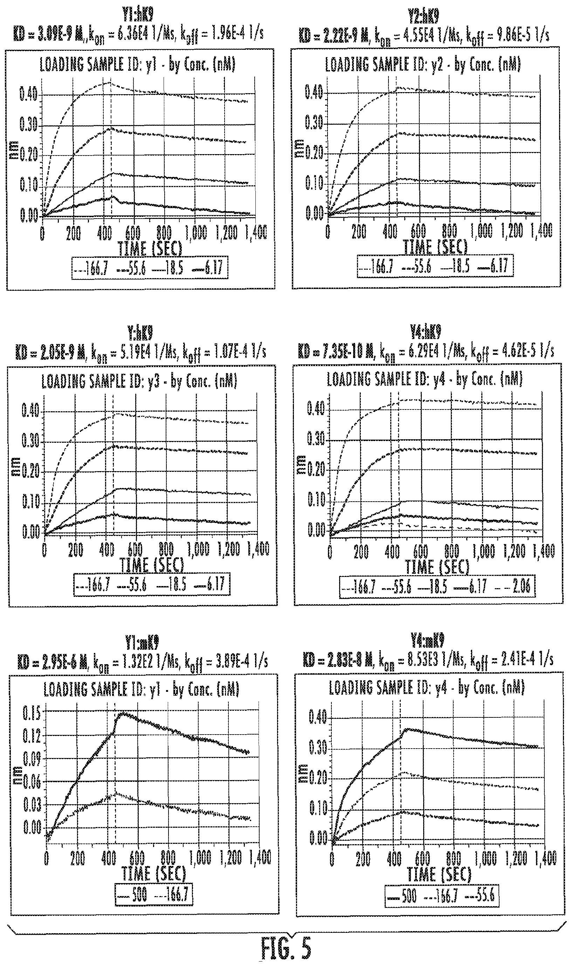

FIG. 5 shows real time measurement of Y-mAbs' binding. Association ($k_{on}$) and dissocation ($k_{off}$) constants were measured by Octet platform to determine the affinity of mAb binding to hKCNK9M1P1 or mKCNK9M1P1;

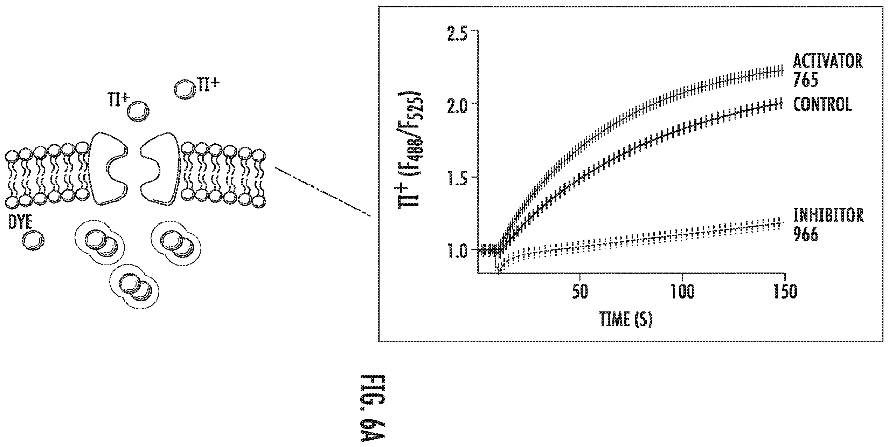

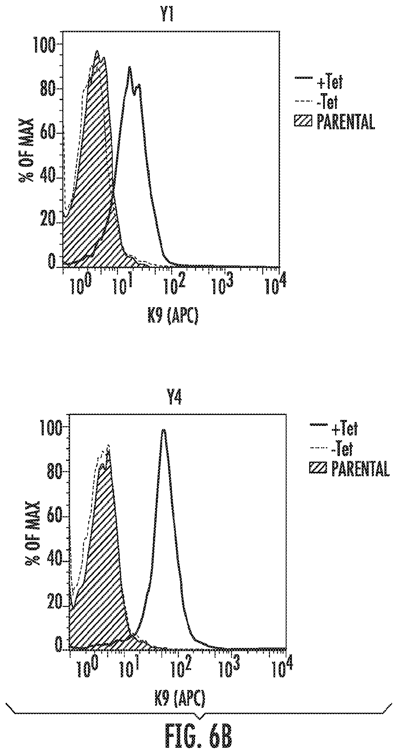

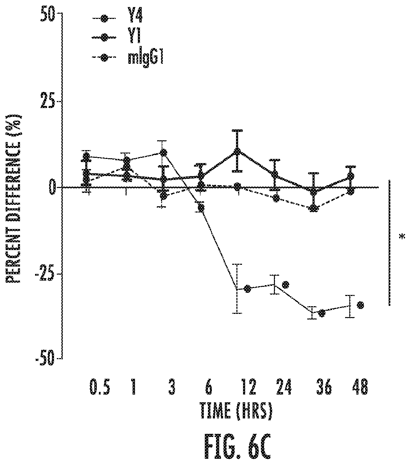

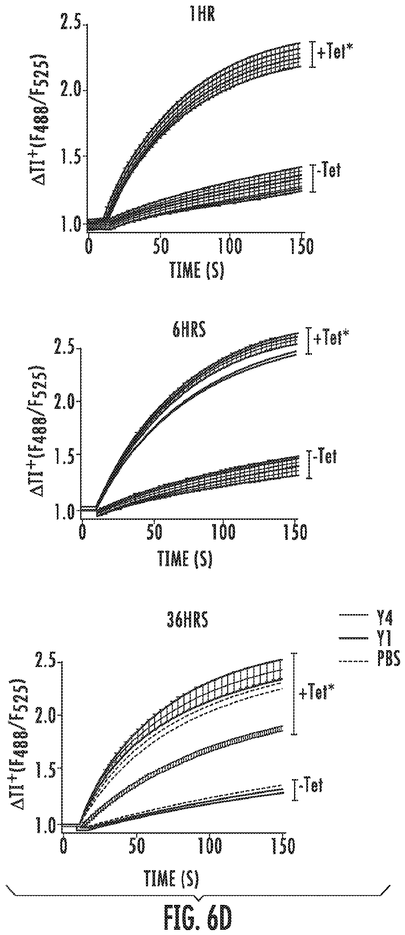

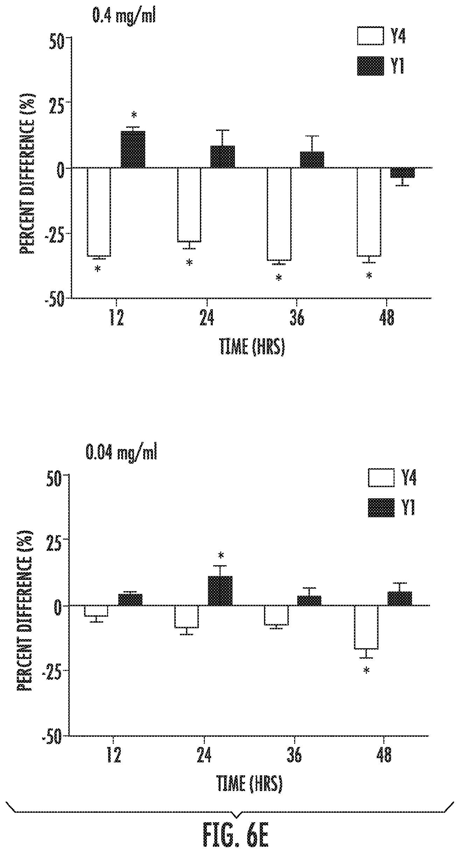

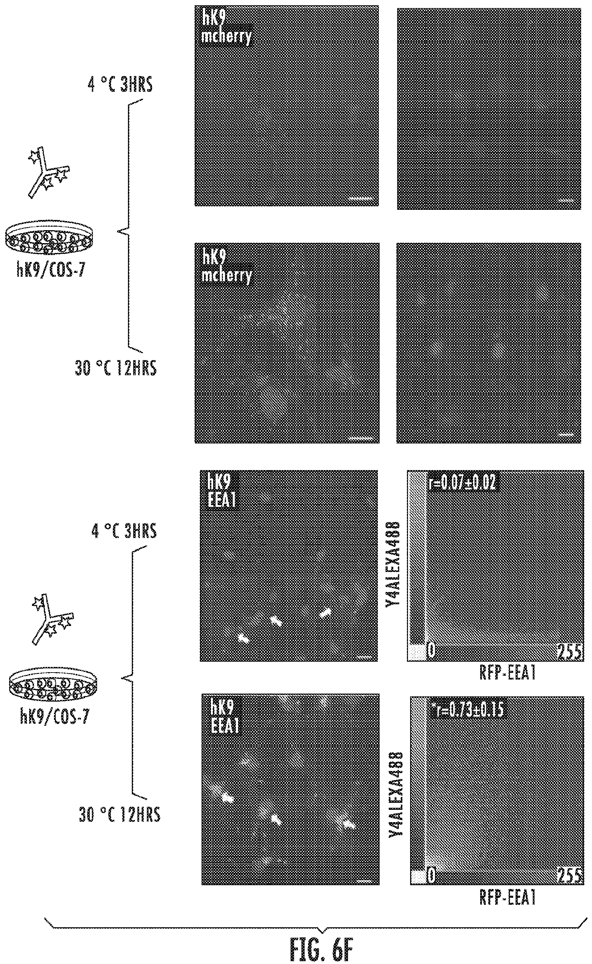

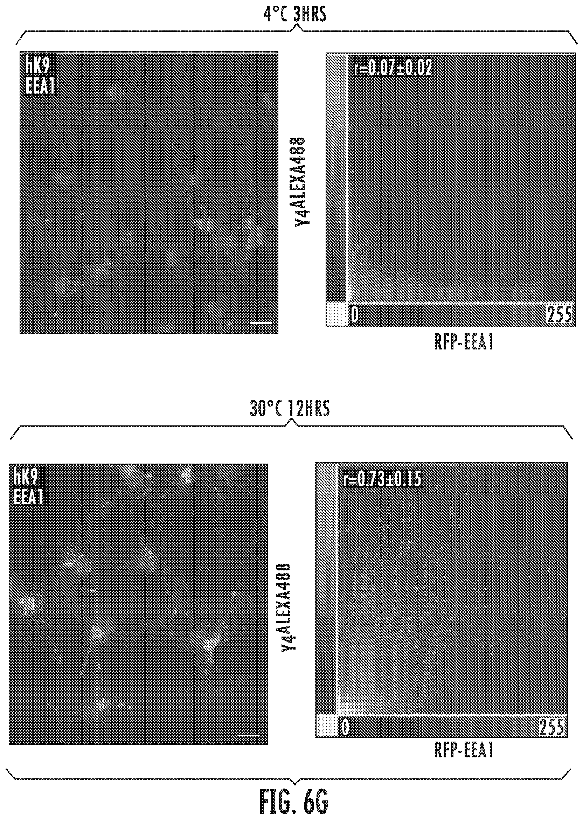

FIG. 6A, FIG. 6B, FIG. 6C, FIG. 6D, FIG. 6E, and FIG. 6F show that Y4 inhibits hKCNK9 by inducing internalization of the channel from the cell surface. FIG. 6A shows a schematic representation of thallium ($Tl^+$)-based fluorescence assay principle (Invitrogen) and readout. 765 and 966 are chemical modulators used as control compounds. FIG. 6B shows flow cytometry analysis of tetracycline (Tet) inducible hKCNK9 stable cell line stained with Y1 and Y4 mAbs in the presence ($Tet^+$) or absence ($Tet^-$) of Tet induction. Parental HEK293 cells are used as negative control. FIG. 6C shows time-dependent inhibition of $Tl^+$ conductance by Y4 applied at 0.4 mg/ml. $Tet^+$ cells were treated with Y4, Y1 mAbs or PBS for 0.5-48 hours prior to $Tl^+$ assay and compared to cells without treatment. Difference in $Tl^+$ conductance at time point 80 is used for comparison. FIG. 6D shows representative $Tl^+$ traces from $Tet^+$ (solid line) and $Tet^-$ (dotted line) cells treated for 1, 6 or 36 hours plotted as fluorescence change over time. All data shown are mean±standard deviation, n=24, *p<0.0001. FIG. 6E shows concentration-dependent inhibition of $Tl^+$ conductance by Y4. $Tet^+$ cells are treated with Y4 and Y1 at 0.4 mg/ml or 0.04 mg/ml μM for 12-48 hours prior to $Tl^+$ assay. All data shown are mean±standard deviation, n=6, *p<0.01. FIG. 6F shows internalization of hKCNK9 from the cell surface induced by Y4. COS-7 cells transiently expressing hKCNK9 and mcherry or RFP-tagged EEA1 were incubated with Alexa488-conjugated Y4 for 3 hours at 4 °C or for 12 hours at 30 °C. Cells transiently expressing hKCNK3 and mcherry were analyzed in comparison. Nuclei are counterstained with DAPI. Co-localization between Alexa488-conjugated Y4 and RFP-tagged EEA1 was analyzed using Imaris software. Statistical analysis is based on Pearson's correlation coefficient (r>0.5 is significant). Bar: 20 μm;

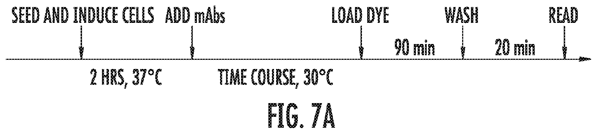

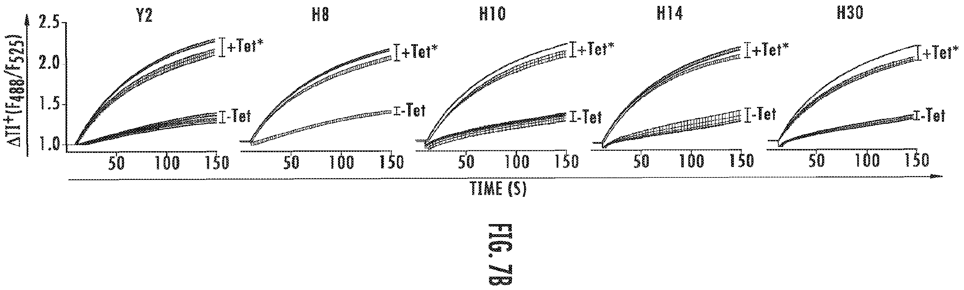

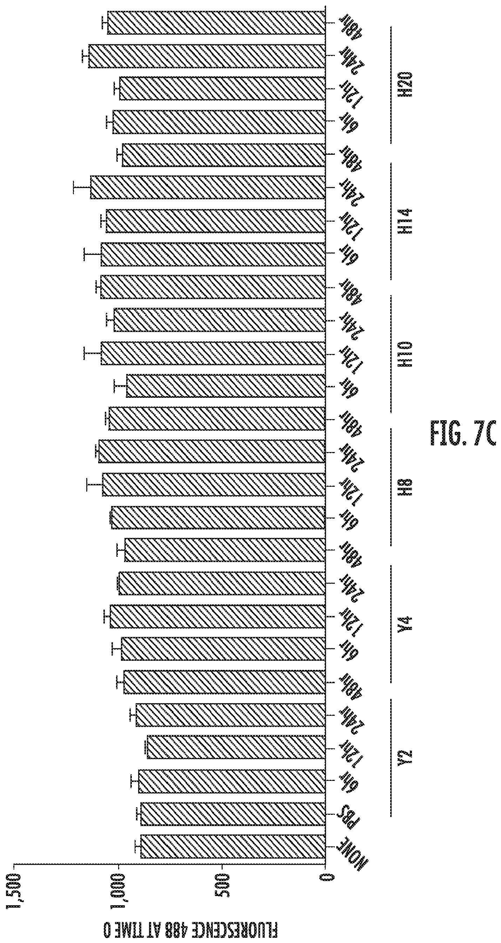

FIG. 7A, FIG. 7B, and FIG. 7C show that mAb-mediated inhibition of hKCNK9 is not due to changes in cell/channel number but channel activity. FIG. 7A shows an outline of Tl+ assay adapted for mAb screening. FIG. 7B shows that representative Tl+ traces upon 36-hour treatment of Y2, H8, H10, H14 and H30 mAbs also showed inhibition. FIG. 7C shows that fluorescence at time 0 did not show significant differences upon mAb incubation at indicated time periods suggesting no dramatic differences in cell number;

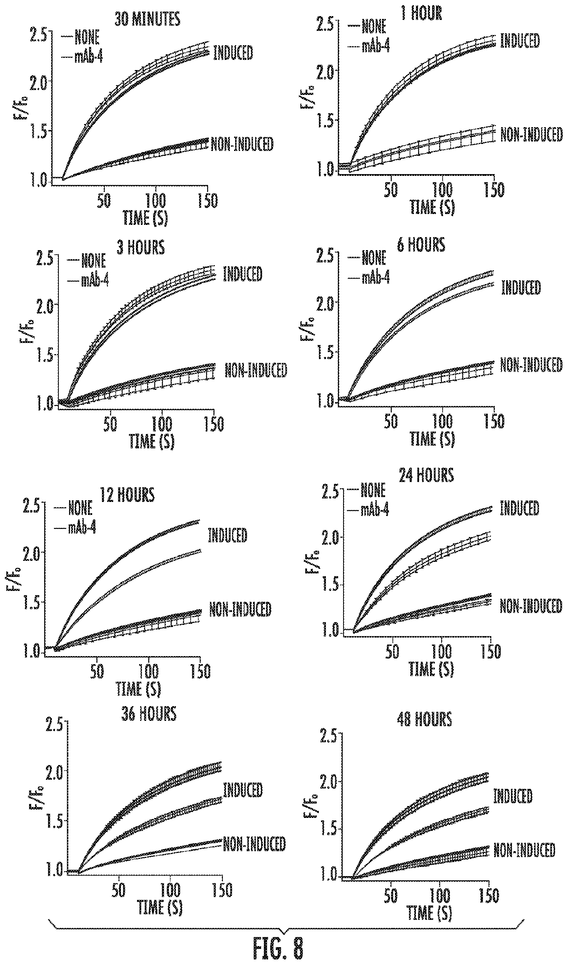

FIG. 8 shows representative Tl$^+$ traces from a Y4 time course experiment. Representative Tl$^+$ traces from and Tet$^-$ cells treated for 0.5-24 hours plotted as fluorescence change over time;

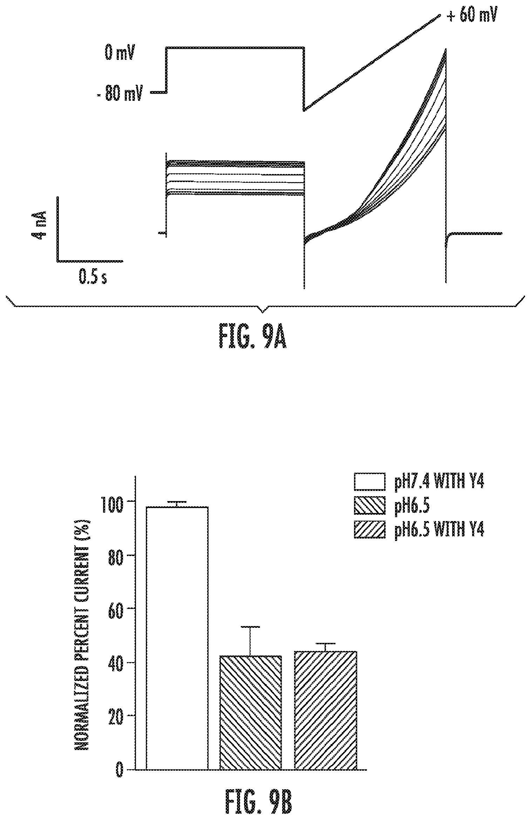

FIG. 9A and FIG. 9B show that Y4 showed no significant acute effects in electrophysiological recording. FIG. 9A shows a electrophysiological patch clamp recording protocol used to examine Y4's acute effects on hKCNK9. FIG. 9B shows quantification of hKCNK9 channel conductance in the presence of Y4. M1P1 domain is involved in pH sensing by hKCNK9. Two different pH conditions were used to find out if Y4 binding to M1P1 domain alters hKCNK9's sensitivity to extracellular pH;

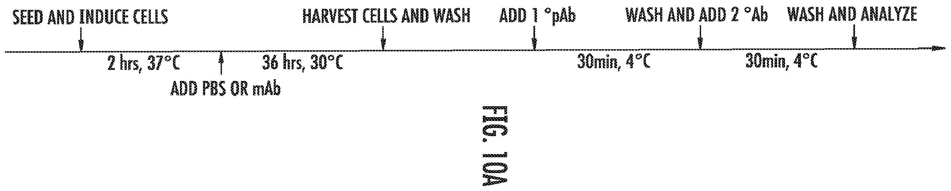

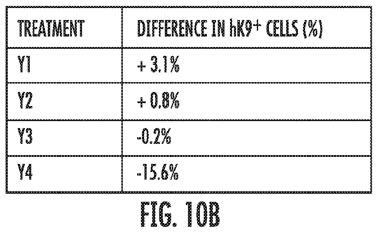

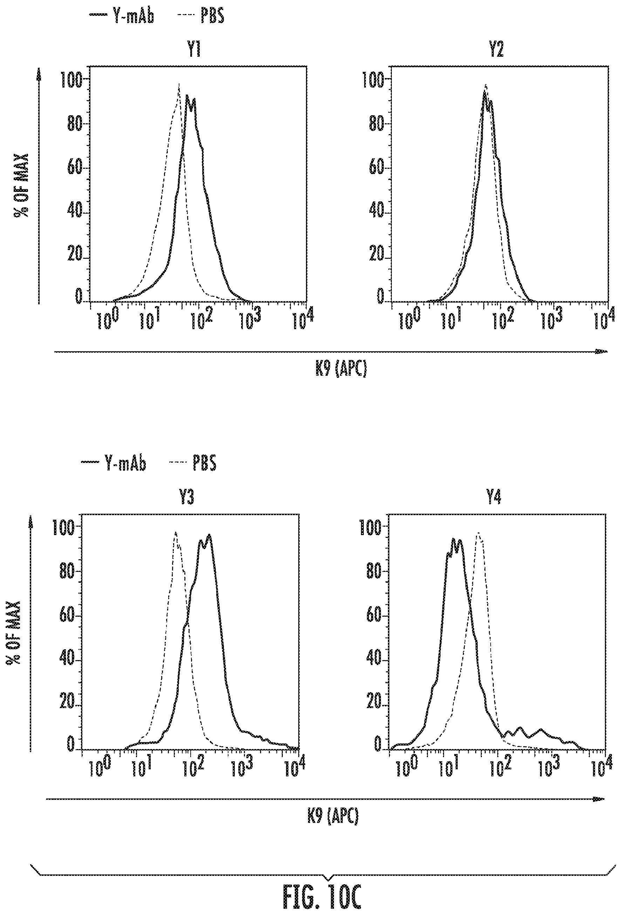

FIG. 10A, FIG. 10B, and FIG. 10C show that Y4 reduced the number of hKCNK9 channels expressed on the cell surface. FIG. 10A shows that an outline of staining and flow cytometry analysis set up to examine hKCNK9 remained on the cell surface after Y-mAbs' incubation. FIG. 10B shows a summary of changes in the absolute percentage of cells stained positive for hKCNK9. FIG. 10C shows representative histograms showing hKCNK9 staining which showed a significant left shift (reduction) of average fluorescence intensity;

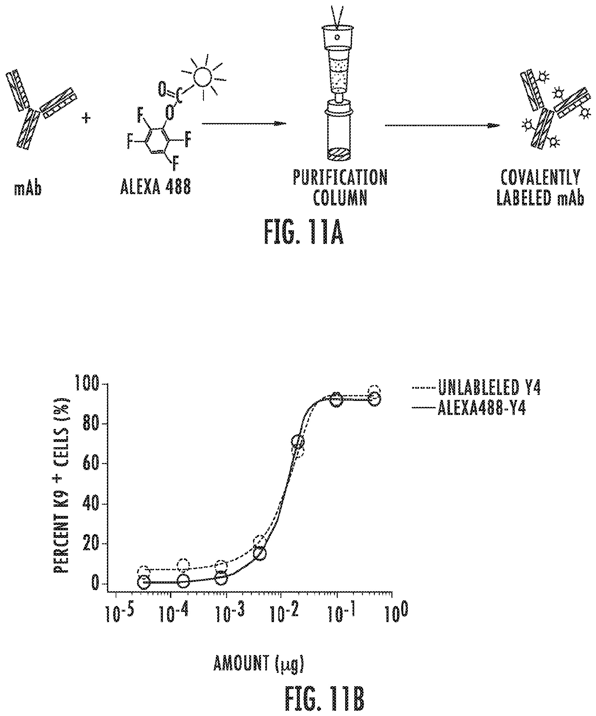

FIG. 11A and FIG. 11B show that conjugation of Y4 with Alexa488 did not alter the binding affinity of cell surface antigen. FIG. 11A shows a schematic of Y4 conjugation (Life technologies). FIG. 11B shows Tet-induced hKCNK9 stable cell line stained with either unlabeled Y4 or Alexa488-labeled Y4 at indicated dilutions followed by APC-labeled anti-mIgG secondary antibody and flow cytometry analysis. Percentage of K9$^+$ cells was plotted over mAb dose;

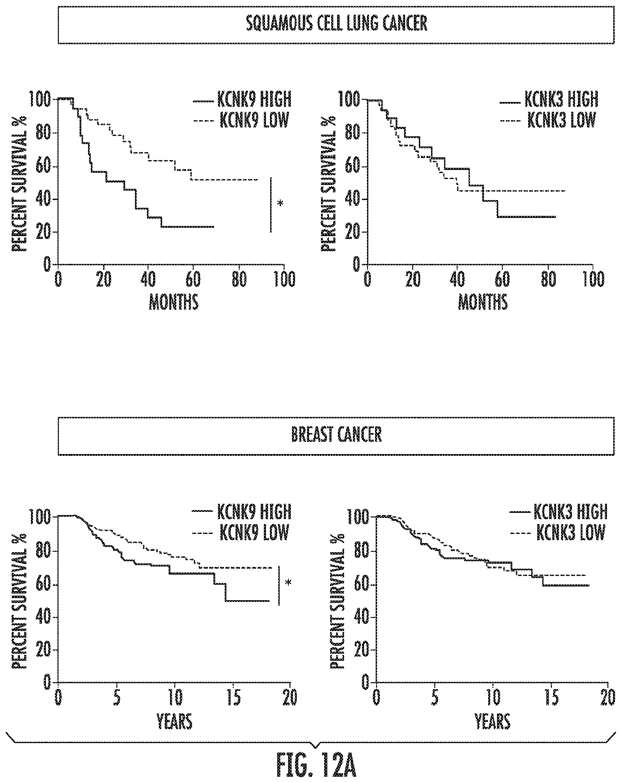

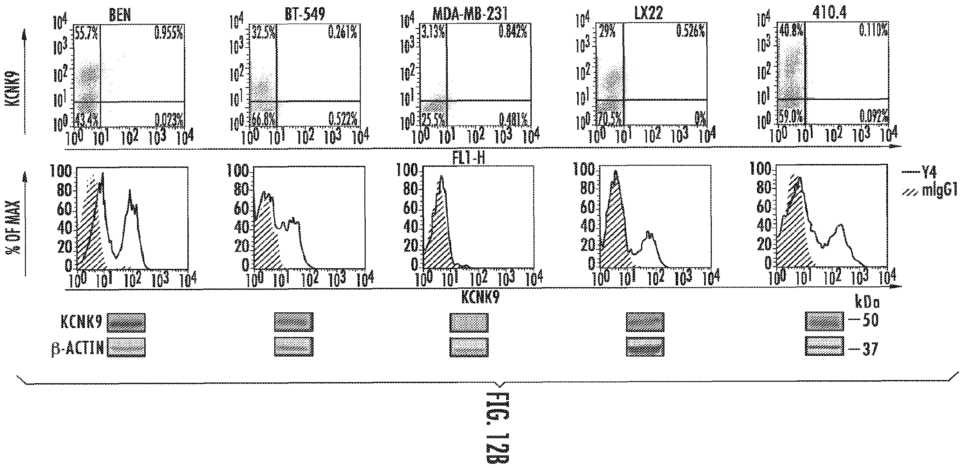

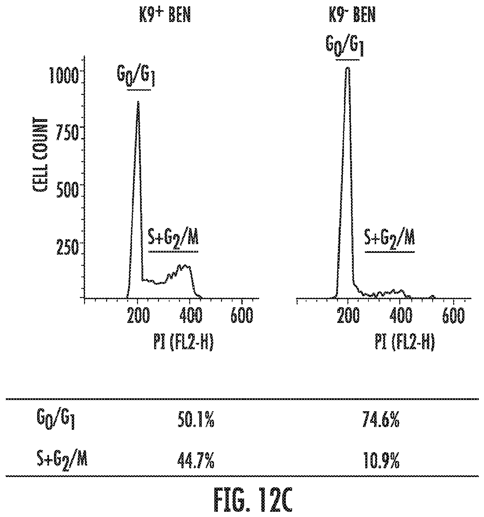

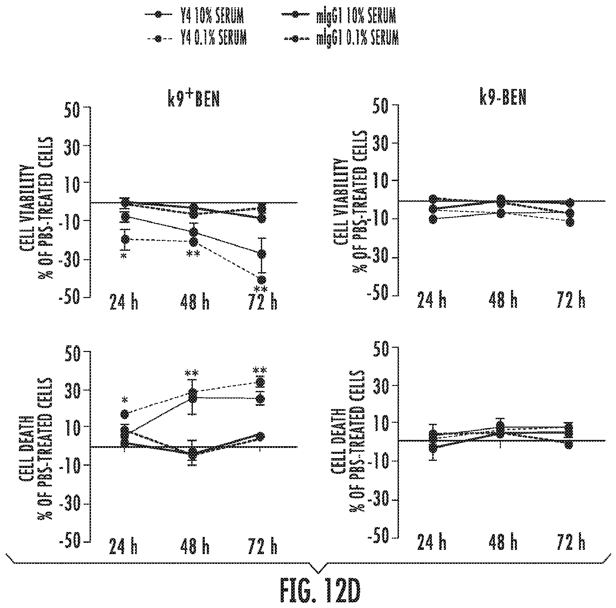

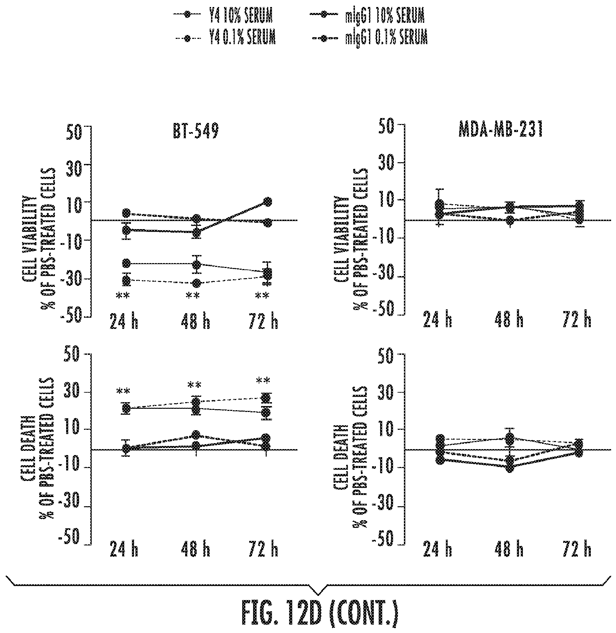

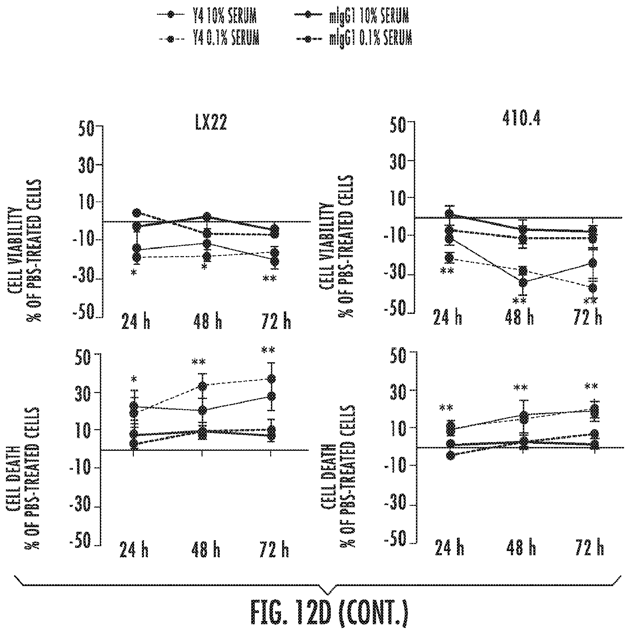

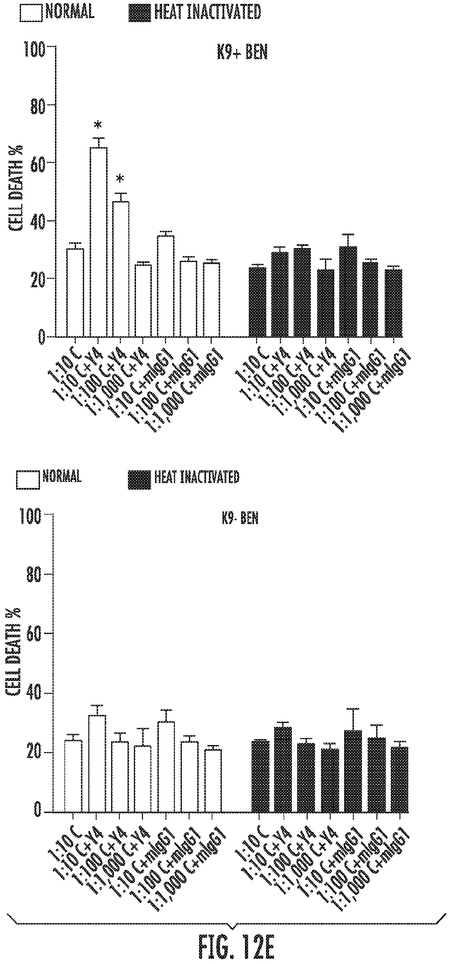

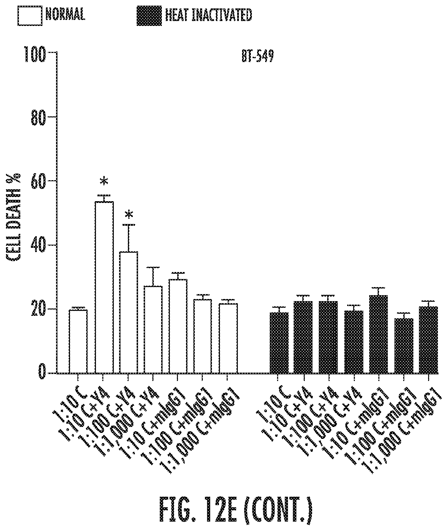

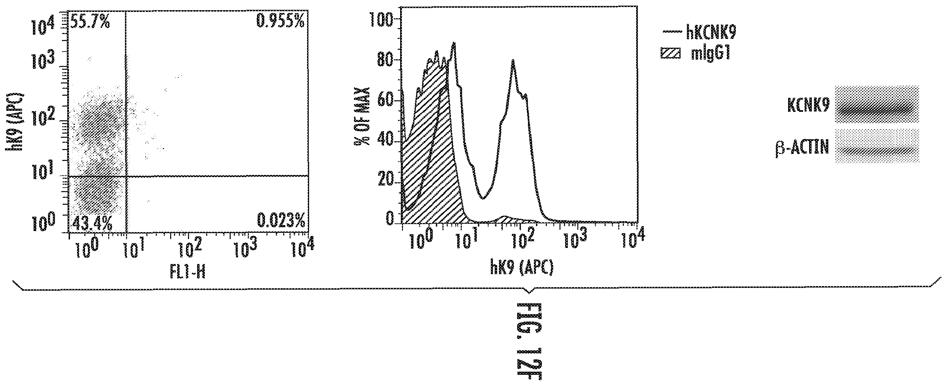

FIG. 12A, FIG. 12B, FIG. 12C, FIG. 12D, and FIG. 12E show that Y4 inhibits survival of cancer cells with high expression of KCNK9. FIG. 12A shows Kaplan-Meier curves for survival of patients with squamous cell lung cancer and breast cancer in relation to hKCNK9 or hKCNK3 gene expression level (two-fold higher or lower than mean). Datasets are from published studies (van de Vijver et al. (2002) New England Journal of Medicine 347, 1999-2009; Bild et al. (2006) Nature 439, 353-357). Patients' gene expression data and clinical data were manually matched, and only patients with both valid gene expression data and clinical data were included for analysis. *$p<0.05$ based on Log-rank (Mantel-Cox) test. FIG. 12B shows Y4's ability to detect endogenous KCNK9 expressed in cancer cell lines as verified by flow cytometry and western blotting analyses. FIG. 12C shows cell cycle analysis of K9$^+$ and K9$^-$ BEN cells based on DNA content. G$_0$/G$_1$: 2N, S: 2N~4N, G$_2$/M: 4N. FIG. 12D shows that Y4 reduces cell viability and increases cell death of KCNK9-expressing cancer cell lines. BEN cells sorted according to hKCNK9 expression level (K9$^+$ or K9$^-$), BT-549, MDA-MB-231, and 410.4 cells were treated with 0.4 mg/ml mIgG1 or Y4 in the presence of 10% serum or 0.1% serum for 24-72 hours. All data shown are mean±standard deviation, n=6, *$p<0.01$ at 0.1% heat-inactivated (HI) serum; **$p<0.01$ at 0.1% and 10% serum, two-tailed Student's *t*-test. FIG. 12E shows that Y4 activates complement, resulting in complement-dependent cancer cell cytotoxicity *in vitro*. K9$^+$ sorted BEN cells and BT-549 cells are treated with normal or heat inactivated murine complement in the presence of 0.4 mg/ml mIgG1 or Y4 for 3 hours before LDH measurement. All data shown are mean±standard deviation, n=6, *$p<0.05$;

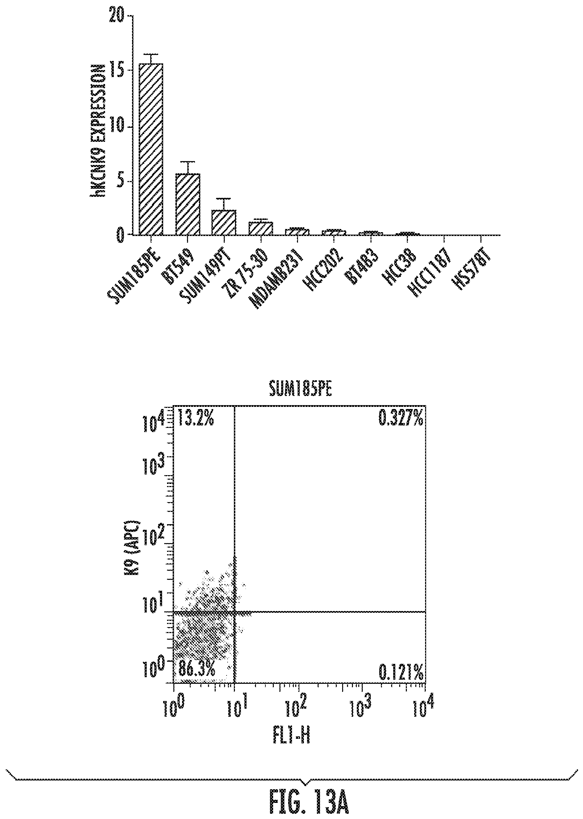

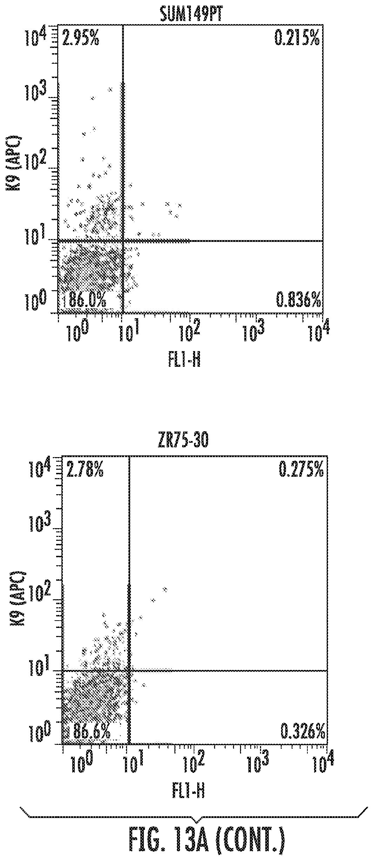

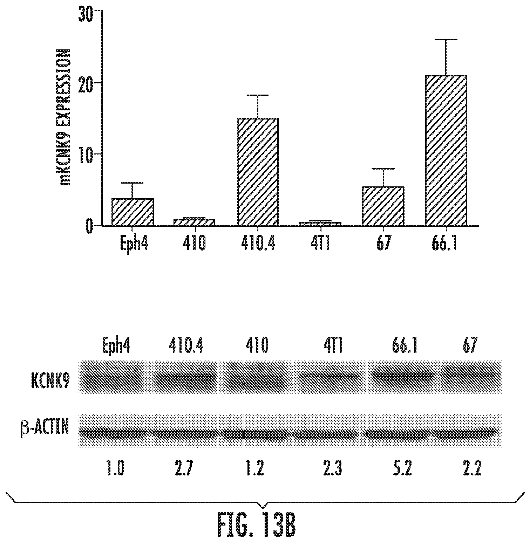

FIG. 13A and FIG. 13B show KCNK9 expression in human and murine cancer cell lines. FIG. 13A shows hKCNK9 expression verified by qRT-PCR and flow cytometry analysis. FIG. 13B shows mKCNK9 expression verified by qRT-PCR and western blot analysis. Densitometry quantification is shown below the blot. 66.1, 4T1, 410.4 are murine metastatic breast cancer cell lines; 67 and 410 are murine non-metastatic cell lines; EpH4 is a murine normal mammary cell line;

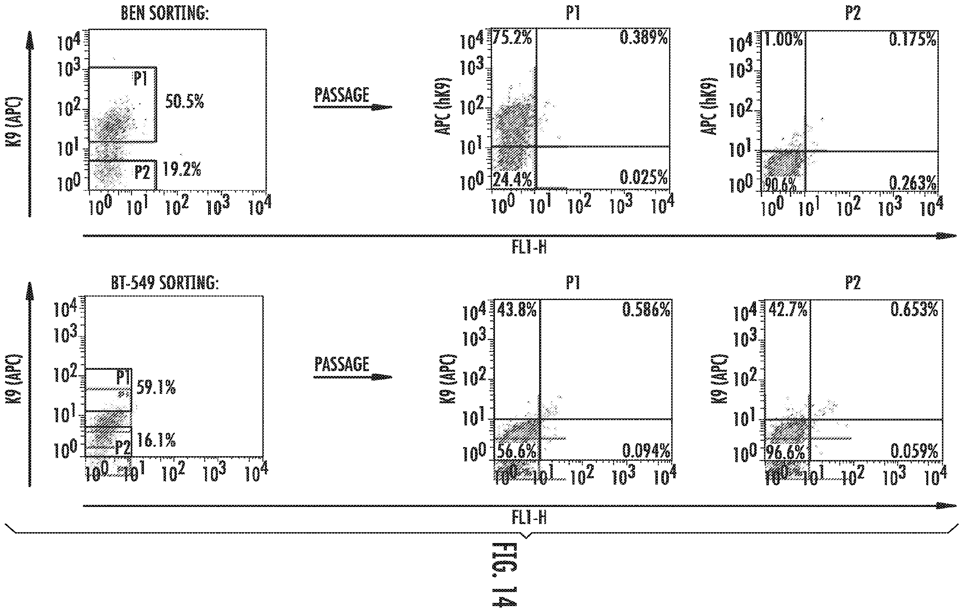

FIG. 14 shows that K9$^-$ BEN cells were stable after passaging *in vitro*. BEN and BT-549 cells were FACS-sorted based on hKCNK9 expression giving rise to PI (K9$^+$) and P2 (K9$^-$) cells. After being passaged three times *in vitro*, they were stained and analyzed by flow cytometry;

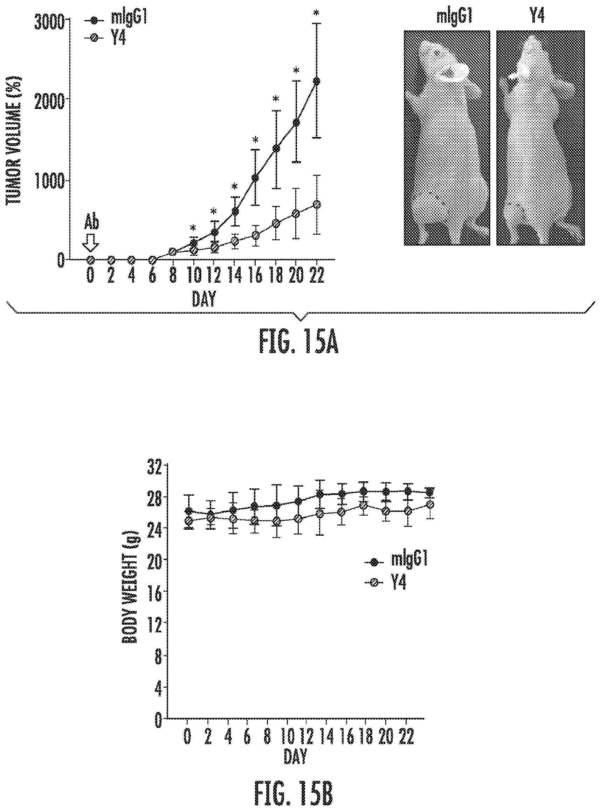

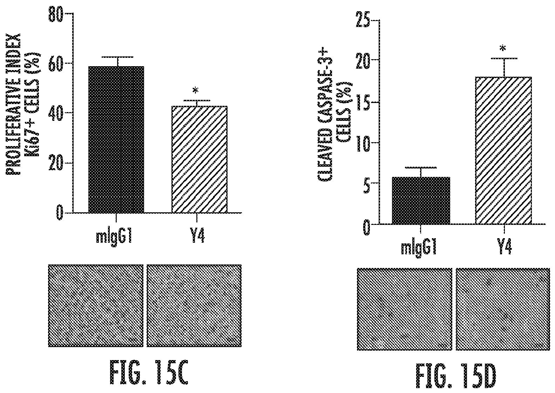

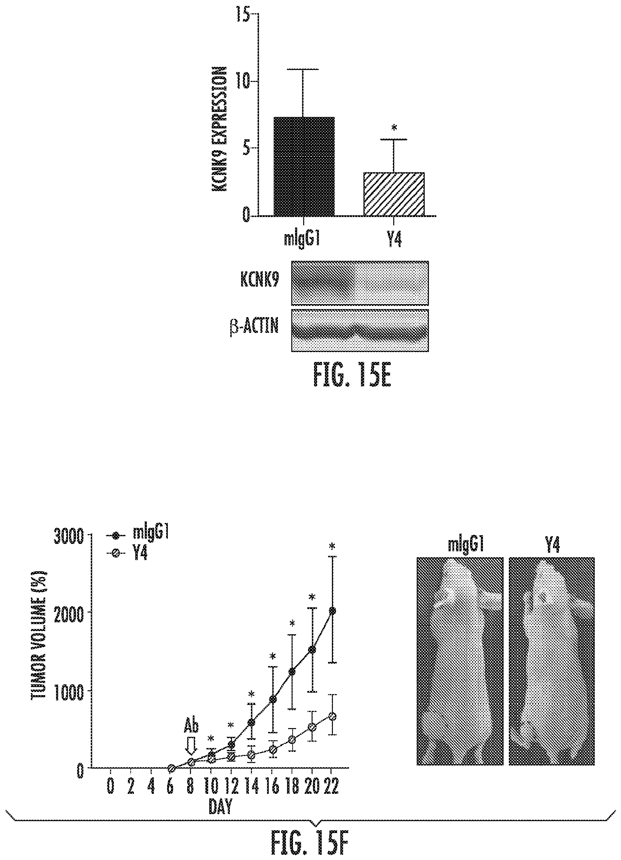

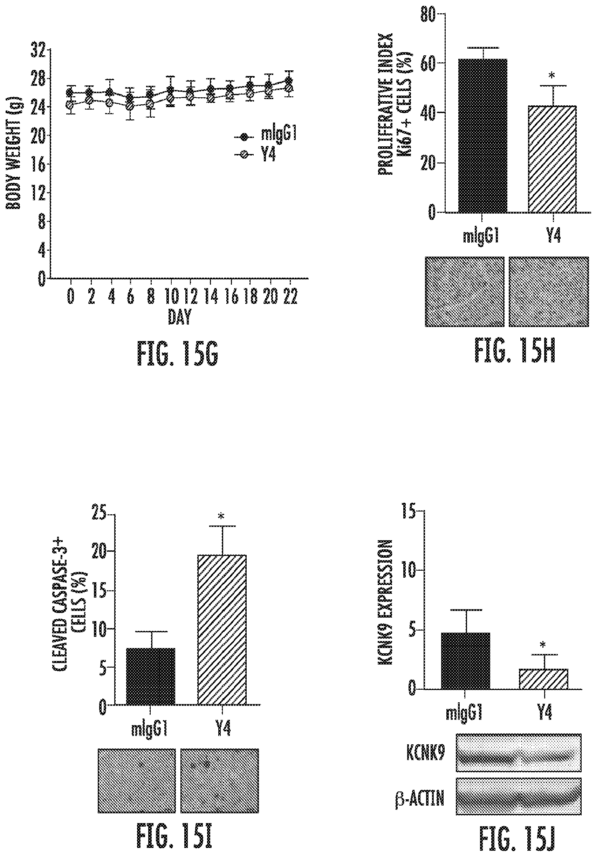

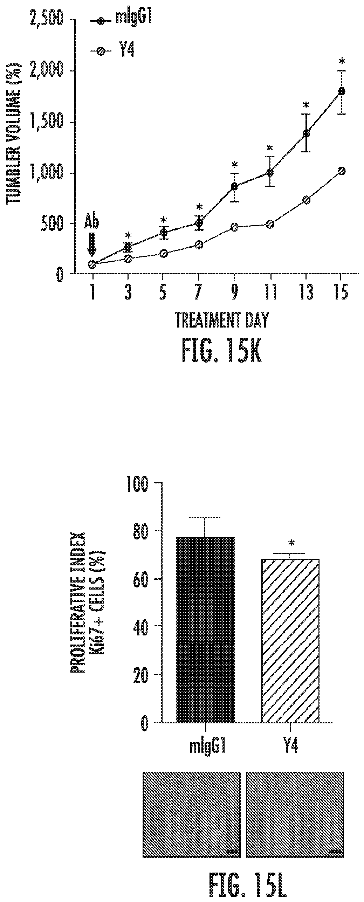

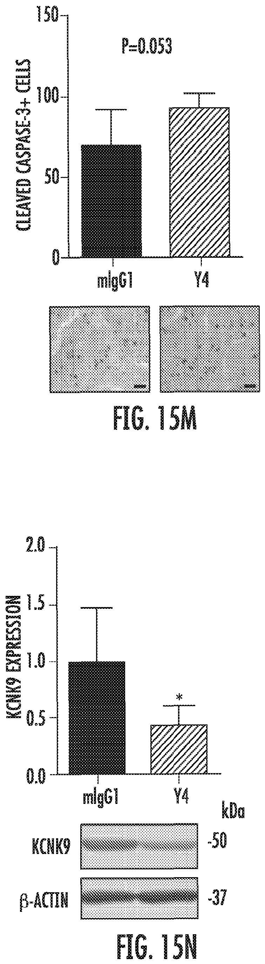

FIG. 15A, FIG. 15B, FIG. 15C, FIG. 15D, FIG. 15E, FIG. 15F, FIG. 15G, FIG. 15H, FIG. 15I, FIG. 15J, FIG. 15K, FIG. 15L, FIG. 15M, and FIG. 15N show that systemic Y4 inhibits the capacity of BEN cells to propagate subcutaneous tumor xenografts in immunodeficient mice. FIG. 15A shows growth curves of BEN engraftments treated with 4 mg/kg mIgG1 or Y4 injected into NOD/SCID mice with PBS i.p. twice a week for 22 days (n=10), *$p<0.01$. mAb treatment starts on the same day of cell injection to monitor mAbs' effects during early tumor establishment. Representative photographs showing tumors (dotted line) formed in NOD/SCID mice on day 22. FIG. 15B shows body weights of mice treated with mIgG1 or Y4 (n=10). FIG. 15C shows quantitative determination of Ki67$^+$ cells in mIgG1 or Y4 treated group and representative photographs of tumor cross-sections showing Ki67 staining. FIG. 15D shows quantitative determination of cleaved caspase-3$^+$ cells in mIgG1 or Y4 treated group and representative photographs of tumor cross-sections showing cleaved caspase-3 staining. All data shown are mean±standard deviation, n=30, *$p<0.01$. FIG. 15E shows quantitative determination of hKCNK9 in mIgG1 or Y4 treated group and representative western blotting of tumor tissues with Y4. All data shown are mean±standard deviation, n=10, *$p<0.05$. FIG. 15F shows growth curves of BEN engraftments treated with 4 mg/kg mIgG1 or Y4 injected into NOD/SCID mice i.p. with PBS twice a week for 14 days (n=10), *$p<0.01$. mAb treatment starts after tumor is measurable on day 8. Representative photographs showing tumors (dotted line) formed in NOD/SCID mice on day 22. FIG. 15G shows body weights of mice treated with mIgG1 or Y4 (n=10). FIG. 15H shows quantitative determination of Ki67$^+$ cells in mIgG1 or Y4 treated group and representative photographs of tumor cross-sections showing Ki67 staining. FIG. 15I shows quantitative determination of cleaved caspase-3[+] cells in mIgG1 or Y4 treated group and representative photographs of tumor cross-sections showing cleaved caspase-3 staining. All data shown are mean±standard deviation, n=30, *p<0.01. FIG. 15J shows quantitative determination of hKCNK9 in mIgG1 or Y4 treated group and representative western blotting of tumor tissues with Y4. All data shown are mean±standard deviation, n=10, *p<0.05. FIG. 15K shows growth curves of LX22 patient-derived xenograft (PDX) engraftments in NSG mice treated with mIgG1 or Y4 i.p twice a week for 15 days. Mean± s.e.m., n=10 per group, *P<0.01, two-tailed Student's $t$-test. FIG. 15L shows quantitative determination of Ki67[+] cells and representative staining of tumor cross-sections. Mean±standard deviation, n=30 per group, *P<0.05, two-tailed Student's t-test; sale bar, 1,000 μm. FIG. 15M shows quantitative determination of cleaved caspase-3[+] cells and representative staining of tumor cross-sections. Mean±standard deviation, =30 per group; scale bar, 1,000 μm. FIG. 15N shows quantitative determination of hKCNK9 and representative western blotting of tumor tissues. Mean±standard deviation, n=10 per group, *P<0.05, two-tailed Student's $t$-test;

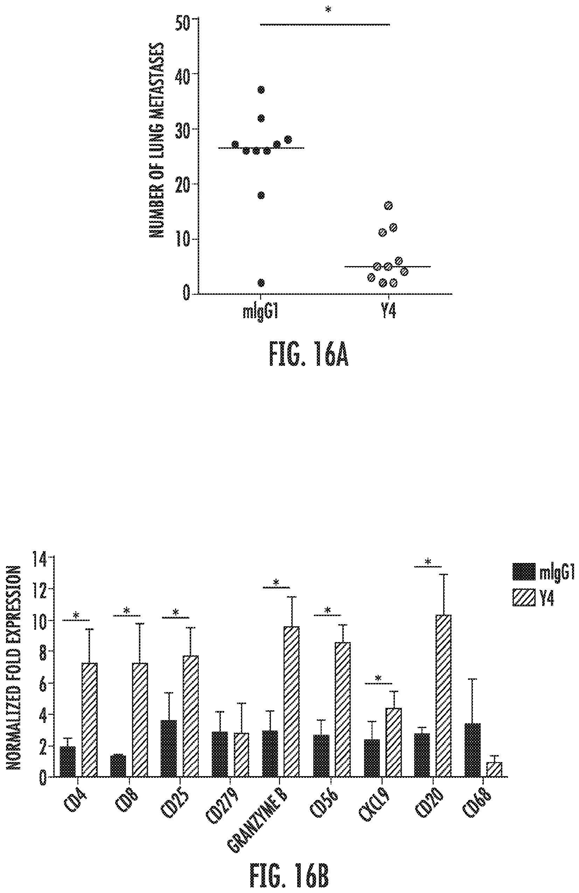

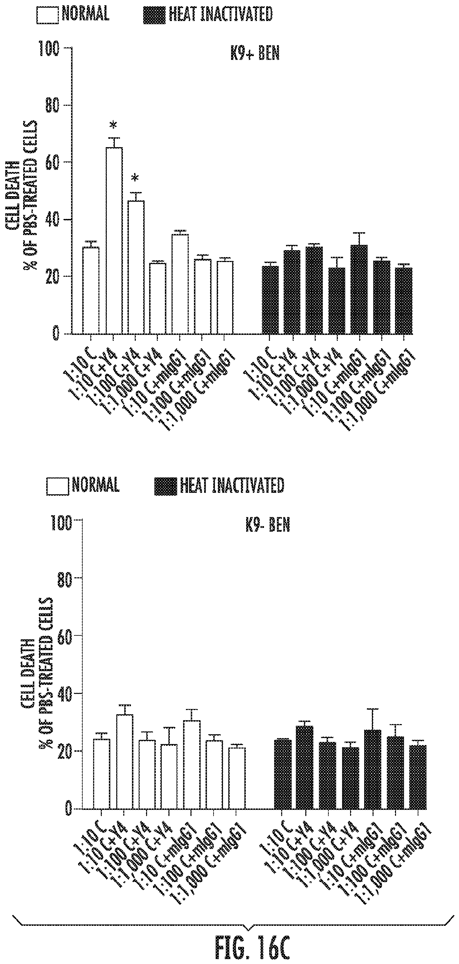

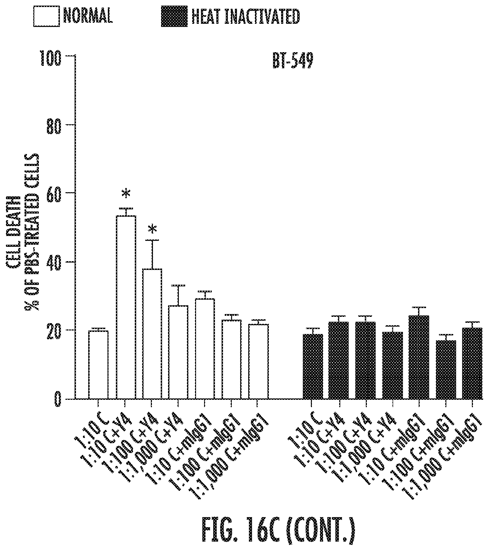

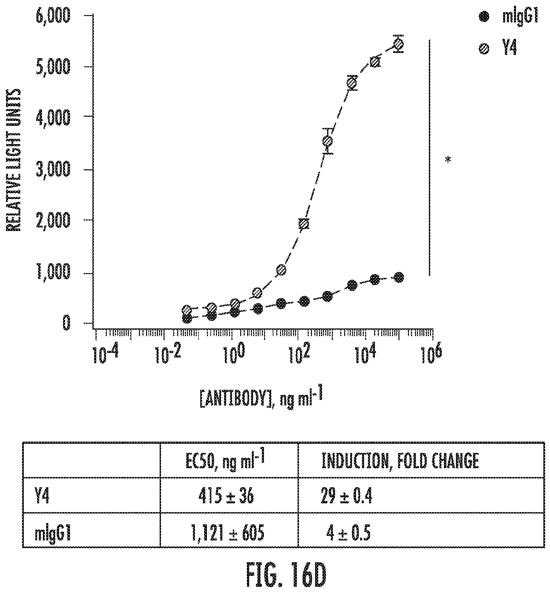

FIG. 16A, FIG. 16B, FIG. 16C, and FIG. 16D show that Y4 inhibits 410.4 cells' lung metastasis and activates immune responses in immunocompetent mice. FIG. 16A shows the number of lung metastasis foci in BALB/cByJ mice treated with 4 mg/kg mIgG1 or Y4 injected i.p. with PBS twice a week (n=10), *p<0.01. FIG. 16B shows expression of immune markers verified by qRT-PCR of mRNAs extracted from lung tissues of mIgG1 or Y4-treated mice (n=5), *p<0.05. FIG. 16C shows Y4 activates complement, resulting in complement-dependent cytotoxicity $in$ $vitro$. K9[+] BEN cells and BT-549 cells were treated with Y4 for 2 hours in the presence of either normal or heat-inactivated murine complement before LDH measurement. Mean±standard deviation, n=6, *P<0.05, two-tailed Student's $t$-test. FIG. 16D shows Y4 activates ADCC reporter cells. BEN cells were incubated with different doses of Y4 and mIgG1, and engineered murine Jurkat T cells stably expressing FCyRIII and an NFAT-response element that drives luciferase expression (NFAT-RE-luc2). Measured luciferase activity indicates ADCC induction. Mean±standard deviation, n=3, *P<0.01, two-tailed Student's $t$-test; and

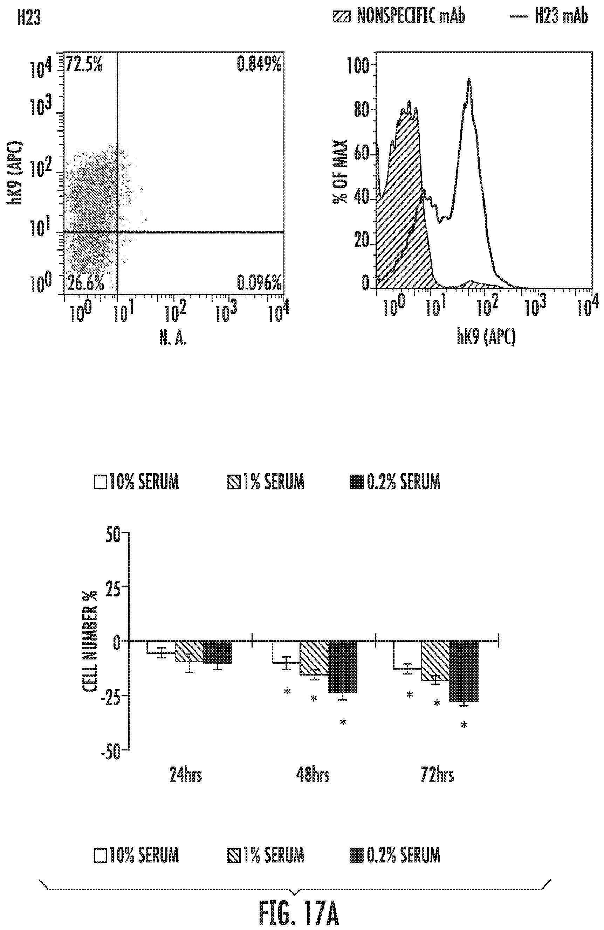

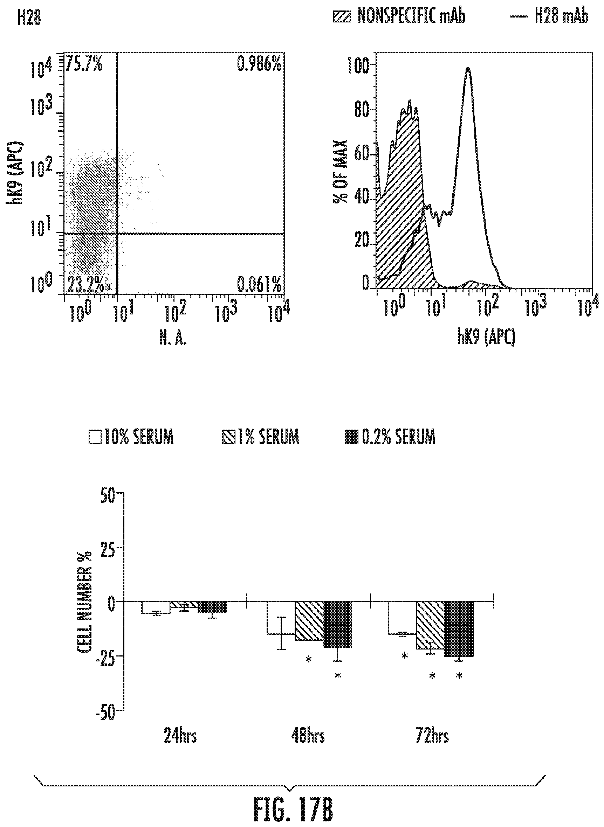

FIG. 17A and FIG. 17B show cell viability assays using mAbs H23 (FIG. 17A) and H28 (FIG. 17B).

The patent or application file contains at least one drawing executed in color. Copies of this patent or patent application publication with color drawings will be provided by the Office upon request and payment of the necessary fee.

DETAILED DESCRIPTION

The invention is as defined in the claims.

The presently disclosed subject matter relates to antibodies, antibody fragments or derivatives thereof, which specifically bind to and/or interact with at least one epitope of the extracellular domain of the mammalian KCNK9 potassium channel, and to nucleic acid molecules encoding the same, as well as to vectors comprising said nucleic acid molecules. The presently disclosed subject matter provides methods for the preparation of said antibodies, antibody fragments or derivatives thereof, as well as pharmaceutical compositions comprising the same. The presently disclosed subject matter further provides diagnostic compositions and kits comprising the antibodies, antibody fragments or derivatives thereof. In some aspects, the presently disclosed subject matter provides a method for assessing for the presence of KCNK9 expressing cells as claimed in claim 10. In certain aspects disclosed, but not claimed, the presently disclosed subject matter provides methods of inhibiting KCNK9 activity in cells. In some aspects disclosed, but not claimed, the presently disclosed subject matter provides methods of inhibiting the growth or survival of KCNK9 expressing cells (e.g., cancer cells). In other aspects disclosed, but not claimed, the presently disclosed subject matter involves the use of the antibodies, antibody fragments or derivatives thereof in methods for inhibiting growth and/or metastases of tumors, stimulating complement-dependent cancer cell cytotoxicity, and related methods for the treatment of cancer (e.g., KCNK9 expressing cancers).

The presently disclosed subject matter now will be described more fully hereinafter with reference to the accompanying Figures, in which some, but not all embodiments of the inventions are shown. Like numbers refer to like elements throughout. The presently disclosed subject matter may be embodied in many different forms and should not be construed as limited to the embodiments set forth herein; rather, these embodiments are provided so that this disclosure will satisfy applicable legal requirements. Indeed, many modifications and other embodiments of the presently disclosed subject matter set forth herein will come to mind to one skilled in the art to which the presently disclosed subject matter pertains having the benefit of the teachings presented in the foregoing descriptions and the associated Figures. Therefore, it is to be understood that the presently disclosed subject matter is not to be limited to the specific embodiments disclosed and that modifications and other embodiments falling within the scope of the appended claims are included.

Potassium channels are ubiquitously present in cells. One reason for this is supposed to be that the channels are involved in the regulation of the resting potential of cells, which has been regarded as their major role. However, given the ubiquitous presence of the channels in different cell types, it has been postulated that they might also be involved in more general functions, such as "housekeeping" functions. In particular, experimental evidence suggests their implication in the cell division cycle [Ouadid-Ahidouch H et al., 2001], hinting at their possible involvement in cancerogenesis. Indeed, KCNK9 has been implicated in cancer based on genetic evidence including genomic amplification, mRNA and protein over-expression in human breast tumors and lung tumors (Mu et al. (2003) Cancer Cell 3, 297-302). Since such channels are also expressed in various cell types, particularly in dividing cells, including cancer cells, such as neoplastic cells, it is of great medical interest to provide tools which might be used in therapeutic and/or diagnostic applications related to potassium channels.

Currently, there exists no monoclonal antibody that specifically recognizes the extracellular domain of hKCNK9. Similarly, there is no subtype-specific pharmacological agent that effectively inhibits KCNK9's biologic functions. In order to further broaden diagnostic and/or therapeutic applications it is desirable to have antibodies that specifically discriminate between mammalian, in particular human, KCNK9 and other two-pore domain potassium (K2P) channel subtypes, such as KCNK3, among the K2P members most closely related to KCNK9.

Thus, the technical problem underlying the presently disclosed subject matter was to provide such antibodies which may be employed for the further specific study, diagnosis, prevention and treatment of defects and/or diseases interrelated with KCNK9 activity from different mammalian species, and in particular hKCNK9 activity.

The solution to said technical problem is achieved by providing the presently disclosed subject matter, including the embodiments characterized in the claims.

Accordingly, the presently disclosed subject matter provides an antibody or antigen-binding fragment thereof as claimed in claim 1 that specifically binds to at least one epitope of the extracellular domain of the KCNK9 potassium channel. In some embodiments, the antibody, antibody fragment, or derivative thereof does not bind to other potassium channels, such as K2P family members KCNK1, KCNK2, KCNK3, KCNK4, KCNK5, KCNK6, KCNK7, KCNK8, KCNK10, KCNK11, KCNK12, KCNK13, KCNK14 and KCNK15. As used herein, "M1P1 domain" refers to the first extracellular domain of mammalian KCNK9 that resides between the M1 domain and PI domain of KCNK9 (amino acids 30~88). The at least one epitope comprises an epitope between amino acids $Glu_{30}$ and $Ile_{88}$ of human potassium channel subfamily K member KCNK9 (hKCNK9) protein (SEQ ID NO: 1).

MKRQNVRTLSLIVCTFTYLLVGAAVFDALESDHEMREEEKLKAEEIR

IKGKYNISSEDYRQLELVILQSEPHRAGVQWKFAGSFYFAITVITTIGYGHAAP

GTDAGKAFCMFYAVLGIPLTLVMFQSLGERMNTFVRYLLKRIKKCCGMRNT

DVSMENMVTVGFFSCMGTLCIGAAAFSQCEEWSFFHAYYYCFITLTTIGFGD

YVALQTKGALQKKPLYVAFSFMYILVGLTVIGAFLNLVVLRFLTMNSEDERR

DAEERASLAGNRNSMVIHIPEEPRPSRPRYKADVPDLQSVCSCTCYRSQDYGG

RSVAPQNSFSAKLAPHYFHSISYKIEEISPSTLKNSLFPSPISSISPGLHSFTDHQR

LMKRRKSV (SEQ ID NO: 1)

In some embodiments disclosed, but not claimed, the at least one epitope comprises an epitope that is at least 80%, at least 85%, at least 90%, at least 91%, at least 92%, at least 93%, at least 94%, at least 95%, at least 96%, at least 97%, at least 98%, or at least 99% identical to SEQ ID NO: 1.

"Sequence identity" or "identity" in the context of proteins or polypeptides refers to the amino acid residues in two amino acid sequences that are the same when aligned for maximum correspondence over a specified comparison window. Thus, "percentage of sequence identity" refers to the value determined by comparing two optimally aligned sequences over a comparison window, wherein the portion of the amino acid sequence in the comparison window may comprise additions or deletions (*i.e.*, gaps) as compared to the reference sequence (which does not comprise additions or deletions) for optimal alignment of the two sequences. The percentage is calculated by determining the number of positions at which the identical amino acid residue occurs in both sequences to yield the number of matched positions, dividing the number of matched positions by the total number of positions in the window of comparison and multiplying the results by 100 to yield the percentage of sequence identity. Useful examples of percent sequence identities include, but are not limited to, 50%, 55%, 60%, 65%, 70%, 75%, 80%, 85%, 90%, or 95%, or any integer percentage from 50% to 100%. These identities can be determined using any of the programs described herein.

Sequence alignments and percent identity or similarity calculations may be determined using a variety of comparison methods designed to detect homologous sequences including, but not limited to, the MegAlign™ program of the LASERGENE bioinformatics computing suite (DNASTAR Inc., Madison, Wis.). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized. The "Clustal V method of alignment" corresponds to the alignment method labeled Clustal V (described by Higgins and Sharp (1989) CABIOS 5:151-153; Higgins et al. (1992) Comput. Appl. Biosci. 8:189-191) and found in the MegAlign™ program of the LASERGENE bioinformatics computing suite (DNASTAR Inc., Madison, Wis.).

It is well understood by one skilled in the art that many levels of sequence identity are useful in identifying proteins or polypeptides (e.g., from other species) wherein the proteins or polypeptides have the same or similar function or activity. Useful examples of percent identities include, but are not limited to, 50%, 55%, 60%, 65%, 70%, 75%, 80%, 85%, 90%, or 95%, or any integer percentage from 50% to 100%. Indeed, any integer amino acid identity from 50% to 100% may be useful in describing the present presently disclosed subject matter, such as 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%.

The presently disclosed antibodies demonstrate advantageous properties with respect to their binding specificity and biological activity. For example, the presently disclosed subject matter demonstrates that the antibodies not only recognize the human KCNK9 potassium channel, but also are able to recognize KCNK9 potassium channels of other mammalian species, such as mouse KCNK9. In some embodiments, the presently disclosed antibodies exhibit one or more of the following characteristics:

- binding to a 3-dimensional or linear epitope in the extracellular pore-domain/binding to the extracellular domain;
- binding with high affinity; and/or
- binding with high specificity.

The presently disclosed antibodies thus have the advantage that they can be used in the specific detection of KCNK9 over a broad range of experimental animals as well as for human tissue. Costs for the production of antibodies recognizing KCNK9 in different species may thus be decreased.

The antibodies of the presently disclosed subject matter allow the specific recognition of mammalian KCNK9 potassium channels both in vitro and in vivo.

In some embodiments, the presently disclosed KCNK9 antibodies exhibit one or more of the following characteristics:

- inhibiting K+ channel mediated current;
- resulting in internalization and endocytosis of ion channels;
- interfering with subunit assembly of ion channels;
- decreasing the release or activation of second messengers;
- decreasing or inhibiting cell growth;
- increasing cell death and/or
- interfering with the formation of ion channel homo-/heteromultimers.

In some embodiments, the antibody or antigen-binding fragment thereof inhibits KCNK9 activity as measured using an ion-influx assay. In some contexts used herein, "KCNK9 activity" refers to potassium channel conductance. As used herein, the term "reduce" or "inhibit," and grammatical derivations thereof, refers to the ability of an agent to block, partially block, interfere, decrease, reduce or deactivate a biological molecule, pathway or mechanism of action. Thus, one of ordinary skill in the art would appreciate that the term "inhibit" encompasses a complete and/or partial loss of activity, e.g., a loss in activity by at least 10%, in some embodiments, a loss in activity by at least 20%, 30%, 50%, 75%, 95%, 98%, and up to and including 100%. In some embodiments, the antibody or antigen-binding fragment thereof inhibits KCNK9 mediated potassium channel conductance by as much as 10%, 20%, 30%, 40%, 50% or more. In some embodiments, the antibody or antigen-binding fragment thereof inhibits KCNK9 mediated potassium channel conductance by as much as 60%, 70%, 80%, 90%, 95% or more. In some embodiments, the antibody or antigen-binding fragment thereof completely inhibits KCNK9 mediated potassium channel conductance in a cell.

Binding of the antibody or antigen-binding fragment thereof to the at least one epitope on the surface of a cell that expresses KCNK9 causes internalization and endocytosis of potassium channels on the surface of the cell. That is, the antibodies or antigen-binding fragment thereof are capable of causing internalization of KCNK9 potassium channels in KCNK9 expressing cells. In some embodiments, binding of the antibody or antigen-binding fragment thereof to at least one epitope on the surface of a KCNK9-expressing cell inhibits survival of the KCNK9-expressing cell. In some embodiments, the antibody or antigen-binding fragment thereof inhibits survival of KCNK9-expressing cells in a population of cells, such as a tumor. In some embodiments, the antibody or antigen-binding fragment thereof inhibits survival of at least 10%, at least 20%, at least 30%, at least 40%, at least 50%, at least 60%, at least 70%, or at least 75% of KCNK9-exprssing cells in a bulk tumor (e.g., a breast tumor, lung tumor, etc.). In some embodiments, the antibody or antigen-binding fragment thereof inhibits survival of at least 85%, at least 90%, at least 95%, at least 96%, at least 97%, at least 98%, or at least 99% of KCNK9-exprssing cells in a bulk tumor (e.g., a breast tumor, lung tumor, etc.). In some embodiments, the antibody or antigen-binding fragment thereof inhibits survival of all KCNK9-exprssing cells in a bulk tumor (e.g., a breast tumor, lung tumor, etc.).

In some embodiments, KCNK9 expressing cells which have bound the presently disclosed antibodies on the cell surface are attacked by immune system functions such as the complement system or cell mediated cytotoxicity. In some embodiments, the KCNK9-expressing cells comprise cancer cells. In some embodiments, the KCNK9-expressing cells comprise breast cancer cells. In some embodiments, the KCNK9-expressing cells comprise lung cancer cells. In some embodiments, the KCNK9-expressing cells are provided from a subject suffering from small cell lung cancer. In some embodiments, the KCNK9-expressing cells are provided from a subject suffering from non-small cell lung cancer

The presently disclosed antibodies demonstrate advantageous properties with respect to their binding specificity and biological activity, in particular with respect to their capacity to recognize epitopes of the KCNK9 potassium channel in various mammals, and to decrease cell growth. Since the pharmaceutical and/or diagnostic applications of the presently disclosed antibodies include, but are not limited to humans, the presently disclosed subject matter contemplates humanizing antibodies to minimize potential negative immunogenic side effects for use in humans. The term "humanized antibody", as used herein, is intended to include antibodies made by a non-human cell having variable and constant regions which have been altered to more closely resemble antibodies that would be made by a human cell. For example, by altering the non-human antibody amino acid sequence to incorporate amino acids found in human germline immunoglobulin sequences. The humanized antibodies of the presently disclosed subject matter may include amino acid residues not encoded by human germline immunoglobulin sequences (e.g., mutations introduced by random or site-specific mutagenesis in vitro or by somatic mutation in vivo), for example in the CDRs. The term "humanized antibody", as used herein, also includes antibodies in which CDR sequences derived from the germline of another mammalian species, such as a mouse, have been grafted onto human framework sequences.

In some embodiments, the KCNK9 potassium channel comprises a human KCNK9 potassium channel. In some embodiments, the antibody is a monoclonal antibody. Monoclonal antibodies can be prepared, for example, by the well-established techniques as originally described in Kohler and Milstein, Nature 256 (1975), 495, and Galfre, Meth. Enzymol. 73 (1981), 3, which comprise the fusion of mouse myeloma cells to spleen cells derived from immunized mammals with modifications developed by the art.

In some embodiments, the antibody is a humanized monoclonal antibody. In some embodiments, the antibody is Y4.

In some embodiments, the antibody or antigen-binding fragment thereof specifically binds to and/or interacts with at least one epitope of the extracellular domain of the mammalian KCNK9 potassium channel, and does not bind to and/or interact with the mammalian KCNK3 potassium channel. In some embodiments, the antibody or antigen-binding fragment thereof specifically binds to and/or interacts with at least one epitope of the extracellular domain of the human KCNK9 potassium channel, and does not bind to and/or interact with the human KCNK3 potassium channel. As used herein, the term "extracellular domain" is a term well-known in the art and relates to the portion of the KCNK9 potassium channel extending into the extracellular environment. This domain comprises, among others, amino acids 30-88 of the mammalian KCNK9 molecule.

In some embodiments, the antibody fragment or derivative thereof is a Fab-fragment, a F(ab$_2$)'-fragment, a single-chain antibody, a chimeric antibody antibody, a CDR-grafted antibody, a bivalent antibody-construct, a humanized antibody, a human, a synthetic antibody, or a chemically modified derivative thereof, a multispecific antibody, a diabody, a Fv-fragment, or another type of a recombinant antibody.

Fragments or derivatives of the presently disclosed antibodies directed to at least one epitope of the extracellular domain of KCNK9 can be obtained by using methods which are described, e.g., in Harlow and Lane "Antibodies, A Laboratory Manual", CSH Press, Cold Spring Harbor, 1988. When derivatives of said antibodies are obtained by the phage display technique, surface plasmon resonance as employed in the BIAcore system can be used to increase the efficiency of phage antibodies which bind to at least one epitope of the extracellular domain of KCNK9 (Schier, Human Antibodies Hybridomas 7 (1996), 97-105; Malmborg, J. Immunol. Methods 183 (1995), 7-13).

The presently disclosed subject matter contemplates using nucleic acid molecules, vectors and host cells to produce mutated KCNK9 antibodies. The antibodies may be mutated in the variable domains of the heavy and/or light chains to alter a binding property of the antibody. For example, a mutation may be made in one or more of the CDR regions to increase or decrease the Kd of the antibody for KCNK9, or to alter the binding specificity of the antibody. Techniques in site directed mutagenesis are well-known in the art. See, e.g., Sambrook et al. and Ausubel et al., supra. Furthermore mutations are made at an amino acid residue that is known to be changed compared to germline in a variable region of an KCNK9 antibody. The nucleic acid molecules may be mutated in one or more of the framework regions. A mutation may be made in a framework region or constant domain to increase the half-life of the KCNK9 antibody. See, e.g., WO 00/09560, published Feb. 24, 2000. A mutation in a framework region or constant domain may also be made to alter the immunogenicity of the antibody, to provide a site for covalent or non-covalent binding to another molecule, or to alter such properties as complement fixation. Mutations may be made in each of the framework regions, the constant domain and the variable regions in a single mutated antibody. Alternatively, mutations may be made in only one of the framework regions, the variable regions or the constant domain in a single mutated antibody.

The production of chimeric antibodies is described, for example, in WO89/09622. Methods for the production of humanized antibodies are described in, e.g., EP-A1 0 239 400 and WO90/07861. A further source of antibodies to be utilized in accordance with the present invention are so-called xenogenic antibodies. The general principle for the production of xenogenic antibodies such as human antibodies in mice is described in, e.g., WO 91/10741, WO 94/02602, WO 96/34096 and WO 96/33735. As discussed above, the antibody of the presently disclosed subject matter may exist in a variety of forms besides complete antibodies; including, for example, Fv, Fab and F(ab)2, as well as in single chains; see e.g. WO88/09344.

In some embodiments, the antibody, antibody fragment or derivative thereof comprises at least one CDR of each of the VH and the $V_L$ chains.

In one embodiment, an antibody for use in the instant presently disclosed subject matter is a bispecific antibody. A bispecific antibody has binding sites for two different antigens within a single antibody polypeptide. Antigen binding may be simultaneous or sequential. Triomas and hybrid hybridomas are two examples of cell lines that can secrete bispecific antibodies. Examples of bispecific antibodies produced by a hybrid hybridoma or a trioma are disclosed in U.S. Patent No. 4,474,893. Bispecific antibodies have been constructed by chemical means (Staerz et al. (1985) Nature 314:628, and Perez et al. (1985) Nature 316:354) and hybridoma technology (Staerz and Bevan (1986) Proc. Natl. Acad. Sci. USA, 83:1453, and Staerz and Bevan (1986) Immunol. Today 7:241). Bispecific antibodies are also described in U.S. Patent No. 5,959,084. Fragments of bispecific antibodies are described in U.S. Patent No. 5,798,229.

Disclosed herein, but not claimed, is a nucleic acid molecule encoding the antibody, antibody fragment or derivative thereof. As used interchangeably herein, the terms "nucleic acids," "oligonucleotides," and "polynucleotides" include RNA, DNA, or RNA/DNA hybrid sequences of more than one nucleotide in either single chain or duplex form. The term "nucleotide" as used herein as an adjective to describe molecules comprising RNA, DNA, or RNA/DNA hybrid sequences of any length in single-stranded or duplex form. The term "nucleotide" is also used herein as a noun to refer to individual nucleotides or varieties of nucleotides, meaning a molecule, or individual unit in a larger nucleic acid molecule, comprising a purine or pyrimidine, a ribose or deoxyribose sugar moiety, and a phosphate group, or phosphodiester linkage in the case of nucleotides within an oligonucleotide or polynucleotide. The term "nucleotide" is also used herein to encompass "modified nucleotides" which comprise at least one of the following modifications: (a) an alternative linking group, (b) an analogous form of purine, (c) an analogous form of pyrimidine, or (d) an analogous sugar. For examples of analogous linking groups, purine, pyrimidines, and sugars, see for example PCT Patent App. Pub. No. WO 95/04064. The polynucleotide sequences of the presently disclosed subject matter may be prepared by any known method, including synthetic, recombinant, *ex vivo* generation, or a combination thereof, as well as utilizing any purification methods known in the art.

As used herein, "expression" refers to the process by which a polynucleotide is transcribed from a DNA template (such as into an mRNA or other RNA transcript) and/or the process by which a transcribed mRNA is subsequently translated into peptides, polypeptides, or proteins. The term "polypeptide" or "protein" as used herein refers to a molecule comprising a string of at least three amino acids linked together by peptide bonds. The terms "protein" and "polypeptide" may be used interchangeably. Proteins may be recombinant or naturally derived.

The presently disclosed nucleic acid molecules encoding an antibody, antibody fragment or derivative thereof may be, e.g. DNA, cDNA, RNA or synthetically produced DNA or RNA or recombinantly produced chimeric nucleic acid molecule comprising any of those nucleic acid molecules either alone or in combination. The nucleic acid molecule may also be genomic DNA corresponding to the entire gene or a substantial portion thereof or to fragments and derivatives thereof. The nucleotide sequence may correspond to the naturally occurring nucleotide sequence or may contain single or multiple nucleotide substitutions, deletions or additions. In some embodiments, the nucleic acid molecule is a cDNA molecule.

The presently disclosed subject matter also relates to a vector comprising a nucleic acid molecule described herein. Said vector may be, for example, a phage, plasmid, viral or retroviral vector. Retroviral vectors may be replication competent or replication defective. In the latter case, viral propagation generally will occur only in complementing host cells.

The nucleic acid molecules described herein may be joined to a vector containing selectable markers for propagation in a host cell. Generally, a plasmid vector is introduced in a precipitate such as a calcium phosphate precipitate or rubidium chloride precipitate, or in a complex with a charged lipid or in carbon-based clusters, such as fullerens. Should the vector be a virus, it may be packaged in vitro using an appropriate packaging cell line prior to application to host cells.

In some embodiments, the vector is an expression vector wherein the nucleic acid molecule is operatively linked to one or more control sequences allowing the transcription and optionally expression in prokaryotic and/or eukaryotic host cells. Expression of said nucleic acid molecule comprises transcription of the nucleic acid molecule, preferably into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers. Possible regulatory elements permitting expression in prokaryotic host cells comprise, e.g., the lac, trp or tac promoter in *E. coli,* and examples for regulatory elements permitting expression in eukaryotic host cells are the AOXI or GAL1 promoter in yeast or the CMV-, SV40-, RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pCDM8, pRc/CMV, pcDNA1, pcDNA3 (In-vitrogene), pSPORTI (GIBCO BRL). Preferably, said vector is an expression vector and/or a gene transfer or targeting vector. Expression vectors derived from viruses such as retroviruses, vaccinia virus, adenoassociated virus, herpes viruses, or bovine papilloma virus, may be used for delivery of the polynucleotides or vector of the presently disclosed subject matter into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (2001, Third Edition) N.Y. and Ausubel, Current Protocols in Molecular Biology, Green Publishing Associates and Wiley Interscience, N.Y. (1994). Alternatively, the nucleic acid molecules of the presently disclosed subject matter can be reconstituted into liposomes for delivery to target cells.

The presently disclosed subject matter further relates to a host cell comprising a vector of the presently disclosed subject matter. Said host cell may be a prokaryotic or eukaryotic cell. The polynucleotide or vector of the presently disclosed subject matter which is present in the host cell may either be integrated into the genome of the host cell or it may be maintained extrachromosomally. In this respect, it is also to be understood that the nucleic acid molecule of the presently disclosed subject matter can be used for "gene targeting" and/or "gene replacement", for restoring a mutant gene or for creating a mutant gene via homologous recombination; see for example Mouellic, Proc. Natl. Acad. Sci. USA, 87 (1990), 4712-4716; Joyner, Gene Targeting, A Practical Approach, Oxford University Press.

The host cell can be any prokaryotic or eukaryotic cell, such as a bacterial, insect, fungal, plant, animal, mammalian or, preferably, human cell. Preferred fungal cells are, for example, those of the genus *Saccharomyces,* in particular those of the species *S. cerevisiae.* The term "prokaryotic" is meant to include all bacteria which can be transformed or transfected with a polynucleotide for the expression of a variant polypeptide of the presently disclosed subject matter. Prokaryotic host cells may include gram negative as well as gram positive bacteria such as, for example, *E. coli, S. typhimurium, Serratia marcescens* and *Bacillus subtilis.* A polynucleotide coding for a mutant form of variant polypeptides of the presently disclosed subject matter can be used to transform or transfect the host cell using any of the techniques commonly known to those of ordinary skill in the art. Methods for preparing fused, operably linked genes and expressing them in bacteria or animal cells are well-known in the art (Sambrook, Molecular Cloning A Laboratory Manual, Cold Spring Harbor Laboratory (2001, Third Edition). The genetic constructs and methods described therein can be utilized for expression of variant antibodies, antibody fragments or derivatives thereof of the presently disclosed subject matter in, e.g., prokaryotic host cells. In general, expression vectors containing promoter sequences which facilitate the efficient transcription of the inserted nucleic acid molecule are used in connection with the host cell. The expression vector typically contains an origin of replication, a promoter, and a terminator, as well as specific genes which are capable of providing phenotypic selection of the transformed cells. The transformed prokaryotic host cells can be grown in fermentors and cultured according to techniques known in the art to achieve optimal cell growth. The antibodies, antibody fragments or derivatives thereof of the presently disclosed subject matter can then be isolated from the grown medium, cellular lysates, or cellular membrane fractions. The isolation and purification of the microbially or otherwise expressed antibodies, antibody fragments or derivatives thereof of the presently disclosed subject matter may be by any conventional means such as, for example, preparative chromatographic separations and immunological separations such as those involving the use of monoclonal or polyclonal antibodies.

In some embodiments, the host cell is a bacteria, fungal, plant, amphibian or animal cell. Animal cells include, but are not limited to, Chinese hamster ovary (CHO) cells, baby hamster kidney (BHK) cells, monkey kidney cells (COS), 3T3 cells, NSO cells, and a number of other cell lines.

In some embodiments, the host cell is an insect cell. Insect cells include, but are not limited to, cells of the SF9 cell lines. In some embodiments, the host cell is a human cell or human cell line. Said human cells include, but are not limited to Human embryonic kidney cells (HEK293, 293T, 293 freestyle). Furthermore, said human cell lines include, but are not limited to HeLa cells, human hepatocellular carcinoma cells (e.g., Hep G2), A549 cells.

Cell lines of particular preference are selected through determining which cell lines have high expression levels (e.g., KCNK9 expressing cells).

It is likely that antibodies expressed by different cell lines or in transgenic animals will have different glycosylation status. However, all antibodies encoded by the nucleic acid molecules provided herein, or comprising the amino acid sequences provided herein are part of the presently disclosed subject matter, regardless of the glycosylation status of the antibodies.

The presently disclosed subject matter also provides transgenic non-human animals comprising one or more nucleic acid molecules of the presently disclosed subject matter that may be used to produce an antibody, antibody fragment, or derivative thereof of the presently disclosed subject matter. Antibodies can be produced in and recovered from tissue or body fluids, such as milk, blood or urine, of goats, cows, horses, pigs, rats, mice, rabbits, hamsters or other mammals. See, e.g., U.S. Pat. Nos. 5,827,690, 5,756,687, 5,750,172, and 5,741,957. As described above, non-human transgenic animals that comprise human immunoglobulin loci can be produced by immunizing with KCNK9 or a portion thereof (e.g., M1P1 domain).

Disclosed herein, but not claimed, is a method for the preparation of an antibody, antibody fragment or derivative thereof, comprising culturing a host cell of the presently disclosed subject matter under conditions that allow synthesis of said antibody, antibody fragment or derivative thereof and recovering said antibody, antibody fragment or derivative thereof from said culture.

The transformed host cells can be grown in fermentors and cultured according to techniques known in the art to achieve optimal cell growth. Once expressed, the whole antibodies, their dimers, individual light and heavy chains, or other immunoglobulin forms of the presently disclosed subject matter, can be purified according to standard procedures of the art, including ammonium sulfate precipitation, affinity columns, column chromatography, gel electrophoresis and the like; see, Scopes, "Protein Purification", Springer-Verlag, N.Y. (1982). The antibody or its corresponding immunoglobulin chain(s) of the presently disclosed subject matter can then be isolated from the growth medium, cellular lysates, or cellular membrane fractions. The isolation and purification of the, e.g., microbially expressed antibodies or immunoglobulin chains of the presently disclosed subject matter may be by any conventional means such as, for example, preparative chromatographic separations and immunological separations such as those involving the use of monoclonal or polyclonal antibodies directed, e.g., against the constant region of the antibody of the presently disclosed subject matter.

It will be apparent to those skilled in the art that the antibodies of the presently disclosed subject matter can be conjugated to other moieties for, e.g., drug targeting and imaging applications. In some embodiments, the antibody or antigen-binding fragment thereof is conjugated to at least one agent. In some embodiments, at least one agent is selected from the group consisting of a therapeutic agent and a labeling agent. Such conjugating may be conducted chemically after expression of the antibody or antigen to site of attachment or the conjugation product may be engineered into the antibody or antigen of the presently disclosed subject matter at the DNA level. The DNAs are then expressed in a suitable host system, and the expressed proteins are collected and renatured, if necessary.

In some embodiments, the antibody or antigen-binding fragment thereof is conjugated to an effector, such as calicheamicin, Auristatin E or monomethylauristatin E (MMAE), a radioisotope or a toxic chemotherapeutic agent such as geldanamycin and maytansine. In some embodiments, the antibody conjugates are useful in targeting cells, e.g. cancer cells, expressing KCNK9, for elimination. Moreover, the linking of antibodies/antibody fragments of the presently disclosed subject matter to radioisotopes e.g. provides advantages to tumor treatments. Unlike chemotherapy and other conventional forms of cancer treatment, radio immunotherapy or the administration of a radioisotope-antibody combination directly targets the cancer cells with minimal damage to surrounding normal, healthy tissue. Radioisotopes contemplated for use in the presently disclosed subject matter include g. $^{3}H$, $^{14}C$, $^{15}N$, $^{35}S$, $^{90}Y$, $^{99}Tc$, $^{111}In$, $^{125}I$, $^{131}I$.

Aspects of the presently disclosed subject matter involve conjugating an antibody, antibody fragment, or derivative thereof described herein to at least one agent for the treatment of cancer. In some embodiments, at least one agent is an anti-neoplastic agent. In some embodiments, the antibodies of the presently disclosed subject matter can be used to treat cancer when being conjugated with at least one agent toxic chemotherapeutic drugs such as geldanamycin (Mandler et al., J. Natl. Cancer Inst., 92(19), 1549-51 (2000)) and maytansine, for example, the maytansinoid drug, DM1 (Liu et al., Proc. Natl. Acad. Sci. U.S.A. 93:8618-8623 (1996)) auristatin-E (Doronina et al., Nat. Biotechnol. 21:778-784 (2003), and monomethyl auristatin E (Francisco et al., Blood (2003); DOI 10.1182/blood-2003-01-0039). In some embodiments, the antibody or antigen-binding fragment thereof is conjugated to at least one agent via a linker. In some embodiments, the linker is selected from the group consisting of a cleavable linker and an uncleavable linker. Different linkers that release the drugs under acidic or reducing conditions or upon exposure to specific proteases are employed with this technology. In some embodiments, the cleavable linker is a peptide linker. In some embodiments, the peptide linker comprises a valine-citrulline peptide linker. In some embodiments, the uncleavable linker is a thioether linker. The antibodies of the presently disclosed subject matter may be conjugated as described in the art.

In some aspects, the presently disclosed subject matter provides a pharmaceutical composition comprising the antibody or antigen-binding fragment thereof. Disclosed, but not claimed, is a pharmaceutical composition comprising the nucleic acid molecule, the vector, the host cell of the presently disclosed subject matter, or an antibody, antibody fragment or derivative thereof obtained by a method of the presently disclosed subject matter.

The term "composition" as employed herein comprises at least one compound of the invention. Preferably, such a composition is a pharmaceutical or a diagnostic composition.

The composition may be in solid, liquid or gaseous form and may be, inter alia, in a form of (a) powder(s), (a) tablet(s), (a) solution(s) or (an) aerosol(s). Said composition may comprise at least two, preferably three, more preferably four, most preferably five compounds of the presently disclosed subject matter or nucleic acid molecules encoding said compounds. Said composition may also comprise optimized antibodies, antibody fragments or derivatives thereof obtainable by the methods of the presently disclosed subject matter.

In some embodiments, the pharmaceutical composition optionally comprises a pharmaceutically acceptable carrier and/or diluent. The herein disclosed pharmaceutical composition may be used for the treatment of a disorder associated with excessive KCNK9 expression levels and/or activity (e.g., KCNK9 expressing or overexpressing diseases (e.g., KCNK9 expressing cancers).

The presently disclosed subject matter invention provides for pharmaceutical compositions comprising the compounds of the presently disclosed subject matter to be used for the treatment of diseases/disorders associated with KCNK9 expression or overexpression.

Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. The compositions of the presently disclosed subject matter may also be administered directly to the target site, e.g., by biolistic delivery to an external or internal target site, like the brain. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Proteinaceous pharmaceutically active matter may be present in amounts between 1 μg and 100 mg/kg body weight per dose; however, doses below or above this exemplary range are envisioned, especially considering the aforementioned factors. If the regimen is a continuous infusion, it should also be in the range of 1 pg to 100 mg per kilogram of body weight per minute.

Progress can be monitored by periodic assessment. The compositions of the presently disclosed subject matter may be administered locally or systemically.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the presently disclosed subject matter may comprise further agents depending on the intended use of the pharmaceutical composition. It is particularly preferred that the pharmaceutical composition comprises further agents like, e.g. an additional antineoplastic agent, small molecule inhibitor, anti-tumor agent or chemotherapeutic agent.

Aspects of the presently disclosed subject matter also relates to a pharmaceutical composition comprising the antibody or antigen-binding fragment thereof in combination with at least one anti-neoplastic agent. Said combination is effective, for example, in inhibiting abnormal cell growth. Many anti-neoplastic agents are known in the art. In one embodiment, the anti-neoplastic agent is selected from the group of therapeutic proteins including but not limited to antibodies or immunomodulatory proteins. In another embodiment the anti-neoplastic agent is selected from the group of small molecule inhibitors or chemotherapeutic agents consisting of mitotic inhibitors, kinase inhibitors, alkylating agents, anti-metabolites, intercalating antibiotics, growth factor inhibitors, cell cycle inhibitors, enzymes, topoisomerase inhibitors, histone deacetylase inhibitors, anti-survival agents, biological response modifiers, anti-hormones, e.g. anti-androgens, and antiangiogenesis agents.

Also disclosed is a pharmaceutical composition comprising the antibody, antibody fragment or derivative thereof in combination with at least one radiotherapeutic agent.

Also disclosed is a pharmaceutical composition comprising the antibody, antibody fragment or derivative thereof in combination with at least one immunotherapeutic agent.

Also disclosed is pharmaceutical composition comprising the antibody, antibody fragment or derivative thereof in combination with at least one photosensitizing agent.

In some embodiments, when administered in combination, the two or more agents can have a synergistic effect. As used herein, the terms "synergy," "synergistic," "synergistically" and derivations thereof, such as in a "synergistic effect" or a "synergistic combination" or a "synergistic composition" refer to circumstances under which the biological activity of a combination of an agent and at least one additional therapeutic agent is greater than the sum of the biological activities of the respective agents when administered individually.

Synergy can be expressed in terms of a "Synergy Index (SI)," which generally can be determined by the method described by F. C. Kull et al. Applied Microbiology 9, 538 (1961), from the ratio determined by:

$$Q_a Q_A + Q_b Q_B = \text{Synergy Index (SI)}$$

wherein:

$Q_A$ is the concentration of a component A, acting alone, which produced an end point in relation to component A;
$Q_a$ is the concentration of component A, in a mixture, which produced an end point;
$Q_B$ is the concentration of a component B, acting alone, which produced an end point in relation to component B; and
$Q_b$ is the concentration of component B, in a mixture, which produced an end point.

Generally, when the sum of $Q_a/Q_A$ and $Q_b/Q_B$ is greater than one, antagonism is indicated. When the sum is equal to one, additivity is indicated. When the sum is less than one, synergism is demonstrated. The lower the SI, the greater the synergy shown by that particular mixture. Thus, a "synergistic combination" has an activity higher that what can be expected based on the observed activities of the individual components when used alone. Further, a "synergistically effective amount" of a component refers to the amount of the component necessary to elicit a synergistic effect in, for example, another therapeutic agent present in the composition.

In some embodiments, the pharmaceutical composition of the presently disclosed subject matter can also be used for veterinary purposes.

Also disclosed is the use of the antibody, antibody fragment or derivative thereof, the nucleic acid molecule, the vector, the host cell of the presently disclosed subject matter, or an antibody, antibody fragment or derivative thereof obtained by the method of the presently disclosed subject matter for the preparation of a pharmaceutical composition for prevention or treatment of a disorder associated with excessive and/or aberrant KCNK9 expression and/or activity.

In some aspects, the presently disclosed subject matter provides a diagnostic composition comprising the antibody or antigen-binding fragment thereof of the presently disclosed subject matter, and optionally a pharmaceutically acceptable carrier. Also disclosed is a diagnostic composition comprising the nucleic acid molecule, the vector, the host cell of the presently disclosed subject matter, or an antibody, antibody fragment or derivative thereof obtained by the method of the presently disclosed subject matter, and optionally a pharmaceutically acceptable carrier.

The diagnostic composition of the presently disclosed subject matter is useful in the detection of an undesired expression or over-expression of the mammalian KCNK9 potassium channel in different cells, tissues, or samples, comprising contacting a sample with an antibody of the presently disclosed subject matter, and detecting the presence of KCNK9 in the sample. Accordingly, the diagnostic composition of the presently disclosed subject matter may be used for assessing the onset or the disease status of a KCNK9-associated disease. As used herein, "KCNK9-associated disease" refers to any disease, condition, or disorder which is correlated directly or indirectly with abnormal levels of expression and/or activity of KCNK9. Furthermore, malignant cells, such as cancer cells expressing KCNK9, can be targeted with the antibody, antibody fragment or derivative thereof of the presently disclosed subject matter. The cells which have bound the antibody of the presently disclosed subject matter might thus be attacked by immune system functions such as the complement system or by cell-mediated cytotoxicity, therefore reducing in number of or eradicating cancer cells. These considerations equally apply to the diagnosis of metastases and re-current tumors.

In another aspect of the presently disclosed subject matter, the antibody or antigen-binding fragment thereof described herein is conjugated to a labelling agent or labelling group. Such antibodies are suitable for diagnostic applications. As used herein, the term "labelling group" refers to a detectable marker, e.g. a radiolabelled amino acid or biotinyl moieties that can be detected by marked avidin. Various methods for labelling polypeptides and glycoproteins, such as antibodies, are known in the art and may be used in performing the presently disclosed subject matter. Examples of suitable labelling groups include, but are not limited to, the following: radioisotopes or radionuclides (e.g. $_3$H, $_{14}$C, $_{15}$N, $_{35}$S, $_{90}$Y, $_{99}$Tc, $_{111}$In, $_{125}$I, $_{131}$I), fluorescent groups (e.g. FITC, rhodamine, lanthanide phosphors), enzymatic groups (e.g. horseradish peroxidase, $\beta$-galactosidase, luciferase, alkaline phosphatase), chemiluminescent groups, biotinyl groups, or predetermined polypeptide epitopes recognized by a secondary reporter (e.g. leucine zipper pair sequences, binding sites for secondary antibodies, metal binding domains, epitope tags).

In certain aspects, it may be desirable, that the labelling groups are attached by spacer arms of various lengths to reduce potential steric hindrance.

In some aspects, the presently disclosed subject matter provides a method of assessing for the presence of KCNK9 expressing cells comprising contacting in vitro cells or a tissue suspected of expressing KCNK9 on its surface with the antibody or antigen-binding fragment thereof of the presently disclosed subject matter, or a pharmaceutical composition, of the presently disclosed subject matter.

Disclosed, but not claimed, is a method of inhibiting KCNK9 activity comprising contacting a cell or tissue expressing KCNK9 on its surface with the antibody, antibody fragment, or derivative thereof of the presently disclosed subject matter, or a pharmaceutical composition of the presently disclosed subject matter.

Disclosed, but not claimed, is a method of inhibiting the growth or survival of a KCNK9 expressing cancer cell comprising contacting a cancer cell expressing KCNK9 on its surface with the antibody, antibody fragment, or derivative thereof of the presently disclosed subject matter, or a pharmaceutical composition of the presently disclosed subject matter.

In some embodiments, the contacting is performed in vitro in cells or tissues. In some embodiments, the cells or tissues comprise human cells or tissues. In some embodiments disclosed, but not claimed, the contacting is performed in vivo in a subject.

The terms "subject" and "patient" are used interchangeably herein. The subject treated by the presently disclosed methods in their many embodiments is desirably a human subject, although it is to be understood that the methods described herein are effective with respect to all vertebrate species, which are intended to be included in the term "subject." Accordingly, a "subject" can include a human subject for medical purposes, such as for the treatment of an existing condition or disease or the prophylactic treatment for preventing the onset of a condition or disease, or an animal subject for medical, veterinary purposes, or developmental purposes. Suitable animal subjects include mammals including, but not limited to, primates, e.g., humans, monkeys, apes, and the like; bovines, e.g., cattle, oxen, and the like; ovines, e.g., sheep and the like; caprines, e.g., goats and the like; porcines, e.g., pigs, hogs, and the like; equines, e.g., horses, donkeys, zebras, and the like; felines, including wild and domestic cats; canines, including dogs; lagomorphs, including rabbits, hares, and the like; and rodents, including mice, rats, and the like. An animal may be a transgenic animal. In some embodiments, the subject is a human including, but not limited to, fetal, neonatal, infant, juvenile, and adult subjects. Further, a "subject" can include a patient afflicted with or suspected of being afflicted with a condition or disease.

Disclosed, but not claimed, is a method of inhibiting growth and/or metastasis of a tumor in a subject, the method comprising administering to the subject the antibody, antibody fragment, or derivative thereof of the presently disclosed subject matter, or a pharmaceutical composition of the presently disclosed subject matter, in an amount effective to inhibit growth and/or metastasis of the tumor in the subject. In some embodiments, the presently disclosed antibody, antibody fragment, or derivative thereof inhibits growth and/or metastasis of a breast tumor in the subject. In some embodiments, the presently disclosed antibody, antibody fragment, or derivative thereof inhibits metastasis of a breast tumor in the subject. In some embodiments, the presently disclosed antibody, antibody fragment, or derivative thereof inhibits growth and/or metastasis of a lung tumor in the subject. In some embodiments, the presently disclosed antibody, antibody fragment, or derivative thereof inhibits metastasis of a lung tumor in the subject.

Disclosed, but not claimed, is a method of stimulating complement-dependent cancer cell cytotoxicity in a subject, the method comprising administering to the subject the antibody, antibody fragment, or derivative thereof of the presently disclosed subject matter, or a pharmaceutical composition of the presently disclosed subject matter, in an amount effective to stimulate complement-dependent cancer cell cytotoxicity in the subject.

Disclosed, but not claimed, is method for the treatment of a KCNK9 expressing cancer in a subject in need thereof, the method comprising administering to the subject the antibody, antibody fragment, or derivative thereof of the presently disclosed subject matter, or a pharmaceutical composition of the presently disclosed subject matter, in an amount effective to treat the KCNK9 expressing cancer in the subject.

Disclosed, but not claimed, is a method of treating a disease correlated directly or indirectly with the abnormal level of expression and/or activity of KCNK9 in a mammal, the method comprising administering to the mammal an antibody, antibody fragment, or derivative thereof in an amount effective to treat the disease.

Disclosed, but not claimed, is a method for the treatment of a disorder associated with excessive KCNK9 expression and/or activity, the method comprising administering to the subject the antibody, antibody fragment, or derivative thereof of the presently disclosed subject matter, or a pharmaceutical composition of the presently disclosed subject matter, in an amount effective to treat the disorder associated with excessive KCNK9 expression.

The presently disclosed subject matter contemplates treatment of any disease, condition, or disorder for which excessive KCNK9 expression and/or activity is implicated, i.e., a disease correlated directly or indirectly with the abnormal level of expression and/or activity of KCNK9. In general, disorders associated with excessive KCNK9 expression and/or activity include, without limitation, cardiovascular diseases, endocrinological diseases, gastroenterological diseases, cancer, inflammation, hematological diseases, neurological diseases, urological diseases and respiratory diseases. In some embodiments, the cancer is breast cancer. In some embodiments, the cancer is small cell lung cancer. In some embodiments, the cancer is non-small cell lung carcinoma.

KCNK9 is expressed in various human tissues (see, e.g., WO/2005/075510).

*Neurological disorders*

Neurological disorders include disorders of the central nervous system (CNS disorders) as well as disorders of the peripheral nervous system (PNS disorders).

CNS disorders include, but are not limited to brain injuries, cerebrovascular diseases and their consequences, Parkinson's disease, corticobasal degeneration, motor neuron disease, dementia, including ALS, multiple sclerosis, traumatic brain injury, stroke, post-stroke, post-traumatic brain injury, and small-vessel cerebrovascular disease. Dementias, such as Alzheimer's disease, vascular dementia, dementia with Lewy bodies, frontotemporal dementia and Parkinsonism linked to chromosome 17, frontotemporal dementias, including Pick's disease, progressive nuclear palsy, corticobasal degeneration, Huntington's disease, thalamic degeneration, Creutzfeld-Jakob dementia, HTV dementia, schizophrenia with dementia, and Korsakoffs psychosis, within the meaning of the definition are also considered to be CNS disorders.

Similarly, cognitive-related disorders, such as mild cognitive impairment, age-associated memory impairment, age-related cognitive decline, vascular cognitive impairment, attention deficit disorders, attention deficit hyperactivity disorders, and memory disturbances in children with learning disabilities are also considered to be CNS disorders.

Pain, within the meaning of this definition, is also considered to be a CNS disorder. Pain can be associated with CNS disorders, such as multiple sclerosis, spinal cord injury, sciatica, failed back surgery syndrome, traumatic brain injury, epilepsy, Parkinson's disease, post-stroke, and vascular lesions in the brain and spinal cord (e. g., infarct, hemorrhage, vascular malformation). Non-central neuropathic pain includes that associated with post mastectomy pain, phantom feeling, reflex sympathetic dystrophy (RSD), trigeminal neuralgiaradioculopathy, post-surgical pain, HIV/AIDS related pain, cancer pain, metabolic neuropathies (e. g. , diabetic neuropathy, vasculitic neuropathy secondary to connective tissue disease), paraneoplastic polyneuropathy associated, for example, with carcinoma of lung, or leukemia, or lymphoma, or carcinoma of prostate, colon or stomach, trigeminal neuralgia, cranial neuralgias, and post-herpetic neuralgia. Pain associated with peripheral nerve damage, central pain (i. e. due to cerebral ischemia) and various chronic pain i. e., lumbago, back pain (low back pain), inflammatory and/or rheumatic pain. Headache pain (for example, migraine with aura, migraine without aura, and other migraine disorders), episodic and chronic tension-type headache, tension-type like headache, cluster headache, and chronic paroxysmal hemicrania are also CNS disorders.

Visceral pain such as pancreatits, intestinal cystitis, dysmenorrhea, irritable Bowel syndrome, Crohn's disease, biliary colic, ureteral colic, myocardial infarction and pain syndromes of the pelvic cavity, e. g. vulvodynia, orchialgia, urethral syndrome and protatodynia are also CNS disorders.

Also considered to be a disorder of the nervous system are acute pain, for example postoperative pain, and pain after trauma.

The human KCNK9 is highly expressed in the following brain tissues: fetal brain, brain, Alzheimer brain, cerebellum, cerebellum (right), cerebellum (left), cerebral cortex, Alzheimer cerebral cortex, frontal lobe, Alzheimer brain frontal lobe, occipital lobe, parietal lobe, temporal lobe, precentral gyrus, postcentral gyrus, tonsilla cerebelli, vermis cerebelli, pons, cerebral meninges, cerebral peduncles, hippocampus, thalamus, dorsal root ganglia, spinal cord, retina. The expression in brain tissues and in particular the differential expression between diseased tissue Alzheimer brain and healthy tissue brain, between diseased tissue Alzheimer cerebral cortex and healthy tissue cerebral cortex, between diseased tissue Alzheimer brain frontal lobe and healthy tissue frontal lobe demonstrates that the human KCNK9 or mRNA can be utilized to diagnose nervous system diseases. Additionally, the activity of the human KCNK9 can be modulated to treat nervous system diseases.

*Cardiovascular Disorders*

Heart failure is defined as a pathophysiological state in which an abnormality of cardiac function is responsible for the failure of the heart to pump blood at a rate commensurate with the requirement of the metabolizing tissue. It includes all forms of pumping failures such as high-output and low- output, acute and chronic, right-sided or left-sided, systolic or diastolic, independent of the underlying cause.

Myocardial infarction (MI) is generally caused by an abrupt decrease in coronary blood flow that follows a thrombotic occlusion of a coronary artery previously narrowed by arteriosclerosis. MI prophylaxis (primary and secondary prevention) is included as well as the acute treatment of MI and the prevention of complications.

Ischemic diseases are conditions in which the coronary flow is restricted resulting in a perfusion which is inadequate to meet the myocardial requirement for oxygen. This group of diseases includes stable angina, unstable angina and asymptomatic ischemia.

Arrhythmias include all forms of atrial and ventricular tachyarrhythmias, atrial tachycardia, atrial flutter, atrial fibrillation, atrio-ventricular reentrant tachycardia, preexitation syndrome, ventricular tachycardia, ventricular flutter, ventricular fibrillation, as well as bradycardic forms of arrhythmias.

Hypertensive vascular diseases include primary as well as all kinds of secondary arterial hypertension, renal, endocrine, neurogenic, others. The genes may be used as drug targets for the treatment of hypertension as well as for the prevention of all complications arising from cardiovascular diseases.

Peripheral vascular diseases are defined as vascular diseases in which arterial and/or venous flow is reduced resulting in an imbalance between blood supply and tissue oxygen demand. It includes chronic peripheral arterial occlusive disease (PAOD), acute arterial thrombosis and embolism, inflammatory vascular disorders, Raynaud's phenomenon and venous disorders.

Atherosclerosis is a cardiovascular disease in which the vessel wall is remodeled, compromising the lumen of the vessel. The atherosclerotic remodeling process involves accumulation of cells, both smooth muscle cells and monocyte/macrophage inflammatory cells, in the intima of the vessel wall. These cells take up lipid, likely from the circulation, to form a mature atherosclerotic lesion.

Although the formation of these lesions is a chronic process, occurring over decades of an adult human life, the majority of the morbidity associated with atherosclerosis occurs when a lesion ruptures, releasing thrombogenic debris that rapidly occludes the artery. When such an acute event occurs in the coronary artery, myocardial infarction can ensue, and in the worst case, can result in death.

The formation of the atherosclerotic lesion can be considered to occur in five overlapping stages such as migration, lipid accumulation, recruitment of inflammatory cells, proliferation of vascular smooth muscle cells, and extracellular matrix deposition. Each of these processes can be shown to occur in man and in animal models of atherosclerosis, but the relative contribution of each to the pathology and clinical significance of the lesion is unclear.

Thus, a need exists for therapeutic methods and agents to treat cardiovascular diseases, such as atherosclerosis and other conditions related to coronary artery disease.

Cardiovascular diseases include but are not limited to disorders of the heart and the vascular system like congestive heart failure, myocardial infarction, ischemic diseases of the heart, all kinds of atrial and ventricular arrhythmias, hypertensive vascular diseases, peripheral vascular diseases, and atherosclerosis.

Excessively high or low levels of fats in the bloodstream, especially cholesterol, can cause long-term problems. The risk to develop atherosclerosis and coronary artery or carotid artery disease (and thus the risk of having a heart attack or stroke) increases with the total cholesterol level increasing. Nevertheless, extremely low cholesterol levels may not be healthy. Examples of disorders of lipid metabolism are hyperlipidemia (abnormally high levels of fats (cholesterol, triglycerides, or both) in the blood, may be caused by family history of hyperlipidemia), obesity, a high-fat diet, lack of exercise, moderate to high alcohol consumption, cigarette smoking, poorly controlled diabetes, and an underactive thyroid gland), hereditary hyperlipidemias (type I hyperlipoproteinemia (familial hyperchylomicronemia), type II hyperlipoproteinemia (familial hypercholesterolemia), type in hyperlipoproteinemia, type IV hyperlipoproteinemia, or type V hyperlipoproteinemia), hypolipo- proteinemia, lipidoses (caused by abnormalities in the enzymes that metabolize fats), Gaucher's disease, Niemann-Pick disease, Fabry's disease, Wolman's disease, cerebrotendinous xanthomatosis, sitosterolemia, Refsum's disease, or Tay-Sachs disease.

Kidney disorders may lead to hypertension or hypotension. Examples for kidney problems possibly leading to hypertension are renal artery stenosis, pyelonephritis, glomerulonephritis, kidney tumors, polycistic kidney disease, injury to the kidney, or radiation therapy affecting the kidney.

Excessive urination may lead to hypotension.

The human KCNK9 is highly expressed in the following cardiovascular related tissues: pericardium, heart atrium (left), heart ventricle (left), aorta, artery, vein, adrenal gland, thrombocytes, kidney, kidney tumor. Expression in the above mentioned tissues demonstrates that the human KCNK9 or mRNA can be utilized to diagnose cardiovascular diseases. Additionally, the activity of the human KCNK9 can be modulated to treat cardiovascular diseases.

The human KCNK9 is highly expressed in kidney tissues: kidney, kidney tumor. Expression in kidney tissues demonstrates that the human KCNK9 or mRNA can be utilized to diagnose blood pressure disorders as a cardiovascular disorder. Additionally, the activity of the human KCNK9 can be modulated to treat-but not limited to-blood pressure disorders as hypertension or hypotension.

The human KCNK9 is highly expressed in adrenal gland. Expression in adrenal gland tissues demonstrates that the human KCNK9 or mRNA can be utilized to diagnose blood pressure disorders as a cardiovascular disorder. Additionally, the activity of the human KCNK9 can be modulated to treat blood pressure disorders as hypertension or hypotension.

*Hematological Disorders*

Hematological disorders comprise diseases of the blood and all its constituents as well as diseases of organs and tissues involved in the generation or degradation of all the constituents of the blood.

They include but are not limited to 1) Anemias, 2) Myeloproliferative Disorders, 3) Hemorrhagic Disorders, 4) Leukopenia, 5) Eosinophilic Disorders, 6) Leukemias, 7) Lymphomas, 8) Plasma Cell Dyscrasias, 9) Disorders of the Spleen in the course of hematological disorders. Disorders according to 1) include, but are not limited to anemias due to defective or deficient hem synthesis, deficient erythropoiesis. Disorders according to 2) include, but are not limited to polycythemia vera, tumor-associated erythrocytosis, myelofibrosis, thrombocythemia. Disorders according to 3) include, but are not limited to vasculitis, thrombocytopenia, heparin-induced thrombocytopenia, thrombotic thrombocytopenic purpura, hemolytic-uremic syndrome, hereditary and acquired disorders of platelet function, hereditary coagulation disorders. Disorders according to 4) include, but are not limited to neutropenia, lymphocytopenia. Disorders according to 5) include, but are not limited to hypereosinophilia, idiopathic hypereosinophilic syndrome. Disorders according to 6) include, but are not limited to acute myeloic leukemia, acute lymphoblastic leukemia, chronic myelocytic leukemia, chronic lymphocytic leukemia, myelodysplastic syndrome. Disorders according to 7) include, but are not limited to Hodgkin's disease, non-Hodgkin's lymphoma, Burkitt's lymphoma, mycosis fungoides cutaneous T-cell lymphoma. Disorders according to 8) include, but are not limited to multiple myeloma, macroglobulinemia, heavy chain diseases. In extension of the preceding idiopathic thrombocytopenic purpura, iron deficiency anemia, megaloblastic anemia (vitamin B12 deficiency), aplastic anemia, thalassemia, malignant lymphoma bone marrow invasion, malignant lymphoma skin invasion, hemolytic uremic syndrome, giant platelet disease are considered to be hematological diseases too.

The human KCNK9 is highly expressed in the following tissues of the hematological system: erythrocytes, lymphnode, thrombocytes, bone marrow CD71+ cells. The expression in the above mentioned tissues demonstrates that the human KCNK9 or mRNA can be utilized to diagnose hematological diseases. Additionally, the activity of the human KCNK9 can be modulated to treat hematological disorders.

*Gastrointestinal and Liver Diseases*

Gastrointestinal diseases comprise primary or secondary, acute or chronic diseases of the organs of the gastrointestinal tract which may be acquired or inherited, benign or malignant or metaplastic, and which may affect the organs of the gastrointestinal tract or the body as a whole. They comprise but are not limited to 1) disorders of the esophagus like achalasia, vigoruos achalasia, dysphagia, cricopharyngeal incoordination, pre-esophageal dysphagia, diffuse esophageal spasm, globus sensation, Barrett's metaplasia, gastroesophageal reflux, 2) disorders of the stomach and duodenum like functional dyspepsia, inflammation of the gastric mucosa, gastritis, stress gastritis, chronic erosive gastritis, atrophy of gastric glands, metaplasia of gastric tissues, gastric ulcers, duodenal ulcers, neoplasms of the stomach, 3) disorders of the pancreas like acute or chronic pancreatitis, insufficiency of the exocrinic or endocrinic tissues of the pancreas like steatorrhea, diabetes, neoplasms of the exocrine or endocrine pancreas like 3.1) multiple endocrine neoplasia syndrome, ductal adenocarcinoma, cystadenocarcinoma, islet cell tumors, insulinoma, gastrinoma, carcinoid tumors, glucagonoma, Zollinger-Ellison syndrome, Vipoma syndrome, malabsorption syndrome, 4) disorders of the bowel like chronic inflammatory diseases of the bowel, Crohn's disease, ileus, diarrhea and constipation, colonic inertia, megacolon, malabsorption syndrome, ulcerative colitis, 4.1) functional bowel disorders like irritable bowel syndrome, 4.2) neoplasms of the bowel like familial polyposis, adenocarcinoma, primary malignant lymphoma, carcinoid tumors, Kaposi's sarcoma, polyps, cancer of the colon and rectum.

Liver diseases comprise primary or secondary, acute or chronic diseases or injury of the liver which may be acquired or inherited, benign or malignant, and which may affect the liver or the body as a whole. They comprise but are not limited to disorders of the bilirubin metabolism, jaundice, syndroms of Gilbert's, Crigler-Najjar, Dubin-Johnson and Rotor; intrahepatic cholestasis, hepatomegaly, portal hypertension, ascites, Budd-Chiari syndrome, portal-systemic encephalopathy, fatty liver, steatosis, Reye's syndrome, liver diseases due to alcohol, alcoholic hepatitis or cirrhosis, fibrosis and cirrhosis, fibrosis and cirrhosis of the liver due to inborn errors of metabolism or exogenous substances, storage diseases, syndromes of Gaucher's, Zellweger's, Wilson's-disease, acute or chronic hepatitis, viral hepatitis and its variants, inflammatory conditions of the liver due to viruses, bacteria, fungi, protozoa, helminths; drug induced disorders of the liver, chronic liver diseases like primary sclerosing cholangitis, alphal-antitrypsin- deficiency, primary biliary cirrhosis, postoperative liver disorders like postoperative intrahepatic cholestasis, hepatic granulomas, vascular liver disorders associated with systemic disease, benign or malignant neoplasms of the liver, disturbance of liver metabolism in the new-born or prematurely born.

The human KCNK9 is highly expressed in the following tissues of the gastroenterological system: esophagus, esophagus tumor, colon, colon tumor, ileum, ileum tumor, ileum chronic inflammation, rectum. The expression in the above mentioned tissues and in particular the differential expression between diseased tissue ileum chronic inflammation and healthy tissue ileum demonstrates that the human KCNK9 or mRNA can be utilized to diagnose gastroenterological disorders.

Additionally, the activity of the human KCNK9 can be modulated to treat gastroenterological disorders.

*Endocrine System and Hormones*

The endocrine system consists of a group of organs whose main function is to produce and secrete hormones directly into the bloodstream. The major organs of the endocrine system are the hypothalamus, the pituitary gland, thyroid gland, the parathyroid glands, the islets of the pancreas, the adrenal glands, the testes, and the ovaries.

The hypothalamus secretes several hormones that stimulate the pituitary: Some trigger the release of pituitary hormones; others suppress the release of pituitary hormones.

The pituitary gland coordinates many functions of the other endocrine glands, but some pituitary hormones have direct effects.

The insulin-secreting cells of the pancreas respond to glucose and fatty acids. Parathyroid cells respond to calcium and phosphate. The adrenal medulla (part of the adrenal gland) responds to direct stimulation by the parasympathetic nervous system.

When endocrine glands malfunction, hormone levels in the blood can become abnormally high or low, disrupting body functions. Many disorders are caused by malfunction of the endocrine system or hormones. Examples of such disorders are presented in the following.

Diabetes mellitus is a disorder in which blood levels of glucose are abnormally high because the body doesn't release or use insulin adequately.

People with type I diabetes mellitus (insulin-dependent diabetes) produce little or no insulin at all.

In type I diabetes more than 90 percent of the insulin-producing cells (beta cells) of the pancreas are permanently destroyed. The resulting insulin deficiency is severe, and to survive, a person with type I diabetes must regularly inject insulin.

In type II diabetes mellitus (non-insulin-dependent diabetes) the body develops resistance to insulin effects, resulting in a relative insulin deficiency.

The pancreas has two major functions: to secrete fluid containing digestive enzymes into the duodenum and to secrete the hormones insulin and glucagon. Chronic pancreatitis is a long- standing inflammation of the pancreas. Eventually, the insulin-secreting cells of the pancreas may be destroyed, gradually leading to diabetes. An insulinoma is a rare type of pancreatic tumor that secretes insulin. The symptoms of an insulinoma result from low blood glucose levels. A gastrinoma is a pancreatic tumor that produces excessive levels of the hormone gastrin, which stimulates the stomach to secrete acid and enzymes, causing peptic ulcers. The excess gastrin secreted by the gastrinoma causes symptoms, called the Zollinger-Ellison syndrome. A glucagonoma is a tumor that produces the hormone glucagon, which raises the level of glucose in the blood and produces a distinctive rash.

Diabetes insipidus is a disorder in which insufficient levels of antidiuretic hormone cause excessive thirst (polydipsia) and excessive production of very dilute urine (polyuria). Diabetes insipidus results from the decreased production of antidiuretic hormone (vasopressin).

The body has two adrenal glands. The medulla of the adrenal glands secretes hormones such as adrenaline (epinephrine) that affect blood pressure, heart rate, sweating, and other activities also regulated by the sympathetic nervous system. The cortex secretes many different hormones, including corticosteroids (cortisone-like hormones), androgens (male hormones), and mineral- ocorticoids, which control blood pressure and the levels of salt and potassium in the body.

A diseases characterized by underactive adrenal glands is Addison's disease (adrenocortical insufficiency).

Several disorders are characterized by overactive Adrenal Glands. The causes can be changes in the adrenal glands themselves or overstimulation by the pituitary gland. Examples of these diseases are listed in the following.

Overproduction of androgenic steroids (testosterone and similar hormones, leads to virilization), overproduction of corticosteroids (causes could be tumors of the pituitary or the adrenal gland, results in Cushing's syndrome), Nelson's syndrome (developed by people who have both adrenal glands removed, characterized by an enlargement of the pituitary gland), Overproduction of aldosterone (hyperaldosteronism), Conn's syndrome (hyperaldosterism caused by a tumor), pheochromocytoma (a tumor that originating from the adrenal gland's chromaffin cells, causing overproduction of catecholamines), The thyroid is a small gland located under the Adam's apple. It secretes thyroid hormones, which control the metabolic rate. The thyroid gland traps iodine and processes it into thyroid hormones.

The euthyroid sick syndrome is characterized by lack of conversion of the T4 form of thyroid hormone to the T3 form. Hyperthyroidism (overactive thyroid gland, production of too much hormone) may have several causes. Thyroiditis (an inflammation of the thyroid gland), typically leads to a phase of hyperthyroidism. The inflammation may damage the thyroid gland, so that in later stages the disease is characterized by transient or permanent underactivity (hypothyroidism).

Toxic thyroid nodules (adenomas) often produce thyroid hormone in large quantities. Toxic multinodular goiter (Plummer's disease) is a disorder in which there are many nodules. Graves' disease (toxic diffuse goiter) is believed to be caused by an antibody that stimulates the thyroid to produce too much thyroid hormone. In toxic nodular goiter, one or more nodules in the thyroid produce too much thyroid hormone and aren't under the control of thyroid-stimulating hormone.

Secondary hyperthyroidism may (rarely) be caused by a pituitary tumor that secretes too much thyroid-stimulating hormone, by resistance of the pituitary to thyroid hormone, which results in the pituitary gland secreting too much thyroid-stimulating hormone, or by a hydatidiform mole in women. Thyroid storm is a sudden extreme overactivity of the thyroid gland is a life-threatening emergency requiring prompt treatment.

Hypothyroidism is a condition in which the thyroid gland is underactive and produces too little thyroid hormone. Very severe hypothyroidism is called myxedema. In Hashimoto's thyroiditis (autoimmune thyroiditis) the thyroid gland is often enlarged, and hypothyroidism results because the gland's functioning areas are gradually destroyed. Rarer causes of hypothyroidism include some inherited disorders which are caused by abnormalities of the enzymes in thyroid cells. In other rare disorders, either the hypothalamus or the pituitary gland fails to secrete enough of the hormone needed to stimulate normal thyroid function.

Other examples of Thyroiditis are silent lymphocytic thyroiditis, Hashimoto's thyroiditis, or subacute granulomatous thyroiditis.

Thyroid cancer is any one of four main types of malignancy of the thyroid: papillary, follicular, anaplastic, or medullary.

The pituitary is a pea-sized gland that sits in a bony structure (sella turcica) at the base of the brain.

The sella turcica protects the pituitary but allows very little room for expansion. If the pituitary enlarges, it tends to push upward, often pressing on the areas of the brain that carry signals from the eyes, possibly resulting in headaches or impaired vision. The pituitary gland has two distinct parts: the anterior (front) and the posterior (back) lobes. The anterior lobe produces (secretes) hormones that ultimately control the function of the thyroid gland, adrenal glands, and reproductive organs (ovaries and testes); milk production (lactation) in the breasts; and overall body growth. It also produces hormones that cause the skin to darken and that inhibit pain sensations. The posterior lobe produces hormones that regulate water balance, stimulate the let- down of milk from the breasts in lactating women, and stimulate contractions of the uterus.

Examples for disorders of the pituitary gland are Empty Sella Syndrome; hypopituitarism (an underactive pituitary gland); acromegaly, which is excessive growth caused by oversecretion of growth hormone, which is almost always caused by a benign pituitary tumor (adenoma); galactorrhea, which is the production of breast milk in men or in women who aren't breastfeeding, in both sexes, the most common cause of galactorrhea is a prolactin-producing tumor (prolactinoma) in the pituitary gland.

The human KCNK9 is highly expressed in the following tissues of the endocrinological system: adrenal gland. The expression in the above mentioned tissues demonstrates that the human KCNK9 or mRNA can be utilized to diagnose endocrinological disorders. Additionally, the activity of the human KCNK9 can be modulated to treat endocrinological disorders.

*Cancer*

Cancer within the scope of this definition comprise any disease of an organ or tissue in mammals characterized by poorly controlled or uncontrolled multiplication of normal or abnormal cells in that tissue and its effect on the body as a whole. Cancer diseases within the scope of the definition comprise benign neoplasms, dysplasias, hyperplasias as well as neoplasms showing metastatic growth or any other transformations like e. g. leukoplakias which often precede a breakout of cancer. Cells and tissues are cancerous when they grow more rapidly than normal cells, displacing or spreading into the surrounding healthy tissue or any other tissues of the body described as metastatic growth, assume abnormal shapes and sizes, show changes in their nucleocytoplasmatic ratio, nuclear polychromasia, and finally may cease. Cancerous cells and tissues may affect the body as a whole when causing paraneoplastic syndromes or if cancer occurs within a vital organ or tissue, normal function will be impaired or halted, with possible fatal results. The ultimate involvement of a vital organ by cancer, either primary or metastatic, may lead to the death of the mammal affected. Cancer tends to spread, and the extent of its spread is usually related to an individual's chances of surviving the disease. Cancers are generally said to be in one of three stages of growth: early, or localized, when a tumor is still confined to the tissue of origin, or primary site; direct extension, where cancer cells from the tumour have invaded adjacent tissue or have spread only to regional lymph nodes; or metastasis, in which cancer cells have migrated to distant parts of the body from the primary site, via the blood or lymph systems, and have established secondary sites of infection. Cancer is said to be malignant because of its tendency to cause death if not treated. Benign tumors usually do not cause death, although they may if they interfere with a normal body function by virtue of their location, size, or paraneoplastic side effects. Hence benign tumors fall under the definition of cancer within the scope of this definition as well. In general, cancer cells divide at a higher rate than do normal cells, but the distinction between the growth of cancerous and normal tissues is not so much the rapidity of cell division in the former as it is the partial or complete loss of growth restraint in cancer cells and their failure to differentiate into a useful, limited tissue of the type that characterizes the functional equilibrium of growth of normal tissue. Cancer tissues may express certain molecular receptors and probably are influenced by the host's susceptibility and immunity and it is known that certain cancers of the breast and prostate, for example, are considered dependent on specific hormones for their existence. The term "cancer" under the scope of the definition is not limited to simple benign neoplasia but comprises any other benign and malign neoplasia like 1) Carcinoma, 2) Sarcoma, 3) Carcinosarcoma, 4) Cancers of the blood-forming tissues, 5) tumors of nerve tissues including the brain, 6) cancer of skin cells. Cancer according to 1) occurs in epithelial tissues, which cover the outer body (the skin) and line mucous membranes and the inner cavitary structures of organs e. g. such as the breast, lung, the respiratory and gastrointestinal tracts, the endocrine glands, and the genitourinary system. Ductal or glandular elements may persist in epithelial tumors, as in adenocarcinomas like e. g. thyroid adenocarcinoma, gastric adenocarcinoma, uterine adeno-carcinoma. Cancers of the pavement-cell epithelium of the skin and of certain mucous membranes, such as e. g. cancers of the tongue, lip, larynx, urinary bladder, uterine cervix, or penis, may be termed epidermoid or squamous-cell carcinomas of the respective tissues and are in the scope of the definition of cancer as well. Cancer according to 2) develops in connective tissues, including fibrous tissues, adipose (fat) tissues, muscle, blood vessels, bone, and cartilage like e. g. osteogenic sarcoma; liposarcoma, fibrosarcoma, synovial sarcoma. Cancer according to 3) is cancer that develops in both epithelial and connective tissue. Cancer disease within the scope of this definition may be primary or secondary, whereby primary indicates that the cancer originated in the tissue where it is found rather than was established as a secondary site through metastasis from another lesion. Cancers and tumor diseases within the scope of this definition may be benign or malign and may affect all anatomical structures of the body of a mammal. By example but not limited to they comprise cancers and tumor diseases of 0 the bone marrow and bone marrow derived cells (leukemias), IT) the endocrine and exocrine glands like e. g. thyroid, parathyroid, pituitary, adrenal glands, salivary glands, pancreas BD the breast, like e. g. benign or malignant tumors in the mammary glands of either a male or a female, the mammary ducts, adenocarcinoma, medullary carcinoma, comedo carcinoma, Paget's disease of the nipple, inflammatory carcinoma of the young woman, IV) the lung, V) the stomach, VI) the liver and spleen, VIn the small intestine, VIM) the colon, IX) the bone and its supportive and connective tissues like malignant or benign bone tumour, e. g. malignant osteogenic sarcoma, benign osteoma, cartilage tumors; like malignant chondrosarcoma or benign chondroma; bone marrow tumors like malignant myeloma or benign eosinophilic granuloma, as well as metastatic tumors from bone tissues at other locations of the body; X) the mouth, throat, larynx, and the esophagus, XI) the urinary bladder and the internal and external organs and structures of the urogenital system of male and female like ovaries, uterus, cervix of the uterus, testes, and prostate gland, XD) the prostate, XHI) the pancreas, like ductal carcinoma of the pancreas; XIV) the lymphatic tissue like lymphomas and other tumors of lymphoid origin, XV) the skin, XVI) cancers and tumor diseases of all anatomical structures belonging to the respiration and respiratory systems including thoracal muscles and linings, XVII) primary or secondary cancer of the lymph nodes XVE) the tongue and of the bony structures of the hard palate or sinuses, XVIV) the mouth, cheeks, neck and salivary glands, XX) the blood vessels including the heart and their linings, XXI) the smooth or skeletal muscles and their ligaments and linings, XXII) the peripheral, the autonomous, the central nervous system including the cerebellum, XXW the adipose tissue.

The human KCNK9 is highly expressed in the following cancer tissues: esophagus tumor, colon tumor, ileum tumor, lung tumor, ovary tumor, breast tumor, kidney tumor. The expression in the above mentioned tissues and in particular the differential expression between diseased tissue esophagus tumor and healthy tissue esophagus, between diseased tissue colon tumor and healthy tissue colon, between diseased tissue ileum tumor and healthy tissue ileum, between diseased tissue lung tumor and healthy tissue lung, between diseased tissue ovary tumor and healthy tissue, between diseased tissue breast tumor and healthy tissue breast, between diseased tissue kidney tumor and healthy tissue kidney demonstrates that the human KCNK9 or mRNA can be utilized to diagnose cancer. Additionally, the activity of the human KCNK9 can be modulated to treat cancer.

*Inflammatory Diseases*

Inflammatory diseases comprise diseases triggered by cellular or non-cellular mediators of the immune system or tissues causing the inflammation of body tissues and subsequently producing an acute or chronic inflammatory condition. Examples for such inflammatory diseases are hypersensitivity reactions of type I-IV, for example but not limited to hypersensitivity diseases of the lung including asthma, atopic diseases, allergic rhinitis or conjunctivitis, angioedema of the lids, hereditary angioedema, antireceptor hypersensitivity reactions and autoimmune diseases, Hashimoto's thyroiditis, systemic lupus erythematosus, Goodpasture's syndrome, pemphigus, myasthenia gravis, Grave's and Raynaud's disease, type B insulin-resistant diabetes, rheumatoid arthritis, psoriasis, Crohn's disease, scleroderma, mixed connective tissue disease, polymyositis, sarcoidosis, glomerulonephritis, acute or chronic host versus graft reactions.

The human KCNK9 is highly expressed in the following tissues of the immune system and tissues responsive to components of the immune system as well as in the following tissues responsive to mediators of inflammation: ileum chronic inflammation, lung COPD. The expression in the above mentioned tissues and in particular the differential expression between diseased tissue ileum chronic inflammation and healthy tissue ileum, between diseased tissue lung COPD and healthy tissue lung demonstrates that the human KCNK9 or mRNA can be utilized to diagnose inflammatory diseases. Additionally, the activity of the human KCNK9 can be modulated to treat inflammatory diseases.

*Disorders Related to Pulmology (Respiratory Disorders)*

Asthma is thought to arise as a result of interactions between multiple genetic and environmental factors and is characterized by three major features: 1) intermittent and reversible airway obstruction caused by bronchoconstriction, increased mucus production, and thickening of the walls of the airways that leads to a narrowing of the airways, 2) airway hyperresponsiveness, and 3) airway inflammation. Certain cells are critical to the inflammatory reaction of asthma and they include T cells and antigen presenting cells, B cells that produce IgE, and mast cells, basophils, eosinophils, and other cells that bind IgE. These effector cells accumulate at the site of allergic reaction in the airways and release toxic products that contribute to the acute pathology and eventually to tissue destruction related to the disorder. Other resident cells, such as smooth muscle cells, lung epithelial cells, mucus-producing cells, and nerve cells may also be abnormal in individuals with asthma and may contribute to its pathology. While the airway obstruction of asthma, presenting clinically as an intermittent wheeze and shortness of breath, is generally the most pressing symptom of the disease requiring immediate treatment, the inflammation and tissue destruction associated with the disease can lead to irreversible changes that eventually make asthma a chronic and disabling disorder requiring long-term management.

Chronic obstructive pulmonary (or airways) disease (COPD) is a condition defined physiologically as airflow obstruction that generally results from a mixture of emphysema and peripheral airway obstruction due to chronic bronchitis [Botstein, 1980]. Emphysema is characterised by destruction of alveolar walls leading to abnormal enlargement of the air spaces of the lung. Chronic bronchitis is defined clinically as the presence of chronic productive cough for three months in each of two successive years. In COPD, airflow obstruction is usually progressive and is only partially reversible. By far the most important risk factor for development of COPD is cigarette smoking, although the disease does also occur in non-smokers.

The human KCNK9 is highly expressed in the following tissues of the respiratory system: lung, lung tumor, lung COPD. The expression in the above mentioned tissues and in particular the differential expression between diseased tissue lung COPD and healthy tissue lung demonstrates that the human KCNK9 or mRNA can be utilized to diagnose respiratory diseases. Additionally, the activity of the human KCNK9 can be modulated to treat those diseases.

*Disorders Related to Urology*

Genitourinary disorders comprise benign and malign disorders of the organs constituting the genitourinary system of female and male, renal diseases like acute or chronic renal failure, immunologically mediated renal diseases like renal transplant rejection, lupus nephritis, immune complex renal diseases, glomerulopathies, nephritis, toxic nephropathy, obstructive uropathies like benign prostatic hyperplasia (BPH), neurogenic bladder syndrome, urinary incontinence like urge-, stress-, or overflow incontinence, pelvic pain, and erectile dysfunction.

The human KCNK9 is highly expressed in the following urological tissues: dorsal root ganglia, spinal cord, penis, corpus cavernosum, kidney, kidney tumor. The expression in the above mentioned tissues demonstrates that the human KCNK9 or mRNA can be utilized to diagnose urological disorders. Additionally, the activity of the human KCNK9 can be modulated to treat urological disorders.

The human KCNK9 is highly expressed in dorsal-root ganglia tissue. Expression in dorsal root ganglia demonstrates that the human KCNK9 or mRNA can be utilized to diagnose incontinence as an urological disorder. The dorsal root ganglia are involved in the neuronal regulation of the urological system. Additionally, the activity of the human KCNK9 can be modulated to treat-but not limited to-incontinence.

The human KCNK9 is highly expressed in spinal cord tissues: spinal cord. Expression in spinal cord tissues demonstrates that the human KCNK9 or mRNA can be utilized to diagnose incontinence as an urological disorder. The spinal cord tissues are involved in the neuronal regulation of the urological system. Additionally, the activity of the human KCNK9 can be modulated to treat-but not limited to-incontinence.

The presently disclosed subject matter provides for both prophylactic and therapeutic methods for cardiovascular diseases, endocrinological diseases, gastroenterological diseases, cancer, inflammation, hemato- logical diseases, neurological diseases, urological diseases and respiratory diseases.

In general, the "effective amount" or "therapeutically effective amount" of an active agent or drug delivery device refers to the amount necessary to elicit the desired biological response. As will be appreciated by those of ordinary skill in this art, the effective amount of an agent or device may vary depending on such factors as the desired biological endpoint, the agent to be delivered, the composition of the encapsulating matrix, the target tissue, and the like.

In some embodiments, the subject has or suspected of having a cancer. In some embodiments disclosed, but not claimed, the method further includes administering to the subject an effective amount of a conventional cancer treatment. Examples of conventional cancer treatments include, without limitation, chemotherapy, radiotherapy, immunotherapy, proton therapy, photodynamic therapy, and surgery.

As used herein, the term "immunotherapeutic agent" refers to a molecule that can aid in the treatment of a disease by inducing, enhancing, or suppressing an immune response in a cell, tissue, organ or subject.

**EP 3 271 397 B1**

As used herein, a "radiotherapeutic agent" and "radiotherapy" are used interchangeably and refer to those agents conventionally adopted in the therapeutic field of cancer treatment and include photons having enough energy for chemical bond ionization such as, for instance, alpha (.alpha.), beta (.beta.), and gamma (.gamma.) rays from radioactive nuclei as well as x-rays. The radiation may be high-LET (linear energy transfer) or low-LET. LET is the energy transferred per unit length of the distance. High LET is said to be densely ionizing radiation and Low LET is said to be sparsely ionizing radiation. Representative examples of high-LET are neutrons and alpha particles. Representative examples of low-LET are x-ray and gamma rays. Low LET radiation including both x-rays and .gamma. rays is most commonly used for radiotherapy of cancer patients. The radiation may be used for external radiation therapy that is usually given on an outpatient basis or for internal radiation therapy that uses radiation that is placed very close to or inside the tumor. In case of internal radiation therapy, the radiation source is usually sealed in a small holder called an implant. Implants may be in the form of thin wires, plastic tubes called catheters, ribbons, capsules, or seeds. The implant is put directly into the body. Internal radiation therapy may require a hospital stay. The ionizing radiation source is provided as a unit dose of radiation and is preferably an x-ray tube since it provides many advantages, such as convenient adjustable dosing where the source may be easily turned on and off, minimal disposal problems, and the like. A unit dose of radiation is generally measured in gray (Gy). The ionizing radiation source may also comprise a radioisotope, such as a solid radioisotopic source (e.g., wire, strip, pellet, seed, bead, or the like), or a liquid radioisotopic filled balloon. In the latter case, the balloon has been specially configured to prevent leakage of the radioisotopic material from the balloon into the body lumen or blood stream. Still further, the ionizing radiation source may comprise a receptacle in the catheter body for receiving radioisotopic materials like pellets or liquids. The radioisotopic material may be selected to emit .alpha., .beta. and .gamma.. Usually, .alpha. and .beta. radiations are preferred since they may be quickly absorbed by the surrounding tissue and will not penetrate substantially beyond the wall of the body lumen being treated. Accordingly, incidental irradiation of the heart and other organs adjacent to the treatment region can be substantially eliminated. The total number of units provided will be an amount determined to be therapeutically effective by one skilled in treatment using ionizing radiation. This amount will vary with the subject and the type of malignancy or neoplasm being treated. The amount may vary but a patient may receive a dosage of about 30-75 Gy over several weeks.

Exemplary radiotherapeutic agents contemplated for use in combination with at least one metabolic reprogramming agent, at least two metabolic reprogramming agents, or at least three metabolic reprogramming agents include, factors that cause DNA damage, such as .gamma.-rays, X-rays, and/or the directed delivery of radioisotopes to tumor cells. Other forms of DNA damaging factors are also contemplated such as microwaves and UV-irradiation. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the target cell. In some embodiments, the radiotherapeutic agent is selected from the group consisting of $^{47}$Sc, $^{61}$Cu, $^{90}$Y, $^{109}$Pd, $^{123}$I, $^{125}$I, $^{131}$I, $^{186}$Re, $^{188}$Re, $^{199}$Au, $^{211}$At, $^{212}$Pb, $^{212}$B, $^{32}$P and $^{33}$P, $^{71}$Ge, $^{77}$As, $^{103}$Pb, $^{105}$Rh, $^{111}$Ag, $^{119}$Sb, $^{121}$Sn, $^{131}$Cs, $^{143}$Pr, $^{161}$Tb, $^{177}$Lu, $^{191}$Os, $^{193M}$Pt, $^{197}$H, $^{43}$K, $^{52}$Fe, $^{57}$Co, $^{67}$Cu, $^{67}$Ga, $^{68}$Ga, $^{77}$Br, $^{81}$Rb/.$^{81M}$Kr, $^{87M}$Sr, $^{99M}$Tc, $^{111}$In, $^{113M}$In, $^{127}$Cs, $^{129}$Cs, $^{132}$I, $^{197}$Hg, $^{203}$Pb and $^{206}$Bi, as described in U.S. Pat. No. 8,946,168.

As used herein, "photodynamic therapy", also known as photoradiation therapy, phototherapy, and photochemotherapy, refers to a treatment that uses photosensitizing agents in combination with light to kill cancer cells. The photosensitizing agents kill cancer cells upon light activation.

As used herein, "proton therapy", also known as proton beam therapy, refers to a treatment that uses a beam of protons to irradiate and kill cancer cells.

In some embodiments, the subject is a human.

The presently disclosed subject matter also relates to kits for practicing the methods of the presently disclosed subject matter, as well as kits comprising the antibody, antibody fragment, or derivative thereof of the presently disclosed subject matter, the nucleic acid molecule encoding said components and/or the vector of the presently disclosed subject matter.

In general, a presently disclosed kit contains some or all of the components, reagents, supplies, and the like to practice a method according to the presently disclosed subject matter. In some embodiments, the term "kit" refers to any intended any article of manufacture (e.g., a package or a container) comprising at least one antibody, antibody fragment, or derivative thereof, a pharmaceutical or diagnostic composition comprising the same, and a set of particular instructions for practicing the methods of the presently disclosed subject matter. The kit can be packaged in a divided or undivided container, such as a carton, bottle, ampule, tube, *etc*. The presently disclosed compositions can be packaged in dried, lyophilized, or liquid form. Additional components provided can include vehicles for reconstitution of dried components. Preferably all such vehicles are sterile and apyrogenic so that they are suitable for injection into a subject without causing adverse reactions. Those skilled in the art will appreciate that a kit can be assembled for the treatment of any cancer or solid tumor described herein.

All embodiments covering the compounds disclosed herein can be used as single compounds or in combination for the preparation of a medicament.

Although specific terms are employed herein, they are used in a generic and descriptive sense only and not for purposes of limitation. Unless otherwise defined, all technical and scientific terms used herein have the same meaning as commonly understood by one of ordinary skill in the art to which this presently described subject matter belongs.

Following long-standing patent law convention, the terms "a," "an," and "the" refer to "one or more" when used in this application, including the claims. Thus, for example, reference to "a subject" includes a plurality of subjects, unless the context clearly is to the contrary (e.g., a plurality of subjects), and so forth.

Throughout this specification and the claims, the terms "comprise," "comprises," and "comprising" are used in a non-exclusive sense, except where the context requires otherwise. Likewise, the term "include" and its grammatical variants are intended to be non-limiting, such that recitation of items in a list is not to the exclusion of other like items that can be substituted or added to the listed items.

For the purposes of this specification and appended claims, unless otherwise indicated, all numbers expressing amounts, sizes, dimensions, proportions, shapes, formulations, parameters, percentages, parameters, quantities, characteristics, and other numerical values used in the specification and claims, are to be understood as being modified in all instances by the term "about" even though the term "about" may not expressly appear with the value, amount or range. Accordingly, unless indicated to the contrary, the numerical parameters set forth in the following specification and attached claims are not and need not be exact, but may be approximate and/or larger or smaller as desired, reflecting tolerances, conversion factors, rounding off, measurement error and the like, and other factors known to those of skill in the art depending on the desired properties sought to be obtained by the presently disclosed subject matter. For example, the term "about," when referring to a value can be meant to encompass variations of, in some embodiments, $\pm$ 100% in some embodiments $\pm$ 50%, in some embodiments $\pm$ 20%, in some embodiments $\pm$ 10%, in some embodiments $\pm$ 5%, in some embodiments $\pm$1%, in some embodiments $\pm$ 0.5%, and in some embodiments $\pm$ 0.1% from the specified amount, as such variations are appropriate to perform the disclosed methods or employ the disclosed compositions.

Further, the term "about" when used in connection with one or more numbers or numerical ranges, should be understood to refer to all such numbers, including all numbers in a range and modifies that range by extending the boundaries above and below the numerical values set forth. The recitation of numerical ranges by endpoints includes all numbers, e.g., whole integers, including fractions thereof, subsumed within that range (for example, the recitation of 1 to 5 includes 1, 2, 3, 4, and 5, as well as fractions thereof, e.g., 1.5, 2.25, 3.75, 4.1, and the like) and any range within that range.

EXAMPLES

The following Examples have been included to provide guidance to one of ordinary skill in the art for practicing representative embodiments of the presently disclosed subject matter. In light of the present disclosure and the general level of skill in the art, those of skill can appreciate that the following Examples are intended to be exemplary only and that numerous changes, modifications, and alterations can be employed without departing from the scope of the presently disclosed subject matter. The synthetic descriptions and specific *examples* that follow are only intended for the purposes of illustration, and are not to be construed as limiting in any manner to make compounds of the disclosure by other methods.

EXAMPLE 1

Summary

Two-pore domain potassium (K2P) channels act to maintain cell resting membrane potential - a prerequisite for many biological processes. KCNK9, a member of K2P family, is implicated in cancer owing to its overexpression in various human tumors and its ability to promote neoplastic cell survival and growth. However, KCNK9's underlying contributions to malignancy remain elusive due to the absence of specific modulators. Here, the development of novel monoclonal antibodies (mAbs) against KCNK9 extracellular domain and their functional effects is described. It is shown that one mAb (Y4) with the highest affinity binding, inhibits channel activity by inducing internalization. When tested on KCNK9-expressing cells, Y4 reduces cell viability; increases cell death and triggers complement-dependent cytotoxicity. Systemic administration of this antibody effectively inhibits human lung cancer xenograft and murine breast cancer metastasis in mice. These studies reveal that mAb-based KCNK9 targeting is a therapeutic strategy in KCNK9-expressing malignancy. Moreover, the presently disclosed mAb-based strategies should be generally applicable to studying other ion channels.

Materials and Methods

*Reagents:* Antibodies were obtained from the following sources: mouse antihuman growth hormone antibody (Abcam), rabbit anti-KCNK9 antibody (Alomone), goat anti-KCNK9 antibody (Santa Cruz), mouse anti-β-actin antibody, rabbit anti-cleaved caspase-3 (Cell Signaling), rabbit anti-Ki67 antibodies (Abcam), and APC-conjugated goat anti-mouse IgG antibody (Biolegend). Isotype matched mouse IgG1 (mIgG1) control was from SouthernBiotech. Anti-KCNK9 mAbs were isotyped using Rapid ELISA Mouse mAb Isotyping Kit (Pierce). Transient transfection of K2P channels was performed using Fugene6 (Promega).

*Cell culture:* HEK293, COS-7, BEN squamous cell lung carcinoma cells (DSMZ, Germany), and MDA-MB-231 breast cancer cells (gift from Dr. Sara Sukumar) were grown in Dulbecco's Modified Eagle Medium (DMEM) supplemented with 10% fetal bovine serum (FBS) and 2 mM L-Glutamine (L-Gln). HEK293-KCNK9 stable cell lines were previously generated (Miller et al. (2012) Probe Reports from the NIH Molecular Libraries Program [Intern*et]*) and KCNK9 expression was induced by incubation with 1 μM tetracycline for 16 hours. These cells were cultured in DMEM supplemented with 10% FBS, 2 mM L-Glutamine, 400 μg/ml hygromycin, and 15 μg/ml blasticidin. BT-549 breast cancer cells (gift from Dr. Sara Sukumar) were maintained in RPMI-1640 medium supplement with 10% FBS and 2 mM L-Gln. 410 and 410.4 murine breast cancer cells (gift from Dr. Amy Fulton) were cultured in DMEM supplemented with 10% FBS, 2 mM L-Gln, 0.15% w/v sodium bicarbonate and 100 μM MEM non-essential amino acids. All cells were maintained at 37°C in 5% $CO_2$.

*QRT-PCR, western blot and flow cytometry:* Total RNA from cell lines and snap-frozen tumor tissues was extracted using RNeasy Mini Kit (Qiagen) and TRIZOL Reagent (Invitrogen) respectively following manufacturer's instructions. QRT-PCR was performed as described previously (Lopez-Bertoni et al. (2014) Oncogene). Relative expression of each gene was normalized to human 18S rRNA or mouse GAPDH RNA. Sequences of all primer pairs are listed in Table 1.

Protein was extracted as described previously (Lopez-Bertoni et al. (2014) Oncogene). Western blot was performed using quantitative western blot system (LI-COR Bioscience) per manufacturer's instructions. Secondary antibodies were labeled with IRDye infrared dyes (LI-COR Biosciences).

Single-cell suspensions were labeled with anti-KCNK9 mAbs for 30 minutes at 4 °C. After washing with ice-cold PBS, cells were incubated with APC-conjugated goat anti-mIgG secondary antibody for 30 minutes at 4°C. Stained cells were then washed and analyzed using FACS Calibur flow cytometer (BD Biosciences) or sorted using FACS Aria-II flow cytometer (BD Biosciences).

Table 1. QRT-PCR primer sequences

| Gene | Accession number | Forward | Reverse |
|---|---|---|---|
| hKCNK9 | NM_016601 | GCTCCTTCTACTTT GCGATCACG (SEQ ID NO: 2) | CTGGAACATGACCAGT GTCAGC (SEQ ID NO: 3) |
| mKCNK9 | NM_001033876 | CCCCTGACGCTGG TTATGTTC (SEQ ID NO: 4) | CATGCCACAGCACTTCT TGAT (SEQ ID NO: 5) |
| mCD4 | NM_013488 | ACACACCTGTGCA AGAAGCA (SEQ ID NO: 6) | GCTCTTGTTGGTTGGGA ATC (SEQ ID NO: 7) |
| mCD8 | NM_009856 | CTCACCTGTGCAC CCTACC (SEQ ID NO: 8) | ATCCGGTCCCCTTCACT G (SEQ ID NO: 9) |
| mCD20 | NM_026956 | AACCTGCTCCAAA AGTGAACC (SEQ ID NO: 10) | CCCAGGGTAATATGGA AGAGGC (SEQ ID NO: 11) |

(continued)

| Gene | Accession number | Forward | Reverse |
|---|---|---|---|
| mCD25 | NM_008367 | GCGTTGCTTAGGAAACTCCTGG (SEQ ID NO: 12) | GCATAGACTGTGTTGGCTTCTGC (SEQ ID NO: 13) |
| mCD56 | NM_001081445 | GGTTCCGAGATGGTCAGTTGCT (SEQ ID NO: 14) | CAAGGACTCCTGTCCAATACGG (SEQ ID NO: 15) |
| mCD68 | NM_009853 | CCATCCTTCACGATGACACCT (SEQ ID NO: 16) | GGCAGGGTTATGAGTGACAGTT (SEQ ID NO: 17) |
| mCD279 | NM_008798 | CGGTTTCAAGGCATGGTCATTGG (SEQ ID NO: 18) | TCAGAGTGTCGTCCTTGCTTCC (SEQ ID NO: 19) |
| mGranzymeB | NM_013542 | CCACTCTCGACCCTACATGG (SEQ ID NO: 20) | GGCCCCCAAAGTGACATTTATT (SEQ ID NO: 21) |
| mCXCL9 | NM_008599 | CCTAGTGATAAGGAATGCACGAG (SEQ ID NO: 22) | CTAGGCAGGTTTGATCTCCGTTC (SEQ ID NO: 23) |
| mCXCL11 | NM_019494 | GGCTTCCTTATGTTCAAACAGGG (SEQ ID NO: 24) | GCCGTTACTCGGGTAAATTACA (SEQ ID NO: 25) |
| h18s rRNA | X03205 | GTAACCCGTTGAACCCCATT (SEQ ID NO: 26) | CCATCCAATCGGTAGTAGCG (SEQ ID NO: 27) |
| mGAPDH | NM_008084 | CATCACTGCCACCCAGAAGACTG (SEQ ID NO: 28) | ATGCCAGTGAGCTTCCCGTTCAG (SEQ ID NO: 29) |

*Antibody generation and purification:* Murine monoclonal antibodies (mAbs) were generated against the first extracellular domain (M1P1) of hKCNK9 (amino acids 30~88) expressed recombinantly in mammalian expression vectors and subsequently affinity-purified for immunization (Leahy et al. (2000) Protein Expression and Purification 20, 500-506; Chapoval et al. (2002) Mol Biotechnol 21, 259-264). After obtaining hybridomas, multiple rounds of limiting dilution cloning were performed. At each stage of subcloning, culture supernatants from hybridomas were screened by enzyme-linked immunosorbent assays (ELISA) using recombinant hKCNK9M1P1 and hKCNK3M1P1. Clones with highest ELISA titers specific to hKCNK9M1P1 were selected for further screen by flow cytometry analysis of staining of HEK293T cells transiently expressing recombinant hKCNK9M1P1-eGFP. This screening strategy aims to obtain single clones with high affinity binding to hKCNK9 M1P1 domain. Selected clones were scaled up and mAbs were purified using protein G agarose beads (Pierce) following manufacturer's recommendations. All mAbs are IgG1 subtypes.

*Antibody-antigen binding characterization:* MAb specificity was examined based on cross-reactivity to 9 recombinant hK2PM1P1 subtypes in western blots and further verified by flow cytometry analysis of staining of cells transiently transfected with full length hKCNK9 and its closely related K2P subtype - hKCNK3. Affinity of binding to recombinant human and murine KCNK9M1P1 was determined using Octet® system (Pall Life Sciences). $K_D$ was estimated by deriving association ($K_{on}$) and dissociation ($K_{off}$) constants from real-time measurement and calculated by:

$$K_D = K_{on}/K_{off}.$$

*Thallium ($Tl^+$)-based fluorescence assay:* A fluorescence assay previously developed for high throughput screening of small molecule modulators of KCNK9 was adapted for examining the functional effects of mAbs (Weaver et al. (2004) Journal of Biomolecular Screening 9, 671-677; Miller et al. (2012) Probe Reports from the NIH Molecular Libraries Program [Internet]). In this assay, $Tl^+$ served as a surrogate ion for $K^+$. Upon tetracycline induction of KCNK9 expression, cells were incubated with 0.4 mg/ml mAbs for 0.5 to 72 hours at 37°C. After wash-off, cells were loaded with a $Tl^+$-sensitive fluorescent dye, FluxOR™ (Invitrogen). Upon channel activation by 2.8 mM $K^+$, $Tl^+$ influx yielded a fluorescence change monitored as function of time (Miller et al. (2012) Probe Reports from the NIH Molecular Libraries Program [Internet]). Parental HEK293 and non-induced HEK293-KCNK9 cells were used as negative controls.

*Flow cytometry analysis of channel internalization:* Tet-induced HEK293-KCNK9 cells were incubated with 0.4mg/ml Y-mAbs for 36 hours at 30°C to induce endocytosis. KCNK9 channels remained on the plasma membrane were stained with a polyclonal anti-KCNK9 antibody followed by secondary antibody staining and flow-cytometry analysis as described above.

*Confocal microscopic analysis of channel internalization:* Prior to experiment, anti-KCNK9 mAbs were conjugated to Alexa Fluor 488 dye (Life Technologies). COS-7 cells were seeded onto coverslips in a 12-well plate and transiently transfected with hKCNK9 or hKCNK3. Mcherry was co-transfected as a transfection control. After 36 hour transfection, cells were treated with 0.4 mg/ml Y4 either for 3 hours at 4 °C or for 12 hours at 30°C. At the end of incubation, each well was washed with PBS. Cells were fixed with 4% paraformaldehyde, washed with PBS and mounted with Pro Long® Gold Antifade Mountant containing DAPI (Life Technologies). Images were taken at 63X/1.4 with scan zoom 1.0. For colocalization analysis, COS-7 cells were co-transfected with RFP-tagged EEA1 (Addgene) followed by the same mAb treatment protocol. Images were taken at 40X/1.3 with scan zoom 1.0. Image quantification was performed using Imaris software (Bitplane). A minimum threshold of red and green channels was selected and images were analyzed from different fields. The data represent analysis of 200 number of cells.

*Cell cycle analysis:* hKCNK9 channels on the BEN cell surface were labeled with Y4 and secondary antibody as described in the previous section. Cells were then fixed and permeablized with 80% ethanol followed by propidium iodide (PI) staining. During flow cytometry analysis, K9$^+$ and K9$^-$ cells were gated and their corresponding PI signal was collected for analysis.

*Cell viability and cell death assay:* Cancer cell lines were seeded in the presence of 10% FBS culture medium overnight at 37°C. Next day, cells were washed with PBS and treated with 2.6$\mu$M antibodies in 0.1% or 10% FBS culture medium for 24 to 72 hours at 37°C. Cell viability was measured using Cell Counting Kit-8 (Dojindo Molecular Technologies) according to manufacturer's recommendations. Cell death was determined by measuring lactate dehydrogenase (LDH) release by CytoTox 96® Non-Radioactive Cytotoxicity Assay (Promega) according to manufacturer's instructions. Percentage of cell viability and cell death was normalized to cells treated with PBS.

*Complement-dependent cytotoxicity (CDC):* The CDC assay protocol was adapted from previous studies (Konishi et al. (2008) Clinical and Vaccine Immunology: CVI 15, 88-94; Hsu et al. (2010) Molecular Cancer 9, 139). Cancer cell lines were serum-deprived for 12 hours and then treated with 0.4 mg/ml antibodies with or without murine serum complement (Rockland) for 2 hours at 37°C. Lyophilized complement sera from mouse was reconstituted in PBS and serially diluted 1:10 to 1:1000. Complement sera that was heat-inactivated for 30 minutes at 56°C was included as a negative control. Cell death was determined as described in the previous section.

*Xenograft studies:* Female 6-to 8-week-old nu/nu mice (Charles River) were anesthetized by intraperitoneal (i.p.) injection of 5 mg/kg xylazine. For BEN subcutaneous (s.c.) xenografts, mice received 4 × 10$^6$ BEN cells in 25 $\mu$L PBS and 25 $\mu$L Matrigel (Corning) s.c. in the dorsal flank. To monitor mAbs' effects on tumor initiation, mice were randomly divided into groups (n=10 per group) and received 4 mg/kg Y4 or isotype matched control in 100 $\mu$L PBS i.p. starting on day 0 followed by i.p. mAb injection twice a week. To monitor mAbs' effects on tumor regression, after tumors reached 50 mm$^3$, mice were randomly divided into groups (n = 10 per group) and received 4 mg/kg Y4 or isotype matched

control in 100 $\mu$L PBS i.p. twice a week. Tumor volumes were estimated by measuring two dimensions [length (a) and width (b)] and calculated using the equation: $V= ab^2/2$. At the end of each experiment, tumors were excised for analysis (Lopez-Bertoni et al. (2014) Oncogene).

*Metastasis studies:* Female 4-to 6-week-old Balb/cByJ mice (Jackson Laboratory) were randomly divided into groups (n=10 per group) and primed by receiving 4 mg/kg Y4 or isotype matched control in 100 $\mu$L PBS i.p. twice a week for one week prior to cell injection. Lung colonization was evaluated by injecting $2 \times 10^5$ viable 410.4 cells i.v. into the lateral tail vein of mice. Mice were then treated with 4 mg/kg Y4 or isotype matched control in 100 $\mu$L PBS i.p. twice a week for 25 days. All mice were euthanized on day 25 post-transplantation or earlier if moribund. Lungs were examined for tumor colonies under a dissecting microscope.

*Immunohistochemistry:* Cryostat sections were stained with anti-cleaved caspase-3 and anti-Ki67 antibodies as previously described (Goodwin et al. (2011) Anti-cancer drugs 22, 905-912). Biotinylated-conjugated secondary antibodies followed by incubation with 3,3'-diaminobenzidine peroxidase substrate was used to detect primary Abs. Anti-cleaved caspase-3 and anti-Ki67 stained sections were counterstained with Methyl Green. Proliferation and apoptotic indices were determined by computer-assisted quantification using ImageJ Software (rsb.info.nih.gov/ij/) as previously reported (Goodwin et al. (2011) Anti-cancer drugs 22, 905-912).

*Statistical analysis:* Two group comparisons were analyzed by t-test or ANOVA and P-values were calculated. $P<0.05$ was considered significant unless otherwise stated. All data shown are mean$\pm$standard deviation. Statistical analysis of colocalization was performed by calculating Pearson's coefficient of correlation between green and red channels. $r>0.5$ was considered significant.

Introduction

Sequences within the M1P1 domain are poorly conserved among K2P subtypes (FIG. 1C), representing a desirable extracellular epitope reservoir. Provided that the M1P1 domain harbors important modulatory sites (Clarke et al. (2008) Journal of Biological Chemistry 283, 16985-16992; Lotshaw (2007) Cell Biochemistry and Biophysics 47, 209-256). It was hypothesized that antibodies raised against the M1P1 domain allow highly selective manipulation of KCNK9 function. In this study, an inhibitory antibody was developed against the extracellular domain of KCNK9. Antibody-based KCNK9 targeting was characterized and found to effectively inhibit cancer cell survival, tumor growth and metastasis. The presently disclosed subject matter provides the first reported ion-channel targeting monocolonal antibody that shows significant therapeutic effects *in vivo*. The presently disclosed subject matter also relates to other related channels and their uses in health and disease.

Results

*M1P1 domain targeting antibodies inhibit KCNK9 channel activity:* To generate antigens that recapitulate native hKCNK9 structure, the M1P1 domain (FIG. 1B, FIG. 1C) was expressed as recombinant protein in CHO-S and HEK293T cells to optimize 3-D structure presentation and post-translational modification (FIG. 1D, FIG. 2A, FIG. 2B). Forty murine mAbs were successfully generated. Among them, four mAbs were raised to hK9M1P1-mIgG, designated as Y-mAbs and 36 mAbs were raised to hGH-hK9M1P1, designated as H-mAbs. All mAbs were of the IgG1 subtype and demonstrated a subtype-specific binding to hKCNK9 over other K2P subtypes including hKCNK3, among the K2P members most closely related to KCNK9 (FIG. 3A, FIG. 3B, FIG. 4). Y-mAbs were found to display nanomolar to sub-nanomolar affinity of binding to recombinant hKCNK9 antigen with Y4 having the highest affinity ($K_D\sim0.7$ nM). Y-mAbs also cross-reacted with the recombinant murine KCNK9 (mKCNK9) M1P1 domain with a lower affinity (FIG. 3C, FIG. 3D, FIG. 5). The degree of cross-reactivity was different among the Y-mAbs. Human and murine KNCK9 M1P1 domains differ by only 5 amino acids (FIG. 1C) providing insight into the distinctive epitopes recognized by the Y-mAbs.

To test the effects of the Y- and H-mAbs on hKCNK9 channel, a thallium (Tl$^+$)-based fluorescent ion-flux assay developed previously for hKCNK9 chemical screen was used (Weaver et al. (2004) Journal of Biomolecular Screening 9, 671-677; Miller et al. (2012) Probe Reports from the NIH Molecular Libraries Program [Internet]). In this assay, recorded fluorescent signal is proportional to the number of open K$^+$ channels on the cell surface (FIG. 6A). Twelve mAbs showed positive staining on the surface of hKCNK9-expressing HEK293 cells used in the Tl$^+$ assay (FIG. 6B). Six of these 12 mAbs were effective in inhibiting hKCNK9 conductance following a pre-incubation protocol. This response was not due to differences in cell viability as indicated by comparable fluorescence intensities at time 0 of fluorescence measurement (FIG. 6C, FIG. 7A, FIG. 7B, FIG. 7C). Among these, Y4 was most potent showing up to 30% inhibition of Tl$^+$ signal after a 36-hour mAb incubation. Hence, Y4 was chosen for follow-up studies. A time-course experiment indicated that Y4-mediated channel inhibition was stronger after longer exposures (FIG. 6C, FIG. 6D, FIG. 8) and dependent on the mAb concentration (FIG. 6E). Acute effects of mAb on channel conductance were also examined using electrophysiological assays and none of the mAbs showed detectable effects on hKCNK9 activity (FIG. 9A, FIG. 9B).

*Y4 inhibits KCNK9 by inducing channel endocytosis at cell surface:* The time dependence of Y4-mediated channel inhibition suggested a mechanism of Ab-induced endocytosis (Bhattacharyya et al. (2010) Proceedings of the National Academy of Sciences 107, 14541-14546); Mendelsohn (1997) Clinical Cancer Research 3, 2703-2707; Molina et al. (2001) Cancer Research 61, 4744-4749). Flow cytometry was used to examine this possibility. hKCNK9 over-expressing COS-7 cells were incubated with antibodies for 12 hours at 37°C, and channels remaining on the cell surface were stained using a polyclonal rabbit anti-hKCNK9 antibody. Flow cytometry analysis showed reduced surface staining in Y4-treated cells but not in cells treated with other mAbs tested (FIG. 10A, FIG. 10B, FIG. 10C), supporting endocytosis induction by Y4. For further analysis, confocal microscopy was used to track fluorescently labeled Y4. Fluorescent conjugation of Y4 did not alter its binding affinity to hKCNK9 (FIG. 11A, FIG. 11B). Upon incubation with hKCNK9-expressing cells, a consistent reduction of surface signal along with increased intracellular signal, predominantly visible in the perinuclear region was observed only at temperature permissive to endocytosis. Internalized Y4 co-localized with EEA1, an early endosome marker (FIG. 6F). This phenomenon is specific to hKCNK9 since no change was detected in cells expressing hKCNK3 (FIG. 6F). Taken together, these data indicate that reduced cell surface channel activity in ion-influx assay is due to mAb-induced internalization and endocytosis.

*Y4 recognizes endogenous KCNK9 expressed in cancer cell lines:* KCNK9 genomic amplification and protein over-expression have been reported in around 30% of breast cancer and lung cancer patients (Mu et al. (2003) Cancer Cell 3, 297-302). Enforced expression of KCNK9 promotes tumor-propagating capacity of mouse embryonic fibroblasts and this transformation was eliminated by a dominant negative KCNK9 mutant, suggesting a role for KCNK9 during tumor growth (Mu et al. (2003) Cancer Cell 3, 297-302; Pei et al. (2003) Proceedings of the National Academy of Sciences 100, 7803-7807). To assess the clinical relevance of KCNK9, correlations between KCNK9 expression and patient survival were analyzed using published datasets. High expression of KCNK9 correlated significantly with shorter overall survival of patients with squamous cell lung cancer and breast cancer. No significant correlations were found between KCNK3 expression and patient survival (FIG. 12A). This supports the notion that KNCK9 promotes tumor growth and might be a therapeutic target in KCNK9-expressing cancers.

Given that Y4 binds and inhibits hKCNK9 in a K2P subtype-specific manner, Y4-mediated channel modulation affords an advantageous strategy to investigate the role of endogenous KCNK9 in cancer biology. The BEN squamous cell lung cancer cell line was previously shown to abundantly express hKCNK9 at the mRNA level (Pei et al. (2003) Proceedings of the National Academy of Sciences 100, 7803-7807). This was confirmed at the protein level using Y4 (FIG. 12B). Subsequently, a panel of human and murine cancer cell lines was screened by qRT-PCR (FIG. 13A), followed by validation via western blotting and flow cytometry analyses to identify cell lines with high hKCNK9 expression. In addition to BEN, the BT-549 breast cancer cell line and cells from LX22 patient-derived xenograft of small cell lung cancer were found to express hKCNK9 at a relatively high level. The MDA-MB-231 breast cancer cell line, on the other hand, had undetectable channel expression and was selected as a negative control (FIG. 12B). Murine KCNK9 (mKCNK9) expression was found in metastatic and non-metastatic murine breast cancer cell lines (FIG. 13B). Higher expression levels were found in the two metastatic cell lines 66.1 and 410.4, suggesting a potential role of KCNK9 in metastasis which has not been investigated previously. Y4 stained 410.4 cells were indicative of sufficient cross-reactivity with mKCNK9 (FIG. 12B). Interestingly, both BEN and BT-549 cell lines showed two cell populations based on surface staining of hKCNK9, designated as K9$^+$ cells and K9$^-$ cells (FIG. 14). To examine if these two sub-populations possess any intrinsic differences besides hKCNK9 expression, cell cycle analysis was performed, and K9$^+$ BEN sub-population showed 34% enrichment of cells in S+G$_2$/M phases compared to K9$^-$ BEN sub-population (FIG. 12C). This suggests that hKCNK9 expression and/or function is related to cell cycle progression. Indeed, potassium channels have been reported to show cell cycle phase-dependent expression, localization and function, which in turn regulate cell cycle progression and proliferation (Huang & Jan (2014) The Journal of Cell Biology 206, 151-162).

*Inhibition of KCNK9 by Y4 reduces cell viability and increases cell death:* Inhibition of KCNK9 via enforced expression of a dominant negative mutant or shRNA had been shown to inhibit proliferation of cancer cell lines by 25~65% (Pei et al. (2003) Proceedings of the National Academy of Sciences 100, 7803-7807; Kosztka et al. (2011) Melanoma Research 21, 308-322). Similar to the effect of Y4 on inducing the internalization of transgenic hKCNK9 in HEK293, we found that Y4 induced internalization of endogenous KCNK9 in BEN cell line with 21.7% reduction of KCNK9-positive cells and 9.8% reduction of overall fluorescence in KCNK9-positive cells. To evaluate if Y4-mediated modulation of hKCNK9 function leads to any biological outcomes, BEN, BT-549, MDA-MB-231, LX22 and 410.4 cells were treated with antibodies 24-72 hours in 10% serum or 0.1% serum. Y4 at 0.4 mg/ml significantly suppressed the viability of K9$^+$ BEN, BT-549, LX22 and 410.4 cells but not K9$^-$ BEN or MDA-MB-231 cells (FIG. 12D). Consistent with results from using human cancer cell lines, Y4 was also effective in inhibiting 410.4 cell viability and increased its cell death (FIG. 12D). Inhibition in K9$^+$ BEN cells and LX22 cells was more prominent at 0.1% serum consistent with previous reports showing more pronounced effects of KCNK9 mutant in low serum.

Changes in cell viability could be due to alterations in cell proliferation, cell death or metabolism. To further dissect Y4's biological effects, Y4's impact on cell death was examined by measuring L-lactate dehydrogenase (LDH) release from cells following mAb treatment. Y4 induced significant cell death of K9$^+$ BEN, BT-549, LX22 and 410.4 cells. No significant release of LDH was detected in K9$^-$ BEN or MDA-MB-231 cells (FIG. 12E). This suggests that the observed reduction in cell viability could be attributed to increased cell death trigged by Y4. The correlation of viability effect with abundance of the target is consistent with a KCNK9-specific mechanism.

The murine IgG1 isotype possesses the ability to mediate effector functions. To investigate if Y4 is capable of eliciting complement-dependent cytotoxicity (CDC), cells were incubated with Y4 for 2 hours in the presence of serially diluted murine complement, and cytotoxicity was measured as LDH release. Y4 was effective in inducing CDC against K9$^+$ BEN and BT-549 cells. This effector response was dependent on the complement concentration, while no effects were found in K9$^-$ BEN cells or if complement was heat-inactivated (FIG. 12E).

*Y4 suppresses tumor growth in vivo:* To investigate the therapeutic effect of mAb-based KCNK9 targeting, Y4 was administered intraperitoneally (i.p.) at 4 mg/kg into nude mice either on the same day of BEN subcutaneous engraftments to monitor mAb effects during tumor initiation or after visible tumors formed to monitor effects on established tumors. Y4 effectively suppressed tumor growth in both experimental designs (FIG. 15A, FIG. 15F). Systemic administration of Y4 was well tolerated in mice as suggested by no dramatic changes in body weight (FIG. 15B, FIG. 15G). Ki67 and cleaved caspase-3 immunohistochemistry was used to determine if the differences in tumor growth rates resulted from reduced cell proliferation or increased apoptosis, respectively. Tumors treated with Y4 showed a lower proliferative index and a higher apoptotic index compared to mIgG1-treated tumors (FIG. 15C, FIG. 15D, FIG. 15H, FIG. 15I). Protein extractions from Y4-treated tumors also showed reduced hKCNK9 (FIG. 15E, FIG. 15J). We verified Y4's in vivo effects in LX22-a highly aggressive patient-derived xenograft model of small cell lung cancer-and found similar results on tumor growth inhibition, reduction of proliferative index and KCNK9 downregulation (FIG. 15K, FIG. 15L, FIG. 15M and FIG. 15N). These results indicate that Y4 treatment is able to hinder tumor growth by affecting proliferation and/or apoptosis and it down-regulated tumor-associated KCNK9.

*Y4 suppresses metastasis in vivo:* Given that Y4 can elicit CDC, it is plausible that therapeutic benefits can be gained through activation of anti-tumor immune components in addition to direct killing via antibody-antigen interaction. The cross-reactivity of Y4 with mKCNK9 provided an advantage to investigate KCNK9's role in an immunocompetent model and to examine the possibility of Y4-induced immune targeting of tumor cells. 410.4 cells were injected into the tail vein of immunocompetent BALB/cByJ mice and Y4's effects on lung metastasis were studied. Based on quantification of lung colonies, Y4-treated animals showed significantly fewer lung metastases with no detectable toxicity (FIG. 16A). These data suggest that Y4 not only is effective against primary tumor formation, but also metastasis. This could be due to direct effects via KCNK9 inhibition or elevated immune responses to tumor cells via mAb effector functions. We extracted mRNA from lung tissues and characterized the expression of immune markers (Arnould, et al. "Trastuzumab-based treatment of HER2-positive breast cancer: an antibody-dependent cellular cytotoxicity mechanism?" Br. J. Cancer 94, 259-267 (2006). Y4-treated mice showed higher infiltration of CD56+ natural killer cells, granzyme B+ cytotoxic T cells and increased T-cell chemoattractant chemokine (C-X-C motif) ligand-9 (FIG. 16B). We have shown previously that enforced expression of chemokine (C-X-C motif) ligand-9 inhibits growth and metastasis of murine breast cancer cells by a mechanism involving natural killer and T cell (Walser et al., "Immune-mediated modulation of breast cancer growth and metastasis by the chemokine Mig (CXCL9) in a murine model," J. Immunother. 30, 490-498(2007)).

To explore Y4's potential for inducing immune effector responses, we performed *in vitro* CDC and ADCC assays. We incubated cancer cells with Y4 for 2 h in the presence of murine complement before LDH assay. Y4 was effective in inducing CDC against K9+ BEN and BT-549 cells. This response was dependent on the complement concentration, whereas no effects were found in K9- BEN cells or if complement was heat inactivated (FIG. 16C). To investigate the possibility of Y4-induced ADCC, we incubated BEN cells with increasing Y4 concentrations in the presence of bioluminescent Jurkat T-reporter cells established for ADCC studies (Parekh, et al., "Development and validation of an antibody-dependent cell-mediated cytotoxicity-reporter gene assay" mAbs 4, 310-318 (2012). We found a dose-dependent induction of ADCC as indicated by luciferase activity (FIG. 16D). Based on these in vitro findings, it is believed that Y4-mediated inhibition of metastasis may involve both cell-autonomous effects and/or immune-dependent pathways.

To elucidate the possible roles of the immune system, mRNA extracted from lung tissues was analyzed with immune markers used to characterize cell-based anti-tumor responses triggered by therapeutic antibodies (Arnould et al. (2006) Br J Cancer 94, 259-267). Y4-treated mice showed higher infiltration of immune cells as indicated by increased CD56$^+$ natural killer cells, T cells including Granzyme B$^+$ cytotoxic T cells and CD25$^+$ regulatory T cells as well as T cell chemoattractant CXCL-9. This suggests that Y4-mediated inhibition of lung metastasis could be due, in part, to activation of anti-tumor immune responses via Y4's effector functions. Hence, Y4 in combination with immunomodulatory agents or cell-based therapies may generate therapeutic benefits.

Discussion

The presently disclosed subject matter discloses a novel mAb-based strategy which selectively and effectively inhibits the function of KCNK9 by inducing channel internalization. In addition, using this strategy, the presently disclosed subject matter discloses KCNK9's pivotal role in promoting primary tumor growth and metastasis. The human genome encodes over 400 ion channel genes. Their vast number and diversity allow ion channels to mediate a broad spectrum of biological processes including nerve and muscle excitation, hormone secretion, cell proliferation, learning and memory. Consequently, defects in ion channel function often have profound physiological effects. To date, mutations in over 60 different ion channel genes have been found to cause human diseases (Kullmann & Waxman (2010) The Journal of Physiology 588, 1823-1827). Because of this clinical significance, ion channels make up the second largest class of drug targets (Rask-Andersen et al. (2011) Nat Rev Drug Discov 10, 579-590). Despite vigorous therapeutic investigation, 40% of characterized ion channels have no known ligand/drug reported and most inhibitors for the remaining 60% are nonselective. Therefore, there is an increased focus toward more selective pharmacological agents (Rask-Andersen et al. (2011) Nat Rev Drug Discov 10, 579-590). Antibodies constitute the fastest growing class of therapeutics. Antibody-based therapies for cancer, autoimmune and inflammatory diseases are well established (Moskal et al. (2005) Neuropharmacology 49, 1077-1087; Scott et al. (2012) Nat Rev Cancer 12, 278-287). While antibodies are effective tools for ion channel research and structural studies, the utilization of antibodies as ion channel modulators is poorly explored. This is due, in part, to the poor epitope accessibility, specificity of extracellular domains, as well as technical challenges in obtaining high quality antigens and effective screening strategies for identifying functional antibodies. This is probably why almost all antibody modulators generated so far are polyclonal with little or no biological activity (Sun & Li (2013) Acta Pharmacol Sin 34, 199-204).

High resolution mapping of a novel amplicon in human breast and lung cancers identified KCNK9 as the single overexpressed gene. Follow-up analysis confirmed KCNK9 overexpression at the genomic, transcriptional and translational levels (Mu et al. (2003) Cancer Cell 3, 297-302). Ectopic expression of KCNK9 conferred tumorigenicity to otherwise nontumorigenic cells (Mu et al. (2003) Cancer Cell 3, 297-302). Since these initial studies, KCNK9's oncogenic properties have been explored in cancer cell line models including breast carcinoma, lung carcinoma, melanoma and glioma cell lines (Pei et al. (2003) Proceedings of the National Academy of Sciences 100, 7803-7807; Kosztka et al. (2011) Melanoma Research 21, 308-322; Meuth et al. (2008) J Neurooncol 87, 263-270; Lee et al. (2012) Acta Physiologica 204, 513-524). Depending on the cancer type studied and method used to manipulate channel function, KCNK9 had different effects on tumorigenicity. For example, inhibiting KCNK9 via a dominant negative mutant, anti-sense shRNA or non-specific chemical blocker suppressed proliferation of lung cancer, melanoma and glioma cells (Pei et al. (2003) Proceedings of the National Academy of Sciences 100, 7803-7807; Kosztka et al. (2011) Melanoma Research 21, 308-322; Meuth et al. (2008) J Neurooncol 87, 263-270). Silencing of KCNK9 in MDA-MB-231 breast cancer cells increased cell invasion and migration (Lee et al. (2012) Acta Physiologica 204, 513-524). These differences could be due to KCNK9's distinct roles in controlling cell growth and migration, differences in cancer types, or off-target effects. KCNK9 is able to form heterodimers with KCNK3. Mutant- and shRNA-based targeting may interfere with the function of KCNK9 homodimers as well as KCNK9/KCNK3 heterodimers, potentially leading to contradictory interpretation of KCNK9 function. In this regard, mAb offers preferential targeting of KCNK9 homodimers, provided that Y4 has no detectable binding to KCNK3 and high affinity/avidity of binding is required to induce endocytosis. Furthermore, mitochondrial KCNK9 was reported and linked to melanoma cell proliferation (Kosztka et al. (2011) Melanoma Research 21, 308-322). Extracellular mAb modulation allows spatial-specific regulation of KCNK9. Indeed, the mAbs used in this report affords the first demonstration of the oncogenic properties of native KCNK9 channel *in vivo* and the present study obtained consistent tumor-promoting properties in tumor growth and metastasis.

Numerous $K^+$ channels in addition to KCNK9 have been reported to be differentially expressed in human cancer and regulate different aspects of tumorigenecity including cell proliferation, migration and survival (Huang & Jan (2014) The Journal of Cell Biology 206, 151-162). However, unlike calcium ions ($Ca^{2+}$), which function as integral components of cell signaling cascades, it is still largely unknown how $K^+$ ion conductance controls biological processes. In non-excitable cells and excitable cells, the resting membrane potential is generally maintained at negative values. $K^+$ channels, K2P in particular, fine tune membrane potential to keep it at a defined range (-30 to -85 mV). As various channels open, the membrane potential can either hyperpolarize to become more negative or depolarize to become more positive, compared to the resting membrane potential. Changes in membrane potential can alter cell physiology such as cell volume dynamics that in turn regulate cell proliferation, adhesion and migration (Huang & Jan (2014) The Journal of Cell Biology 206, 151-162). For example, $Ca^{2+}$-activated $K^+$ channels ($K_{Ca}$) and inward rectifying $K^+$ channels ($K_{ir}$) are capable of regulating local cell volume at the leading edge and trailing edge to promote cancer cell migration and invasion (Huang & Jan (2014) The Journal of Cell Biology 206, 151-162). Maintenance of resting membrane potential is also critical to the function and regulation of proteins that are voltage-sensitive. For example, hyperpolarization caused by opening of K2P channel could increase $Ca^{2+}$ influx and regulate $G_1$-S phase transition during cell cycle progression. In this study, it was found that cytotoxicity induced by Y4 was more prominent at low serum concentrations, supporting previous findings that KCNK9 function protects cells in serum-deprived microenvironment (Pei et al. (2003) Proceedings of the National Academy of Sciences 100, 7803-7807). This protective role may be indirectly mediated via $Ca^{2+}$-dependent survival cascades that activate in response to stress (Li et al. (2006) Journal of the American Society of Nephrology 17, 1848-1857). Membrane potential oscillation may directly control cell cycle progression. $K^+$ channels have been shown to have cell cycle phase-dependent expression, localization and activity in multiple cell types (Huang & Jan (2014) The Journal of Cell Biology 206, 151-162). A number of reports indicate that increased $K^+$ accompanied with membrane hyperpolarization takes place at $G_1$-S progression and it is required for S phase initiation. Depolarization can prevent $G_1$/S transition in multiple cell lines including murine neuroblastoma cells and MCF-7 human breast cancer cells (Huang & Jan (2014) The Journal of Cell Biology 206, 151-162); Yang, & Brackenbury (2013) Frontiers in Physiology 4, 185); Boonstra et al. (1981) Journal of Cellular Physiology 107, 75-83 (1981); Wonderlin et al. (1995) Journal of Cellular Physiology 165, 177-185). In this study, an association was found between cell cycle phase and KCNK9 expression. The fact that KCNK9 expression disproportionally associated with S+$G_2$/M phases and opening of KCNK9 results in hyperpolarization suggests that increased expression/activity of KCNK9 promotes cell cycle progression. This would support the findings that KCNK9 targeting by mAb reduced proliferative indices and decreased tumor burden *in vivo*.

There are multiple mechanisms by which ion-channel targeting antibodies perturb channel function. Most antibodies generated by rational design act by occluding ion permeation pathway or altering modalities essential for channel gating by various stimuli (Sun & Li (2013) Acta Pharmacol Sin 34, 199-204; Lee et al. (2014) Cell 157, 1393-1404). The antibody described herein induces channel internalization. This mode of action has been reported for self-reactive antibodies in studies of paraneoplastic channelopathies such as acquired neuromyotonia (Sun & Li (2013) Acta Pharmacol Sin 34, 199-204). Tumor antigens are thought to trigger generation of self-reactive antibodies to tumor cell surface proteins including voltage-gated $K^+$ channels. These self-reactive antibodies induce channel internalization and lead to neuromuscular transmission defects (Shillito et al. (1995) Annals of Neurology 38, 714-722; Tomimitsu et al. (2004) Annals of Neurology 56, 440-444). An advantage of antibodies that act by inducing internalization compared to blocking antibodies is that efficacious inhibition depends on the amount and density of antigens expressed on the cell surface. This offers a mechanism-based specificity such that only tumor cells with sufficiently high expression of channel are targeted by antibodies. Furthermore, antibodies that efficiently endocytose are selected for developing antibody-drug conjugates where antibodies facilitate tumor-specific delivery of cytotoxic payloads to kill tumor cells. Hence, the mechanism of Y4 makes it an advantageous treatment strategy with a desirable therapeutic index by enhanced specificity and/or efficacy in combination of cytotoxic conjugates.

The presently disclosed subject matter discloses that ion channels are targetable by antibodies and that this approach has therapeutic promise. The strategies described herein to develop and characterize antibodies can be applied to other ion channels, providing insight into the physiological and pathological significance of this under-studied class of channel proteins.

**Claims**

1. An isolated antibody or antigen-binding fragment thereof that specifically binds to an epitope of the KCNK9 potassium channel between amino acids $Glu_{30}$ and $Ile_{88}$ of SEQ ID NO: 1 with an affinity $K_D$ of 0.7 nM or less, wherein the antibody or antigen-binding fragment thereof induces internalization of the KCNK9 potassium channel from the surface of a cell.

2. The antibody or antigen-binding fragment thereof according to claim 1, which does not bind to other potassium channels.

3. The antibody or antigen-binding fragment thereof according to claim 1 or claim 2, which inhibits KCNK9 activity as measured using an ion-influx assay.

4. The antibody or antigen-binding fragment thereof according to any one of claims 1-3, wherein binding of the antibody or antigen-binding fragment thereof to the KCNK9 epitope on the surface of a KCNK9-expressing cell inhibits survival of the KCNK9-expressing cell.

5. The antibody or antigen-binding fragment thereof according to any one of claims 1-4, which is conjugated to an agent selected from a therapeutic agent, a labeling agent, and an anti-neoplastic agent.

6. The antibody or antigen-binding fragment thereof according to claim 5, which is conjugated to the agent via a cleavable linker or an uncleavable linker.

7. A pharmaceutical composition comprising the antibody or antigen-binding fragment thereof according to any one of claims 1-6 and at least one anti-neoplastic agent.

8. A diagnostic composition comprising the antibody or antigen-binding fragment thereof according to any one of claims 1-6.

9. A kit comprising the antibody or antigen-binding fragment thereof according to any one of claims 1-6.

10. A method of assessing for the presence of KCNK9 expressing cells comprising contacting *in vitro* a cell or tissue suspected of expressing KCNK9 on its surface with the antibody or antigen-binding fragment thereof according to any one of claims 1-6, or the diagnostic composition according to claim 8.

11. The antibody or antigen-binding fragment thereof according to any one of claims 1-6, for use in inhibiting growth and/or metastasis of a tumor in a subject having or suspected of having breast cancer or lung cancer.

**Patentansprüche**

1. Ein isolierter Antikörper oder ein Antigen-bindendes Fragment davon, der/das spezifisch an ein Epitop des KCNK9-Kaliumkanals zwischen den Aminosäuren Glu$_{30}$ und Ile$_{88}$ von SEQ ID NO: 1 mit einer Affinität K$_D$ von 0,7 nM oder weniger bindet, wobei der Antikörper oder das Antigen-bindende Fragment davon die Internalisierung des KCNK9-Kaliumkanals von der Oberfläche einer Zelle induziert.

2. Antikörper oder Antigen-bindendes Fragment davon gemäß Anspruch 1, das nicht an andere Kaliumkanäle bindet.

3. Antikörper oder Antigen-bindendes Fragment davon gemäß Anspruch 1 oder Anspruch 2, der/das die KCNK9-Aktivität hemmt, wie gemessen unter Verwendung eines Ioneneinstrom-Assays.

4. Antikörper oder Antigen-bindendes Fragment davon gemäß einem der Ansprüche 1-3, wobei die Bindung des Antikörpers oder des Antigen-bindenden Fragments davon an das KCNK9-Epitop auf der Oberfläche einer KCNK9-exprimierenden Zelle das Überleben der KCNK9-exprimierenden Zelle hemmt.

5. Antikörper oder Antigen-bindendes Fragment davon gemäß einem der Ansprüche 1-4, der/das an ein Mittel konjugiert ist, das aus einem therapeutischen Mittel, einem Markierungsmittel und einem antineoplastischen Mittel ausgewählt ist.

6. Antikörper oder Antigen-bindendes Fragment davon gemäß Anspruch 5, der/das über einen spaltbaren Linker oder einen nicht spaltbaren Linker an das Mittel konjugiert ist.

7. Eine pharmazeutische Zusammensetzung, beinhaltend den Antikörper oder das Antigen-bindende Fragment davon gemäß einem der Ansprüche 1-6 und mindestens ein antineoplastisches Mittel.

8. Eine diagnostische Zusammensetzung, beinhaltend den Antikörper oder das Antigen-bindende Fragment davon gemäß einem der Ansprüche 1-6.

9. Ein Kit, beinhaltend den Antikörper oder das Antigen-bindende Fragment davon gemäß einem der Ansprüche 1-6.

10. Ein Verfahren zum Bewerten des Vorhandenseins von KCNK9-exprimierenden Zellen, beinhaltend das In-vitro-In-Kontakt-Bringen einer Zelle oder eines Gewebes, von der/dem vermutet wird, dass sie/es KCNK9 auf ihrer/seiner Oberfläche exprimiert, mit dem Antikörper oder dem Antigen-bindenden Fragment davon gemäß einem der Ansprüche 1-6 oder mit der diagnostischen Zusammensetzung gemäß Anspruch 8.

11. Antikörper oder Antigen-bindendes Fragment davon gemäß einem der Ansprüche 1-6 zur Verwendung bei der Hemmung des Wachstums und/oder der Metastasierung eines Tumors bei einem Subjekt mit oder mit Verdacht auf Brustkrebs oder Lungenkrebs.

**Revendications**

1. Un anticorps isolé ou un fragment de liaison à l'antigène de celui-ci qui se lie spécifiquement à un épitope du canal potassique KCNK9 entre les acides aminés Glu$_{30}$ et Ile$_{88}$ de SEQ ID NO : 1 avec une affinité K$_D$ de 0,7 nM ou moins, l'anticorps ou le fragment de liaison à l'antigène de celui-ci induisant une internalisation du canal potassique KCNK9 depuis la surface d'une cellule.

2. L'anticorps ou le fragment de liaison à l'antigène de celui-ci selon la revendication 1, lequel ne se lie pas à d'autres canaux potassiques.

3. L'anticorps ou le fragment de liaison à l'antigène de celui-ci selon la revendication 1 ou la revendication 2, lequel inhibe l'activité du KCNK9 telle que mesurée à l'aide d'un essai de flux entrant d'ions.

4. L'anticorps ou le fragment de liaison à l'antigène de celui-ci selon l'une quelconque des revendications 1 à 3, où la liaison de l'anticorps ou du fragment de liaison à l'antigène de celui-ci à l'épitope de KCNK9 sur la surface d'une cellule exprimant KCNK9 inhibe la survie de la cellule exprimant KCNK9.

5. L'anticorps ou le fragment de liaison à l'antigène de celui-ci selon l'une quelconque des revendications 1 à 4, lequel est conjugué à un agent sélectionné parmi un agent thérapeutique, un agent de marquage, et un agent antinéoplasique.

**6.** L'anticorps ou le fragment de liaison à l'antigène de celui-ci selon la revendication 5, lequel est conjugué à l'agent via un linker clivable ou un linker non clivable.

**7.** Une composition pharmaceutique comprenant l'anticorps ou le fragment de liaison à l'antigène de celui-ci selon l'une quelconque des revendications 1 à 6 et au moins un agent antinéoplasique.

**8.** Une composition pour diagnostic comprenant l'anticorps ou le fragment de liaison à l'antigène de celui-ci selon l'une quelconque des revendications 1 à 6.

**9.** Un kit comprenant l'anticorps ou le fragment de liaison à l'antigène de celui-ci selon l'une quelconque des revendications 1 à 6.

**10.** Une méthode d'évaluation de la présence de cellules exprimant KCNK9 comprenant la mise en contact *in vitro* d'une cellule ou d'un tissu supposés exprimer KCNK9 sur leur surface avec l'anticorps ou le fragment de liaison à l'antigène de celui-ci selon l'une quelconque des revendications 1 à 6, ou la composition pour diagnostic selon la revendication 8.

**11.** L'anticorps ou le fragment de liaison à l'antigène de celui-ci selon l'une quelconque des revendications 1 à 6, pour une utilisation dans l'inhibition de la croissance et/ou de la métastase d'une tumeur chez un sujet atteint ou supposé être atteint du cancer du sein ou du cancer du poumon.

FIG. 1A

FIG. 1B

K2P M1P1 DOMAIN ALIGNMENT                                                                                                    % OF IDENTITY

         M1                                                                                                      P1
hKCNK9   FDALESDHEMREEEK-----LKAEEIRI--KGKYNISSEDYRQLELVILQSEPH-------------RAGVQWKFAG          100 (SEQ. ID. NO. 30)
hKCNK15  FDALESEAESGRQRL-----LVQKRGAL--RRKFGFSAEDYRELERLALQAEPH-------------RAGRQWKFPG           46 (SEQ. ID. NO. 31)
hKCNK3   FDALESEPELIERQR-----LELRQQEL--RARYNLSQGGYEELERVVLRLKPH-------------KAGVQWRFAG           40 (SEQ. ID. NO. 32)
hKCNK16  FQLLERQAEAQSRDQ------FQLEKLRFL-ENYTCLDQWAMEQFVQVIMEAWVKGVNPKGNSTNP-----SNWDFGS           18 (SEQ. ID. NO. 33)
hKCNK1   FSSVELPYEDLLRQE-----LRKLKRRFL-EEHECLSEQQLEQFLGRVLEASNYGVSVLSNASGN-----WNWDFTS           16 (SEQ. ID. NO. 34)
hKCNK7   FQALEGPPACRLQAE-----LRAELAAFQAEHRACLPPGALEELLGTALATQAHGVSTLGNSSEG-----RTWDLPS           16 (SEQ. ID. NO. 35)
hKCNK2   FKALEQPHEISQRTT-----IVIQKQTFI-SQHSCVNSTELDELIQQIVAAINAGIIPLGNTSNQ--I--SHWDLGS           14 (SEQ. ID. NO. 36)
hKCNK4   FRALEQPHEQQAQRE-----LGEVREKFL-RAHPCVSDQELGLLIKEVADALGGGADPETNSTSN--SSHSAWDLGS           14 (SEQ. ID. NO. 37)
hKCNK10  FRALEQPFESSQKNT-----IALEKAEFL-RDHVCVSPQELETLIQHALDADNAGVSPIGNSSNN--S--SHWDLGS           14 (SEQ. ID. NO. 38)
hKCNK13  FSALELAHERQAKQR-----WEERLANF--SRGHNLSRDELRGFLRHYEEATRAGIRVDN--------VRPRWDFTG           14 (SEQ. ID. NO. 39)
hKCNK6   VARLEGPHEARLRAE-----LETLRAQLL-QRSPCVAAPALDAFVERVLAAGRLGRVVLANASGSANASDPAWDFAS           12 (SEQ. ID. NO. 40)
hKCNK12  FSALESPGEAEARAR-----WGATLRNF--SAAHGVAEPELRAFLRHYEAALAAGVRADA--------LRPRWDFPG           12 (SEQ. ID. NO. 41)
hKCNK18  FSAIEDGQVLVAADDGEFEKFLEELCRIL-NCSETVVEDRKQDLQGHLQKVKPQ-----------WFNRTTHWSFLS           10 (SEQ. ID. NO. 42)
hKCNK17  FWTLEGRAAQDSSRS-----FQRDKWELL-QNFTCLDRPALDSLIRDVVQAYKNGASLLSNTTSM-----GRWELVG            8 (SEQ. ID. NO. 43)
hKCNK5   FEVLEEPHWKEAKKN-----YYTQKLHLL-KEFPCLGQEGLDKILEVVSDAAGQGVAITGNQT-F-----NNWNWPN            6 (SEQ. ID. NO. 44)

FIG. 1C

EP 3 271 397 B1

N —— | hK9M1P1 | mIgG2a Fc | —— C

N —— | hGH | His-8 | Tev | hK9M1P1 | —— C

# FIG. 1D

FIG. 1E

FIG. 1F

ANTIGEN K9M1P1-mIgG FOR Y-mAbs

ANTIGEN hGH-K9M1P1 FOR H-mAbs

FIG. 2A

FIG. 2B

FIG. 3A

FIG. 3B

hKCNK9    ESDHEMREEEKLKAEEIRIKGKYNISSEDYRQLELVILQSEPHRAGVQWK (SEQ. ID. NO.45)
mKCNK9    ESDHEMREEEKLKAEEVRLRGKYNISSDDYQQLELVILQSEPHRAGVQWK (SEQ. ID. NO.46)

|     | $K_D$ (nM) | |
| --- | --- | --- |
|     | hKCNK9 | mKCNK9 |
| Y1 | 3.1 | 2950 |
| Y2 | 2.2 | 81.8 |
| Y3 | 2 | 61.5 |
| Y4 | 0.7 | 28.3 |

## FIG. 3C

FIG. 3D

FIG. 3E

FIG. 4

FIG. 5

FIG. 6A

## Y1

## Y4

## FIG. 6B

FIG. 6C

FIG. 6D

FIG. 6E

FIG. 6F

FIG. 6G

SEED AND INDUCE CELLS  ADD mAbs          LOAD DYE        WASH          READ
                                                  90 min          20 min

         2 HRS, 37°C          TIME COURSE, 30°C

# FIG. 7A

71

FIG. 7B

FIG. 7C

FIG. 8

FIG. 9A

FIG. 9B

SEED AND INDUCE CELLS    HARVEST CELLS AND WASH    ADD 1 °pAb    WASH AND ADD 2 °Ab    WASH AND ANALYZE

2 hrs, 37°C    36 hrs, 30°C    30min, 4°C    30min, 4°C

ADD PBS OR mAb

FIG. 10A

| TREATMENT | DIFFERENCE IN hK9$^+$ CELLS (%) |
|-----------|-------------------------------|
| Y1        | + 3.1%                        |
| Y2        | + 0.8%                        |
| Y3        | -0.2%                         |
| Y4        | -15.6%                        |

**FIG. 10B**

FIG. 10C

mAb + ALEXA 488 → PURIFICATION COLUMN → COVALENTLY LABELED mAb

FIG. 11A

FIG. 11B

FIG. 12A

FIG. 12B

81

FIG. 12C

FIG. 12D

FIG. 12D (CONT.)

FIG. 12D (CONT.)

FIG. 12E

FIG. 12E (CONT.)

FIG. 12F

FIG. 13A

SUM149PT

ZR75-30

FIG. 13A (CONT.)

FIG. 13B

FIG. 14

FIG. 15A

FIG. 15B

FIG. 15C

FIG. 15D

FIG. 15E

FIG. 15F

FIG. 15G

FIG. 15H

FIG. 15I

FIG. 15J

FIG. 15K

FIG. 15L

**FIG. 15M**

**FIG. 15N**

FIG. 16A

FIG. 16B

FIG. 16C

FIG. 16C (CONT.)

FIG. 16D

| | EC50, ng ml$^{-1}$ | INDUCTION, FOLD CHANGE |
|---|---|---|
| Y4 | 415 ± 36 | 29 ± 0.4 |
| mIgG1 | 1,121 ± 605 | 4 ± 0.5 |

H23

[ZZ] NONSPECIFIC mAb ⎯ H23 mAb

□10% SERUM    [N]1% SERUM    [■]0.2% SERUM

□10% SERUM    [N]1% SERUM    [■]0.2% SERUM

## FIG. 17A

FIG. 17B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012057421 A
- WO 0009560 A
- WO 8909622 A
- EP 0239400 A1
- WO 9007861 A
- WO 9110741 A
- WO 9402602 A
- WO 9634096 A
- WO 9633735 A
- WO 8809344 A
- US 4474893 A
- US 5959084 A
- US 5798229 A
- WO 9504064 A
- US 5827690 A
- US 5756687 A
- US 5750172 A
- US 5741957 A
- WO 2005075510 A
- US 8946168 B

### Non-patent literature cited in the description

- **SUN ; LI.** *Acta Pharmacol Sin,* 2013, vol. 34, 199-204
- **ENYEDI ; CZIRJAK.** *Physiological Reviews,* 2010, vol. 90, 559-605
- **BAREL et al.** *The American Journal of Human Genetics,* 2008, vol. 83, 193-199
- **LAFRENIERE et al.** *Nat Med,* 2010, vol. 16, 1157-1160
- **MU et al.** *Cancer Cell,* 2003, vol. 3, 297-302
- **PEI et al.** *Proceedings of the National Academy of Sciences,* 2003, vol. 100, 7803-7807
- **LINDEN et al.** *Journal of Pharmacology and Experimental Therapeutics,* 2008, vol. 327, 277-286
- **MILLER et al.** *Probe Reports from the NIH Molecular Libraries Program,* 2012
- **CHAN ; CARTER.** *Nat Rev Immunol,* 2010, vol. 10, 301-316
- **ZHANG et al.** *Cell Res,* 2007, vol. 17, 89-99
- **FINK et al.** *Embo J,* 1998, vol. 17, 3297-3308
- **LESAGE et al.** *Embo J,* 1996, vol. 15, 1004-1011
- **CLARKE et al.** *Journal of Biological Chemistry,* 2008, vol. 283, 16985-16992
- **LOTSHAW.** *Cell Biochemistry and Biophysics,* 2007, vol. 47, 209-256
- **ILONA KOVÁCS et al.** Virchows Archiv. Springer, 01 April 2005, vol. 446, 402-410
- **ANNI INNAMAAA et al.** *Anticancer Research,* 01 January 2013, vol. 33, 1401-1408
- Current Protocols in Molecular Biology. John Wiley & Sons, December 2008
- Current Protocols in Immunology. John Wiley & Sons, December 2008
- Current Protocols in Protein Science. John Wiley & Sons, December 2008
- Current Protocols in Cell Biology. John Wiley & Sons, December 2008
- **SAMBROOK ; RUSSELL ; SAMBROOK.** Molecular Cloning. A Laboratory Manual. Cold Spring Harbor Laboratory Press, 2001
- **HARLOW, E. ; LANE, D.** Antibodies-A Laboratory Manual. Cold Spring Harbor Laboratory Press, 1988
- **FRESHNEY, R. I.** Culture of Animal Cells, A Manual of Basic Technique. John Wiley & Sons, 2005
- Goodman and Gilman's The Pharmacological Basis of Therapeutics. McGraw Hill, 2005
- Basic and Clinical Pharmacology. McGraw-Hill/Appleton & Lange, 2006
- BASIC AND CLINICAL PHARMACOLOGY. July 2009
- Mendelian Inheritance in Man. **MCKUSICK, V. A.** A Catalog of Human Genes and Genetic Disorders. Johns Hopkins University Press, 1998
- **VAN DE VIJVER et al.** *New England Journal of Medicine,* 2002, vol. 347, 1999-2009
- **BILD et al.** *Nature,* 2006, vol. 439, 353-357
- **HIGGINS ; SHARP.** *CABIOS,* 1989, vol. 5, 151-153
- **HIGGINS et al.** *Comput. Appl. Biosci.,* 1992, vol. 8, 189-191
- **KOHLER ; MILSTEIN.** *Nature,* 1975, vol. 256, 495
- **GALFRE.** *Meth. Enzymol.,* 1981, vol. 73, 3
- **HARLOW ; LANE.** Antibodies, A Laboratory Manual. CSH Press, 1988
- **SCHIER.** *Human Antibodies Hybridomas,* 1996, vol. 7, 97-105

- **MALMBORG.** *J. Immunol. Methods,* 1995, vol. 183, 7-13
- **STAERZ et al.** *Nature,* 1985, vol. 314, 628
- **PEREZ et al.** *Nature,* 1985, vol. 316, 354
- **STAERZ ; BEVAN.** *Proc. Natl. Acad. Sci. USA,* 1986, vol. 83, 1453
- **STAERZ ; BEVAN.** *Immunol. Today,* 1986, vol. 7, 241
- **SAMBROOK.** Molecular Cloning A Laboratory Manual. Cold Spring Harbor Laboratory, 2001
- **AUSUBEL.** Current Protocols in Molecular Biology. Green Publishing Associates and Wiley Interscience, 1994
- **MOUELLIC.** *Proc. Natl. Acad. Sci. USA,* 1990, vol. 87, 4712-4716
- **JOYNER.** Gene Targeting, A Practical Approach. Oxford University Press
- **SCOPES.** Protein Purification. Springer-Verlag, 1982
- **MANDLER et al.** *J. Natl. Cancer Inst.,* 2000, vol. 92 (19), 1549-51
- **LIU et al.** *Proc. Natl. Acad. Sci. U.S.A.,* 1996, vol. 93, 8618-8623
- **DORONINA et al.** *Nat. Biotechnol.,* 2003, vol. 21, 778-784
- **FRANCISCO et al.** *Blood,* 2003
- **F. C. KULL et al.** *Applied Microbiology,* 1961, vol. 9, 538
- **LOPEZ-BERTONI et al.** *Oncogene,* 2014
- **LEAHY et al.** *Protein Expression and Purification,* 2000, vol. 20, 500-506
- **CHAPOVAL et al.** *Mol Biotechnol,* 2002, vol. 21, 259-264
- **WEAVER et al.** *Journal of Biomolecular Screening,* 2004, vol. 9, 671-677
- **MILLER et al.** *Probe Reports from the NIH Molecular Libraries Program [Internet],* 2012
- **KONISHI et al.** *Clinical and Vaccine Immunology: CVI,* 2008, vol. 15, 88-94
- **HSU et al.** *Molecular Cancer,* 2010, vol. 9, 139
- **GOODWIN et al.** *Anti-cancer drugs,* 2011, vol. 22, 905-912
- **BHATTACHARYYA et al.** *Proceedings of the National Academy of Sciences,* 2010, vol. 107, 14541-14546
- **MENDELSOHN.** *Clinical Cancer Research,* 1997, vol. 3, 2703-2707
- **MOLINA et al.** *Cancer Research,* 2001, vol. 61, 4744-4749
- **HUANG ; JAN.** *The Journal of Cell Biology,* 2014, vol. 206, 151-162
- **KOSZTKA et al.** *Melanoma Research,* 2011, vol. 21, 308-322
- **ARNOULD et al.** Trastuzumab-based treatment of HER2-positive breast cancer: an antibody-dependent cellular cytotoxicity mechanism?. *Br. J. Cancer,* 2006, vol. 94, 259-267
- **WALSER et al.** Immune-mediated modulation of breast cancer growth and metastasis by the chemokine Mig (CXCL9) in a murine model. *J. Immunother.,* 2007, vol. 30, 490-498
- **PAREKH et al.** Development and validation of an antibody-dependent cell-mediated cytotoxicity-reporter gene assay. *mAbs,* 2012, vol. 4, 310-318
- **ARNOULD et al.** *Br J Cancer,* 2006, vol. 94, 259-267
- **KULLMANN ; WAXMAN.** *The Journal of Physiology,* 2010, vol. 588, 1823-1827
- **RASK-ANDERSEN et al.** *Nat Rev Drug Discov,* 2011, vol. 10, 579-590
- **MOSKAL et al.** *Neuropharmacology,* 2005, vol. 49, 1077-1087
- **SCOTT et al.** *Nat Rev Cancer,* 2012, vol. 12, 278-287
- **MEUTH et al.** *J Neurooncol,* 2008, vol. 87, 263-270
- **LEE et al.** *Acta Physiologica,* 2012, vol. 204, 513-524
- **LI et al.** *Journal of the American Society of Nephrology,* 2006, vol. 17, 1848-1857
- **YANG ; BRACKENBURY.** *Frontiers in Physiology,* 2013, vol. 4, 185
- **BOONSTRA et al.** *Journal of Cellular Physiology,* 1981, vol. 107, 75-83
- **WONDERLIN et al.** *Journal of Cellular Physiology,* 1995, vol. 165, 177-185
- **LEE et al.** *Cell,* 2014, vol. 157, 1393-1404
- **SHILLITO et al.** *Annals of Neurology,* 1995, vol. 38, 714-722
- **TOMIMITSU et al.** *Annals of Neurology,* 2004, vol. 56, 440-444

SEQUENCE LISTING

<110> THE JOHNS HOPKINS UNIVERSITY

<120> NOVEL MONOCLONAL ANTIBODY INHIBITORS TARGETING POTASSIUM CHANNEL KCNK9

<130> 111232-00498

<160> 46

<170> PatentIn version 3.5

<210> 1
<211> 374
<212> PRT
<213> Homo sapiens

<400> 1

```
Met Lys Arg Gln Asn Val Arg Thr Leu Ser Leu Ile Val Cys Thr Phe
1               5               10              15

Thr Tyr Leu Leu Val Gly Ala Ala Val Phe Asp Ala Leu Glu Ser Asp
        20              25              30

His Glu Met Arg Glu Glu Glu Lys Leu Lys Ala Glu Glu Ile Arg Ile
        35              40              45

Lys Gly Lys Tyr Asn Ile Ser Ser Glu Asp Tyr Arg Gln Leu Glu Leu
    50              55              60

Val Ile Leu Gln Ser Glu Pro His Arg Ala Gly Val Gln Trp Lys Phe
65              70              75              80

Ala Gly Ser Phe Tyr Phe Ala Ile Thr Val Ile Thr Thr Ile Gly Tyr
            85              90              95

Gly His Ala Ala Pro Gly Thr Asp Ala Gly Lys Ala Phe Cys Met Phe
        100             105             110

Tyr Ala Val Leu Gly Ile Pro Leu Thr Leu Val Met Phe Gln Ser Leu
        115             120             125

Gly Glu Arg Met Asn Thr Phe Val Arg Tyr Leu Leu Lys Arg Ile Lys
    130             135             140

Lys Cys Cys Gly Met Arg Asn Thr Asp Val Ser Met Glu Asn Met Val
145             150             155             160

Thr Val Gly Phe Phe Ser Cys Met Gly Thr Leu Cys Ile Gly Ala Ala
            165             170             175
```

```
Ala Phe Ser Gln Cys Glu Glu Trp Ser Phe Phe His Ala Tyr Tyr Tyr
        180                 185                 190

Cys Phe Ile Thr Leu Thr Thr Ile Gly Phe Gly Asp Tyr Val Ala Leu
        195                 200                 205

Gln Thr Lys Gly Ala Leu Gln Lys Lys Pro Leu Tyr Val Ala Phe Ser
        210                 215                 220

Phe Met Tyr Ile Leu Val Gly Leu Thr Val Ile Gly Ala Phe Leu Asn
225                 230                 235                 240

Leu Val Val Leu Arg Phe Leu Thr Met Asn Ser Glu Asp Glu Arg Arg
                245                 250                 255

Asp Ala Glu Glu Arg Ala Ser Leu Ala Gly Asn Arg Asn Ser Met Val
        260                 265                 270

Ile His Ile Pro Glu Glu Pro Arg Pro Ser Arg Pro Arg Tyr Lys Ala
        275                 280                 285

Asp Val Pro Asp Leu Gln Ser Val Cys Ser Cys Thr Cys Tyr Arg Ser
        290                 295                 300

Gln Asp Tyr Gly Gly Arg Ser Val Ala Pro Gln Asn Ser Phe Ser Ala
305                 310                 315                 320

Lys Leu Ala Pro His Tyr Phe His Ser Ile Ser Tyr Lys Ile Glu Glu
                325                 330                 335

Ile Ser Pro Ser Thr Leu Lys Asn Ser Leu Phe Pro Ser Pro Ile Ser
        340                 345                 350

Ser Ile Ser Pro Gly Leu His Ser Phe Thr Asp His Gln Arg Leu Met
        355                 360                 365

Lys Arg Arg Lys Ser Val
        370
```

<210> 2
<211> 23
<212> DNA
<213> Artificial Sequence

<220>
<223> Forward primer sequence

<400> 2
gctccttcta ctttgcgatc acg          23

<210> 3
<211> 22
<212> DNA
<213> Artificial Sequence

<220>
<223> Reverse primer sequence

<400> 3
ctggaacatg accagtgtca gc          22

<210> 4
<211> 21
<212> DNA
<213> Artificial Sequence

<220>
<223> Forward primer sequence

<400> 4
cccctgacgc tggttatgtt c          21

<210> 5
<211> 21
<212> DNA
<213> Artificial Sequence

<220>
<223> Reverse primer sequence

<400> 5
catgccacag cacttcttga t          21

<210> 6
<211> 20
<212> DNA
<213> Artificial Sequence

<220>
<223> Forward primer sequence

<400> 6
acacacctgt gcaagaagca          20

<210> 7
<211> 20
<212> DNA
<213> Artificial Sequence

<220>
<223> Reverse primer sequence

<400> 7
gctcttgttg gttgggaatc          20

<210> 8
<211> 19
<212> DNA

<213> Artificial Sequence

<220>
<223> Forward primer sequence

<400> 8
ctcacctgtg caccctacc          19

<210> 9
<211> 18
<212> DNA
<213> Artificial Sequence

<220>
<223> Reverse primer sequence

<400> 9
atccggtccc cttcactg          18

<210> 10
<211> 21
<212> DNA
<213> Artificial Sequence

<220>
<223> Forward primer sequence

<400> 10
aacctgctcc aaaagtgaac c          21

<210> 11
<211> 22
<212> DNA
<213> Artificial Sequence

<220>
<223> Reverse primer sequence

<400> 11
cccagggtaa tatggaagag gc          22

<210> 12
<211> 22
<212> DNA
<213> Artificial Sequence

<220>
<223> Forward primer sequence

<400> 12
gcgttgctta ggaaactcct gg          22

<210> 13
<211> 23
<212> DNA
<213> Artificial Sequence

<220>

<223> Reverse primer sequence

<400> 13
gcatagactg tgttggcttc tgc        23

<210> 14
<211> 22
<212> DNA
<213> Artificial Sequence

<220>
<223> Forward primer sequence

<400> 14
ggttccgaga tggtcagttg ct        22

<210> 15
<211> 22
<212> DNA
<213> Artificial Sequence

<220>
<223> Reverse primer sequence

<400> 15
caaggactcc tgtccaatac gg        22

<210> 16
<211> 21
<212> DNA
<213> Artificial Sequence

<220>
<223> Forward primer sequence

<400> 16
ccatccttca cgatgacacc t        21

<210> 17
<211> 22
<212> DNA
<213> Artificial Sequence

<220>
<223> Reverse primer sequence

<400> 17
ggcagggtta tgagtgacag tt        22

<210> 18
<211> 23
<212> DNA
<213> Artificial Sequence

<220>
<223> Forward primer sequence

<400> 18

cggtttcaag gcatggtcat tgg          23

<210> 19
<211> 22
<212> DNA
<213> Artificial Sequence

<220>
<223> Reverse primer sequence

<400> 19
tcagagtgtc gtccttgctt cc          22

<210> 20
<211> 20
<212> DNA
<213> Artificial Sequence

<220>
<223> Forward primer sequence

<400> 20
ccactctcga ccctacatgg          20

<210> 21
<211> 22
<212> DNA
<213> Artificial Sequence

<220>
<223> Reverse primer sequence

<400> 21
ggcccccaaa gtgacattta tt          22

<210> 22
<211> 23
<212> DNA
<213> Artificial Sequence

<220>
<223> Forward primer sequence

<400> 22
cctagtgata aggaatgcac gag          23

<210> 23
<211> 23
<212> DNA
<213> Artificial Sequence

<220>
<223> Reverse primer sequence

<400> 23
ctaggcaggt ttgatctccg ttc          23

<210> 24

<211> 23
<212> DNA
<213> Artificial Sequence

<220>
<223> Forward primer sequence

<400> 24
ggcttcctta tgttcaaaca ggg         23

<210> 25
<211> 22
<212> DNA
<213> Artificial Sequence

<220>
<223> Reverse primer sequence

<400> 25
gccgttactc gggtaaatta ca          22

<210> 26
<211> 20
<212> DNA
<213> Artificial Sequence

<220>
<223> Forward primer sequence

<400> 26
gtaacccgtt gaaccccatt         20

<210> 27
<211> 20
<212> DNA
<213> Artificial Sequence

<220>
<223> Reverse primer sequence

<400> 27
ccatccaatc ggtagtagcg         20

<210> 28
<211> 23
<212> DNA
<213> Artificial Sequence

<220>
<223> Forward primer sequence

<400> 28
catcactgcc acccagaaga ctg         23

<210> 29
<211> 23
<212> DNA
<213> Artificial Sequence

<220>
<223> Reverse primer sequence

<400> 29
atgccagtga gcttcccgtt cag          23

<210> 30
<211> 57
<212> PRT
<213> Homo sapiens

<400> 30

```
        Phe Asp Ala Leu Glu Ser Asp His Glu Met Arg Glu Glu Glu Lys Leu
        1               5               10              15

        Lys Ala Glu Glu Ile Arg Ile Lys Gly Lys Tyr Asn Ile Ser Ser Glu
                        20              25              30

        Asp Tyr Arg Gln Leu Glu Leu Val Ile Leu Gln Ser Glu Pro His Arg
                    35              40              45

        Ala Gly Val Gln Trp Lys Phe Ala Gly
                50              55
```

<210> 31
<211> 57
<212> PRT
<213> Homo sapiens

<400> 31

```
        Phe Asp Ala Leu Glu Ser Glu Ala Glu Ser Gly Arg Gln Arg Leu Leu
        1               5               10              15

        Val Gln Lys Arg Gly Ala Leu Arg Arg Lys Phe Gly Phe Ser Ala Glu
                        20              25              30

        Asp Tyr Arg Glu Leu Glu Arg Leu Ala Leu Gln Ala Glu Pro His Arg
                    35              40              45

        Ala Gly Arg Gln Trp Lys Phe Pro Gly
                50              55
```

<210> 32
<211> 57
<212> PRT
<213> Homo sapiens

<400> 32

```
Phe Asp Ala Leu Glu Ser Glu Pro Glu Leu Ile Glu Arg Gln Arg Leu
1                   5                   10                  15

Glu Leu Arg Gln Gln Glu Leu Arg Ala Arg Tyr Asn Leu Ser Gln Gly
            20                  25                  30

Gly Tyr Glu Glu Leu Glu Arg Val Val Leu Arg Leu Lys Pro His Lys
            35                  40                  45

Ala Gly Val Gln Trp Arg Phe Ala Gly
        50                  55
```

<210> 33
<211> 66
<212> PRT
<213> Homo sapiens

<400> 33

```
Phe Gln Leu Leu Glu Arg Gln Ala Glu Ala Gln Ser Arg Asp Gln Phe
1                   5                   10                  15

Gln Leu Glu Lys Leu Arg Phe Leu Glu Asn Tyr Thr Cys Leu Asp Gln
            20                  25                  30

Trp Ala Met Glu Gln Phe Val Gln Val Ile Met Glu Ala Trp Val Lys
            35                  40                  45

Gly Val Asn Pro Lys Gly Asn Ser Thr Asn Pro Ser Asn Trp Asp Phe
            50                  55                  60

Gly Ser
65
```

<210> 34
<211> 66
<212> PRT
<213> Homo sapiens

<400> 34

```
Phe Ser Ser Val Glu Leu Pro Tyr Glu Asp Leu Leu Arg Gln Glu Leu
1                   5                   10                  15

Arg Lys Leu Lys Arg Arg Phe Leu Glu Glu His Glu Cys Leu Ser Glu
            20                  25                  30

Gln Gln Leu Glu Gln Phe Leu Gly Arg Val Leu Glu Ala Ser Asn Tyr
            35                  40                  45
```

```
        Gly Val Ser Val Leu Ser Asn Ala Ser Gly Asn Trp Asn Trp Asp Phe
            50                  55                  60


        Thr Ser
        65
```

<210> 35
<211> 67
<212> PRT
<213> Homo sapiens

<400> 35

```
        Phe Gln Ala Leu Glu Gly Pro Pro Ala Cys Arg Leu Gln Ala Glu Leu
        1               5                   10                  15


        Arg Ala Glu Leu Ala Ala Phe Gln Ala Glu His Arg Ala Cys Leu Pro
                    20                  25                  30


        Pro Gly Ala Leu Glu Glu Leu Leu Gly Thr Ala Leu Ala Thr Gln Ala
                    35                  40                  45


        His Gly Val Ser Thr Leu Gly Asn Ser Ser Glu Gly Arg Thr Trp Asp
                    50                  55                  60


        Leu Pro Ser
        65
```

<210> 36
<211> 67
<212> PRT
<213> Homo sapiens

<400> 36

```
        Phe Lys Ala Leu Glu Gln Pro His Glu Ile Ser Gln Arg Thr Thr Ile
        1               5                   10                  15


        Val Ile Gln Lys Gln Thr Phe Ile Ser Gln His Ser Cys Val Asn Ser
                    20                  25                  30


        Thr Glu Leu Asp Glu Leu Ile Gln Gln Ile Val Ala Ala Ile Asn Ala
                    35                  40                  45


        Gly Ile Ile Pro Leu Gly Asn Thr Ser Asn Gln Ile Ser His Trp Asp
                    50                  55                  60


        Leu Gly Ser
        65
```

<210> 37

<211> 69
<212> PRT
<213> Homo sapiens

<400> 37

```
Phe Arg Ala Leu Glu Gln Pro His Glu Gln Gln Ala Gln Arg Glu Leu
1               5               10                  15

Gly Glu Val Arg Glu Lys Phe Leu Arg Ala His Pro Cys Val Ser Asp
                20              25                  30

Gln Glu Leu Gly Leu Leu Ile Lys Glu Val Ala Asp Ala Leu Gly Gly
                35              40                  45

Gly Ala Asp Pro Glu Thr Asn Ser Thr Ser Asn Ser Ser His Ser Ala
        50              55                  60

Trp Asp Leu Gly Ser
65
```

<210> 38
<211> 67
<212> PRT
<213> Homo sapiens

<400> 38

```
Phe Arg Ala Leu Glu Gln Pro Phe Glu Ser Ser Gln Lys Asn Thr Ile
1               5               10                  15

Ala Leu Glu Lys Ala Glu Phe Leu Arg Asp His Val Cys Val Ser Pro
                20              25                  30

Gln Glu Leu Glu Thr Leu Ile Gln His Ala Leu Asp Ala Asp Asn Ala
                35              40                  45

Gly Val Ser Pro Ile Gly Asn Ser Ser Asn Asn Ser Ser His Trp Asp
        50              55                  60

Leu Gly Ser
65
```

<210> 39
<211> 62
<212> PRT
<213> Homo sapiens

<400> 39

```
Phe Ser Ala Leu Glu Leu Ala His Glu Arg Gln Ala Lys Gln Arg Trp
1               5               10                  15
```

```
            Glu Glu Arg Leu Ala Asn Phe Ser Arg Gly His Asn Leu Ser Arg Asp
                    20                  25                  30

            Glu Leu Arg Gly Phe Leu Arg His Tyr Glu Glu Ala Thr Arg Ala Gly
                    35                  40                  45

            Ile Arg Val Asp Asn Val Arg Pro Arg Trp Asp Phe Thr Gly
                    50                  55                  60
```

<210> 40
<211> 71
<212> PRT
<213> Homo sapiens

<400> 40

```
            Val Ala Arg Leu Glu Gly Pro His Glu Ala Arg Leu Arg Ala Glu Leu
            1               5                   10                  15

            Glu Thr Leu Arg Ala Gln Leu Leu Gln Arg Ser Pro Cys Val Ala Ala
                    20                  25                  30

            Pro Ala Leu Asp Ala Phe Val Glu Arg Val Leu Ala Ala Gly Arg Leu
                    35                  40                  45

            Gly Arg Val Val Leu Ala Asn Ala Ser Gly Ser Ala Asn Ala Ser Asp
                    50                  55                  60

            Pro Ala Trp Asp Phe Ala Ser
            65                  70
```

<210> 41
<211> 63
<212> PRT
<213> Homo sapiens

<400> 41

```
            Phe Ser Ala Leu Glu Ser Pro Gly Glu Ala Glu Ala Arg Arg Ala Arg
            1               5                   10                  15

            Trp Gly Ala Thr Leu Arg Asn Phe Ser Ala Ala His Gly Val Ala Glu
                    20                  25                  30

            Pro Glu Leu Arg Ala Phe Leu Arg His Tyr Glu Ala Ala Leu Ala Ala
                    35                  40                  45

            Gly Val Arg Ala Asp Ala Leu Arg Pro Arg Trp Asp Phe Pro Gly
                    50                  55                  60
```

<210> 42
<211> 66
<212> PRT
<213> Homo sapiens

<400> 42

```
Phe Ser Ala Ile Glu Asp Gly Gln Val Leu Val Ala Ala Asp Asp Gly
1               5                   10                  15

Glu Phe Glu Lys Phe Leu Glu Glu Leu Cys Arg Ile Leu Asn Cys Ser
            20                  25                  30

Glu Thr Val Val Glu Asp Arg Lys Gln Asp Leu Gln Gly His Leu Gln
            35                  40                  45

Lys Val Lys Pro Gln Trp Arg Phe Asn Arg Thr Thr His Trp Ser Phe
        50                  55                  60

Leu Ser
65
```

<210> 43
<211> 66
<212> PRT
<213> Homo sapiens

<400> 43

```
Phe Trp Thr Leu Glu Gly Arg Ala Ala Gln Asp Ser Ser Arg Ser Phe
1               5                   10                  15

Gln Arg Asp Lys Trp Glu Leu Leu Gln Asn Phe Thr Cys Leu Asp Arg
            20                  25                  30

Pro Ala Leu Asp Ser Leu Ile Arg Asp Val Val Gln Ala Tyr Lys Asn
            35                  40                  45

Gly Ala Ser Leu Leu Ser Asn Thr Thr Ser Met Gly Arg Trp Glu Leu
        50                  55                  60

Val Gly
65
```

<210> 44
<211> 65
<212> PRT
<213> Homo sapiens

<400> 44

```
Phe Glu Val Leu Glu Glu Pro His Trp Lys Glu Ala Lys Lys Asn Tyr
1               5                   10                  15

Tyr Thr Gln Lys Leu His Leu Leu Lys Glu Phe Pro Cys Leu Gly Gln
            20                  25                  30

Glu Gly Leu Asp Lys Ile Leu Glu Val Val Ser Asp Ala Ala Gly Gln
            35                  40                  45

Gly Val Ala Ile Thr Gly Asn Gln Thr Phe Asn Asn Trp Asn Trp Pro
        50                  55                  60

Asn
65
```

<210> 45
<211> 50
<212> PRT
<213> Homo sapiens

<400> 45

```
Glu Ser Asp His Glu Met Arg Glu Glu Glu Lys Leu Lys Ala Glu Glu
1               5                   10                  15

Ile Arg Ile Lys Gly Lys Tyr Asn Ile Ser Ser Glu Asp Tyr Arg Gln
            20                  25                  30

Leu Glu Leu Val Ile Leu Gln Ser Glu Pro His Arg Ala Gly Val Gln
            35                  40                  45

Trp Lys
    50
```

<210> 46
<211> 50
<212> PRT
<213> Mus musculus

<400> 46

```
Glu Ser Asp His Glu Met Arg Glu Glu Glu Lys Leu Lys Ala Glu Glu
1               5               10              15


Val Arg Leu Arg Gly Lys Tyr Asn Ile Ser Ser Asp Asp Tyr Gln Gln
        20                  25              30


Leu Glu Leu Val Ile Leu Gln Ser Glu Pro His Arg Ala Gly Val Gln
        35                  40              45


Trp Lys
    50
```